# EUROPEAN PATENT APPLICATION

(11) **EP 4 663 653 A1**
(43) Date of publication of application: **17.12.2025**
(21) Application number: 24774935.1
(22) Date of filing: 19.03.2024
(51) Int. Cl.: C07K 11/02, A61K 38/12, A61K 38/15

(54) **METHOD FOR PRODUCING EUTECTIC OF CYCLIC PEPTIDE**

(30) Priority: 20.03.2023 JP 2023044727
(71) Applicant: CHUGAI SEIYAKU KABUSHIKI KAISHA, Tokyo 115-8543 (JP)
(72) Inventor: IWATA, Tomoya, Yokohama City, Kanagawa 244-8602 (JP); TANIDA, Satoshi, Yokohama City, Kanagawa 244-8602 (JP); MAENO, Yusuke, Yokohama City, Kanagawa 244-8602 (JP)
(74) Representative: Vossius & Partner Patentanwälte Rechtsanwälte mbB
(86) International application number: PCT/JP2024/010825
(87) International publication number: WO 2024/195801

(57) **Abstract**

A method for producing a cocrystal containing a cyclic peptide and a compound with a melting point of 20°C or higher, the method comprising a step of bringing the cyclic peptide into contact with the compound and a solvent.

## Description

### [Technical Field]

The present invention relates to a method for producing a cocrystal of a cyclic peptide and a method for screening the cocrystal thereof.

### [Background Art]

Conventional compounds used as oral drugs have been considered to be desirable to have, as one of the conditions for example, a molecular weight of 500 g/mol or less, as is known as the Lipinski rule (Non Patent Literature 1). In recent years, it has become known that compounds with a molecular weight of more than 500 g/mol may contribute to the inhibition of the interaction at the surface of a target protein, such as protein-protein interaction, which is difficult to interact by conventional small molecule compounds and has been referred to as a tough target. These molecules are called middle molecule compounds, which are neither small molecules with a molecular weight of 500 g/mol or less that have been mainly used as oral drugs, nor polymeric molecules with a molecular weight of more than 100000 g/mol, such as antibody pharmaceuticals, and have gained prominence as new modalities that can realize drug discovery against tough targets (Non Patent Literature 2).

Peptides composed of natural amino acids, such as insulin used in the treatment of hyperglycemia, have poor metabolic stability and have conventionally been difficult to be developed as oral drugs. However, it has been found that the metabolic stability and membrane permeability of the peptide is improved by cyclizing the peptide and using a non-natural amino acid such as N-methylamino acid in the peptide (Non Patent Literatures 3 and 4).

Among cyclic peptides containing non-natural amino acids, it has become known that cyclic peptides particularly containing N-substituted amino acids may have metabolic stability or membrane permeability, that is, may have drug-likeness (Patent Literature 1). It has also been suggested that cyclic peptides containing non-natural amino acids are useful for the creation of inhibitors of protein-protein interactions (Non Patent Literature 5).

While cyclic peptides as middle molecule compounds are attracting attention in drug discovery, there are few descriptions on crystals for most of the currently known cyclic peptides. As for cyclosporin A, which has long been known as a cyclic peptide, it has been reported to have a variety of crystal forms (Patent Literatures 2 and 3, Non Patent Literatures 6, 7, and 8). There have also been disclosed specific crystals of GNP-bound G-αS bound to the cyclic peptide GN13 and GDP-bound G-αS bound to the cyclic peptide inhibitor GD20 (Non Patent Literature 9). However, there are many cyclic peptides that are difficult to crystallize, and more options for crystal production methods are desired.

### [Citation List]

### [Patent Literature]

[Patent Literature 1] International Publication No. WO 2013/100132
[Patent Literature 2] International Publication No. WO 2012/166610
[Patent Literature 3] Japanese Patent Laid-Open No. 2017-210488

### [Non Patent Literature]

[Non Patent Literature 1] Adv. Drug Del. Rev. 1997, 23, 3-25.
[Non Patent Literature 2] Future Med. Chem., 2009, 1, 1289-1310.
[Non Patent Literature 3] Acc. Chem. Res., 2008, 41, 1331-1342.
[Non Patent Literature 4] Angew. Chem. Int. Ed., 2013, 52, 254-269.
[Non Patent Literature 5] Chem. Rev., 2019, 119, 10360-10391.
[Non Patent Literature 6] J. Pharm. Sci., 2018, 107, 3070-3079.
[Non Patent Literature 7] J. Inclusion Phenomena & Macrocyclic Chem., 2000, 37, 137-153.
[Non Patent Literature 8] Zeitschrift fuer Kristallographie, 1996, 211, 313-318.
[Non Patent Literature 9] Cell, 2022, 185, 3950.

### [Summary of Invention]

### [Technical Problem]

As described in Cryst. Growth. Des. 2012, 12, 2147-2152, pharmaceutical crystals are broadly classified into single-component crystals and multi-component crystals, and multi-component crystals are broadly classified into hydrates/solvates, salts, and cocrystals.

Here, as crystals related to cyclic peptides, for example, cyclosporin A, which has long been known, has been reported to have a variety of crystal forms (Patent Literatures 2 and 3, Non Patent Literatures 6, 7, and 8), but crystals composed of multiple components are all considered to be in the hydrate/solvate category, and there have been no reports on cocrystals containing a compound (coformer) that is solid at normal temperature. A general and simple technology for obtaining these cyclic peptides as cocrystals is needed.

The present invention has been made in view of such circumstances. In one aspect, an object of the present invention is to provide a method for producing a cocrystal of a cyclic peptide, in particular, a method for producing a cocrystal of a cyclic peptide containing a N-substituted amino acid residue. In one aspect, an object of the present invention is to provide a method for screening a cocrystal of a cyclic peptide, in particular, a method for screening a cocrystal of a cyclic peptide containing a N-substituted amino acid residue. In one aspect, an object of the present invention is to provide a method for screening a method for producing a cocrystal of a cyclic peptide. Furthermore, in one aspect, an object of the present invention is to provide a method for isolating and purifying a cyclic peptide as a cocrystal in order to produce the target cyclic peptide or salt thereof, or solvate thereof, without using column chromatography.

### [Solution to Problem]

As a result of diligent investigations in order to solve the above problems, the present inventors have found a method for efficiently screening a cocrystal in order to obtain a cyclic peptide as a cocrystal, by bringing the cyclic peptide into contact with a specific compound and a specific solvent. Furthermore, the present invention has been completed as a method for producing a cocrystal in which the cyclic peptide is the only peptide component.

The present disclosure relates to the following.
[A1] A method for producing a cocrystal containing a cyclic peptide and a compound with a melting point of 20°C or higher, the method comprising a step of bringing the cyclic peptide into contact with the compound and a solvent.
[A2] The method according to [A1], wherein the compound is a compound with a molecular weight (g/mol) of 500 or less, preferably 400 or less, more preferably 300 or less, still more preferably 200 or less, and most preferably 150 or less or 125 or less.
[A3] The method according to [A1], wherein the compound is a compound with a molecular weight (g/mol) of 150 or less.
[A3-1] The method according to any one of [A1] to [A3], wherein the compound is a compound with a molecular weight (g/mol) of 50 or more, and preferably 55 or more or 58 or more.
[A3-2] The method according to any one of [A1] to [A3], wherein the compound is a compound with a molecular weight of 58 or more.
[A4] The method according to any one of [A1] to [A3-2], wherein the compound is a compound represented by R¹-C(=O)-NR²R³ or R¹-C≡N, where R¹ is a C₁-C₄ alkyl, phenyl, 2-pyridyl, 3-pyridyl, 4-pyridyl, 2-furyl, -NH₂, -NH-OH, -C(=O)-NH₂, -CH₂-C(=O)-NH₂, or -(CH₂)₂-C(=O)-NH₂, and R² and R³ are each independently hydrogen or a C₁-C₄ alkyl.
[A5] The method according to any one of [A1] to [A4], wherein the compound is a compound represented by R¹-C(=O)-NR²R³, where R¹ is a C₁-C₄ alkyl, phenyl, 2-pyridyl, 3-pyridyl, 4-pyridyl, 2-furyl, -NH₂, -NH-OH, -C(=O)-NH₂, -CH₂-C(=O)-NH₂, or -(CH₂)₂-C(=O)-NH₂, and R² and R³ are each independently hydrogen or a C₁-C₄ alkyl.
[A6] The method according to [A4] or [A5], wherein R² and/or R³ are hydrogen.
[A6-1] The method according to [A4] or [A5], wherein R² is hydrogen and R³ is hydrogen.
[A7] The method according to [A4] or [A5], wherein R¹ is 2-pyridyl, 3-pyridyl, 2-furyl, or -NH₂, and R² and R³ are hydrogen.
[A8] The method according to [A4], wherein the compound is a compound represented by R¹-C≡N, where R¹ is 2-pyridyl, 3-pyridyl, or 4-pyridyl.
[A9] A method for producing a cocrystal containing a cyclic peptide and a compound selected from the group consisting of urea, nicotinamide, 2-furamide, and 4-cyanopyridine, the method comprising a step of bringing the cyclic peptide into contact with the compound and a solvent.
[A10] The method according to [A9], wherein the compound is urea, nicotinamide, or 2-furamide.
[A11] The method according to [A9], wherein the compound is urea or nicotinamide.
[A12] The method according to [A9], wherein the compound is urea.
[A13] The method according to [A9], wherein the compound is nicotinamide.
[A14] The method according to any one of [A1] to [A13], wherein the cyclic peptide has the following characteristic (i):
   (i) a characteristic of containing a cyclic portion composed of 4 to 16 residues of amino acids in total.
[A14-1] The method according to any one of [A1] to [A13], wherein the cyclic peptide has the following characteristic (i):
   (i) a characteristic of containing a cyclic portion composed of 8 to 16 residues of amino acids in total.
[A14-2] The method according to any one of [A1] to [A13], wherein the cyclic peptide has the following characteristic (i):
   (i) a characteristic of containing a cyclic portion composed of 8 to 16 residues of amino acids in total, with a total number of amino acids being 8 to 20 residues.
[A15] The method according to [A14] or [A14-1], wherein the cyclic peptide further has the following characteristic (ii):
   (ii) a characteristic of containing at least one N-substituted amino acid.
[A16] The method according to any one of [A1] to [A13], wherein the cyclic peptide has the following characteristics (i) and (ii):
   (i) a characteristic of containing a cyclic portion composed of 8 to 16 residues of amino acids in total, with a total number of amino acids being 8 to 20 residues; and
   (ii) a characteristic of containing at least one N-substituted amino acid.
[A16-1] The method according to any one of [A1] to [A13], wherein the cyclic peptide has the following characteristics (i) and (ii):
   (i) a characteristic of containing a cyclic portion composed of 9 to 15 residues of amino acids in total, with a total number of amino acids being 9 to 18 residues; and
   (ii) a characteristic of containing at least three N-substituted amino acids.
[A16-2] The method according to any one of [A1] to [A13], wherein the cyclic peptide has the following characteristics (i) and (ii):
   (i) a characteristic of containing a cyclic portion composed of 10 to 14 residues of amino acids in total, with a total number of amino acids being 10 to 16 residues; and
   (ii) a characteristic of containing at least four N-substituted amino acids.
[A16-3] The method according to any one of [A1] to [A13], wherein the cyclic peptide has the following characteristics (i) and (ii):
   (i) a characteristic of containing a cyclic portion composed of 11 to 13 residues of amino acids in total, with a total number of amino acids being 11 to 15 residues; and
   (ii) a characteristic of containing at least four N-substituted amino acids.
[A17] The method according to any one of [A1] to [A13], wherein the cyclic peptide has the following characteristics (i) and (ii):
   (i) a characteristic of containing a cyclic portion composed of 11 to 13 residues of amino acids in total, with a total number of amino acids being 11 to 15 residues; and
   (ii) a characteristic of containing at least five N-substituted amino acids.
[A17-1] The method according to any one of [A1] to [A13], wherein the cyclic peptide has the following characteristics (i) and (ii):
   (i) a characteristic of containing a cyclic portion composed of 11 to 12 residues of amino acids in total, with a total number of amino acids being 11 to 12 residues; and
   (ii) a characteristic of containing at least six N-substituted amino acids.
[A18] The method according to any one of [A14] to [A17-1], wherein the cyclic peptide further has the following characteristic (iii):
   (iii) a characteristic of having a molecular weight (g/mol) of 1110 or more and 3000 or less.
[A19] The method according to any one of [A14] to [A17-1], wherein the cyclic peptide further has the following characteristic (iii):
   (iii) a characteristic of having a molecular weight (g/mol) of 1110 or more and 3000 or less, preferably 1204 or more and 2500 or less, more preferably 1204 or more and 2000 or less, still more preferably 1300 or more and 1800 or less, and most preferably 1300 or more and 1700 or less.
[A20] The method according to any one of [A14] to [A17-1], wherein the cyclic peptide further has the following characteristic (iii):
   (iii) a characteristic of having a molecular weight (g/mol) of 1300 to 1700.
[A21] The method according to any one of [A1] to [A20], wherein the solvent is solvent (A) with a molecular weight of 30 or more and 170 or less, or a mixed solvent of solvent (A) with a molecular weight of 30 or more and 170 or less and solvent (B) with a molecular weight of 18 or more and 75 or less, the solvent (A) is one or more types selected from the group consisting of an amide-based solvent, a sulfoxide-based solvent, an aromatic hydrocarbon-based solvent, a halogen-based solvent, an alcohol-based solvent, an ether-based solvent, an ester-based solvent, a nitrile-based solvent, a carboxylic acid-based solvent, and a ketone-based solvent, and the solvent (B) is one or more types selected from the group consisting of an alkyl alcohol-based solvent and water.
[A22] The method according to [A21], wherein the solvent (A) is one or more types selected from the group consisting of an amide-based solvent, an alcohol-based solvent, an ether-based solvent, a carboxylic acid-based solvent, and a ketone-based solvent.
[A23] The method according to [A21] or [A22], wherein the solvent (A) is an alcohol-based solvent.
[A24] The method according to any one of [A21] to [A23], wherein the alcohol-based solvent is one or more types selected from the group consisting of methanol, ethanol, 1-propanol, 2-propanol, 1-butanol, 1-pentanol, 2-methyl-1-propanol, 2-methyl-1-butanol, 2-methoxyethanol, 2-ethoxyethanol, 2,2,2-trifluoroethanol, 1,1,1,3,3,3-hexafluoro-2-propanol, and benzyl alcohol.
[A25] The method according to any one of [A21] to [A23], wherein the alcohol-based solvent is 2-methoxyethanol or 2-ethoxyethanol.
[A25-1] The method according to [A21] or [A22], wherein the solvent (A) is an amide-based solvent.
[A25-2] The method according to any one of [A21] to [A23], wherein the amide-based solvent is one or more types selected from the group consisting of N,N-dimethylformamide, N,N-dimethylacetamide, N-formylmorpholine, N-methyl-2-pyrrolidone, N-ethyl-2-pyrrolidone, N-butyl-2-pyrrolidone, and formamide.
[A26] The method according to any one of [A21] to [A23], wherein the amide-based solvent is N-methyl-2-pyrrolidone.
[A26-1] The method according to [A21] or [A22], wherein the solvent (A) is an ether-based solvent.
[A27] The method according to any one of [A21] to [A22] and [A24] to [A26-1], wherein the ether-based solvent is one or more types selected from the group consisting of diethyl ether, tetrahydrofuran, 2-methyltetrahydrofuran, cyclopentyl methyl ether, 4-methyltetrahydropyran, 1,3-dioxolane, 1,4-dioxane, 1,2-dimethoxyethane, diisopropyl ether, anisole, and t-butyl methyl ether.
[A28] The method according to any one of [A21] to [A22] and [A24] to [A26-1], wherein the ether-based solvent is 1,4-dioxane.
[A29] The method according to [A21] or [A22], wherein the solvent (A) is a carboxylic acid-based solvent.
[A30] The method according to any one of [A21] to [A22], [A24] to [A25], and [A27] to [A29], wherein the carboxylic acid-based solvent is one or more types selected from the group consisting of formic acid, acetic acid, and propionic acid.
[A31] The method according to any one of [A21] to [A22], [A24] to [A25], and [A27] to [A29], wherein the carboxylic acid-based solvent is acetic acid.
[A32] The method according to [A21] or [A22], wherein the solvent (A) is a ketone-based solvent.
[A33] The method according to any one of [A21] to [A22], [A24] to [A25], [A27] to [A28], and [A30] to [A32], wherein the ketone-based solvent is one or more types selected from the group consisting of acetone, methyl ethyl ketone, methyl isobutyl ketone, methyl butyl ketone, cyclohexanone, diethyl ketone, and cyclopentanone.
[A34] The method according to any one of [A21] to [A22], [A24] to [A25], [A27] to [A28], and [A30] to [A32], wherein the ketone-based solvent is acetone.
[A35] The method according to any one of [A21] to [A34], wherein the solvent (B) is one or more types of solvents selected from the group consisting of an alkyl alcohol-based solvent and water.
[A36] The method according to any one of [A21] to [A34], wherein the solvent (B) is an alkyl alcohol-based solvent or water.
[A37] The method according to any one of [A21] to [A34], wherein the solvent (B) is an alkyl alcohol-based solvent.
[A38] The method according to any one of [A35] to [A37], wherein the alkyl alcohol-based solvent is one or more types selected from the group consisting of methanol, ethanol, 1-propanol, 2-propanol, 1-butanol, and 2-methyl-1-propanol.
[A39] The method according to any one of [A35] to [A37], wherein the alkyl alcohol-based solvent is ethanol.
[A40] The method according to any one of [A21] to [A34], wherein the solvent (B) is water.
[A41] The method according to any one of [A21] to [A40], wherein the volume ratio (v/v) between the solvent (A) and the solvent (B) in the solvent is 1:0 to 1:10.
[A42] The method according to any one of [A21] to [A40], wherein the volume ratio (v/v) between the solvent (A) and the solvent (B) in the solvent is 1:0 to 1:8, 1:0 to 1:5, 1:0 to 1:4, 1:0 to 1:3, 1:1 to 1:4, or 1:1 to 1:3.
[A43] The method according to any one of [A21] to [A40], wherein the volume ratio (v/v) between the solvent (A) and the solvent (B) in the solvent is 1:1 to 1:3.
[A44] The method according to any one of [A21] to [A43], wherein the solvent (A) has a melting point of 25°C or lower and the solvent (B) has a melting point of 25°C or lower.
[A45] The method according to any one of [A1] to [A44], wherein, in the step of bringing the cyclic peptide into contact with the compound and the solvent, the concentration of the cyclic peptide is 1 mg to 700 mg/mL.
[A46] The method according to any one of [A1] to [A44], wherein, in the step of bringing the cyclic peptide into contact with the compound and the solvent, the concentration of the cyclic peptide is 5 mg to 700 mg/mL, preferably 10 mg to 500 mg/mL, more preferably 10 mg to 400 mg/mL, still more preferably 10 mg to 100 mg/mL, and most preferably 10 mg to 50 mg/mL.
[A47] The method according to any one of [A1] to [A46], wherein the cyclic peptide used in the step of bringing the cyclic peptide into contact with the compound and the solvent is a freeze-dried product.
[A48] The method according to any one of [A1] to [A47], wherein the cyclic peptide used in the step of bringing the cyclic peptide into contact with the compound and the solvent is a cyclic peptide contained in a mixture containing the cyclic peptide, and the weight ratio of the cyclic peptide in the mixture is 50% or more.
[A48-1] The method according to any one of [A1] to [A47], wherein the cyclic peptide used in the step of bringing the cyclic peptide into contact with the compound and the solvent is a cyclic peptide contained in a composition containing the cyclic peptide, and the weight ratio of the cyclic peptide in the mixture is 60% or more, preferably 70% or more, more preferably 85% or more, and most preferably 95% or more.
[A48-2] The method according to any one of [A1] to [A48-1], wherein the cyclic peptide used in the step of bringing the cyclic peptide into contact with the compound and the solvent is a cyclic peptide contained in a composition containing the cyclic peptide, and the area of a peak of HPLC at 225 nm accounts for 50% or more of the total area of all peaks detected in HPLC analysis of the composition.
[A48-3] The method according to any one of [A1] to [A48-2], wherein the cyclic peptide used in the step of bringing the cyclic peptide into contact with the compound and the solvent is a cyclic peptide contained in a composition containing the cyclic peptide, and the area of a peak at 225 nm derived from the cyclic peptide in the case where the mixture is analyzed by HPLC accounts for 50% or more of the total area of all peaks detected in HPLC analysis of the composition.
[A49] The method according to any one of [A1] to [A48-2], wherein the cyclic peptide used in the step of bringing the cyclic peptide into contact with the compound and the solvent is a cyclic peptide contained in a composition containing the cyclic peptide, and the area of a peak of HPLC at 225 nm accounts for 60% or more, preferably 70% or more, more preferably 85% or more, and most preferably 95% or more of the total area of all peaks detected in HPLC analysis of the composition.
[A49-1] The method according to any one of [A1] to [A49-1], wherein the cyclic peptide used in the step of bringing the cyclic peptide into contact with the compound and the solvent is a cyclic peptide contained in a composition containing the cyclic peptide, and the area of a peak of HPLC at 225 nm derived from the cyclic peptide in the case where the mixture is analyzed by HPLC accounts for 60% or more, preferably 70% or more, more preferably 85% or more, and most preferably 95% or more of the total area of all peaks detected in HPLC analysis of the composition.
[A50] The method according to any one of [A1] to [A49], wherein the amount of the cyclic peptide used in the step of bringing the cyclic peptide into contact with the compound and the solvent is 0.5 mg to 200 kg.
[A51] The method according to any one of [A1] to [A49], wherein the cyclic peptide used in the step of bringing the cyclic peptide into contact with the compound and the solvent is 0.5 mg to 10 g, preferably 1 mg to 1 g, more preferably 1 mg to 100 mg, and most preferably 1 mg to 5 mg.
[A52] The method according to any one of [A1] to [A49], wherein the cyclic peptide used in the step of bringing the cyclic peptide into contact with the compound and the solvent is 10 g to 200 kg, 100 g to 100 kg, 0.5 mg to 10 g, 1 mg to 1 g, 1 mg to 100 mg, 1 mg to 10 mg, or 1 mg to 5 mg.
[A53] The method according to any one of [A1] to [A52], wherein the step of bringing the cyclic peptide into contact with the compound and the solvent does not comprise adding a seed crystal.
[A54] The method according to any one of [A1] to [A52], wherein the step of bringing the cyclic peptide into contact with the compound and the solvent comprises adding a seed crystal.
[A55] The method according to any one of [A1] to [A54], further comprising a solid-liquid separation step after the step of bringing the cyclic peptide into contact with the compound and the solvent.
[A55-1] The method according to [A55], wherein the solid-liquid separation is centrifugation or filtration.
[A55-2] The method according to [A55], wherein the solid-liquid separation is filtration.
[A56] The method according to any one of [A1] to [A55-2], wherein the step of bringing the cyclic peptide into contact with the compound and the solvent comprises bringing into contact with beads.
[A57] The method according to any one of [A1] to [A55-2], wherein the step of bringing the cyclic peptide into contact with the compound and the solvent is carried out under grinding conditions using tungsten beads.
[A58] The method according to any one of [A1] to [A57], wherein the step of bringing the cyclic peptide into contact with the compound and the solvent is carried out under stirring or shaking conditions.
[A58-1] The method according to any one of [A1] to [A58], wherein the cocrystal has one or more diffraction peaks in powder X-ray diffraction using CuKα radiation.
[A59] The method according to any one of [A1] to [A58-1], wherein the step of bringing the cyclic peptide into contact with the compound and the solvent is carried out at a temperature of - 10°C to 120°C for 30 minutes to 12 weeks.
[A60] The method according to any one of [A1] to [A59], wherein the step of bringing the cyclic peptide into contact with the compound and the solvent is carried out at a constant temperature in 0°C to 110°C, and carried out for 1 hour to 6 weeks, 2 hours to 4 weeks, 4 hours to 2 weeks, or 6 hours to 8 days.
[A61] The method according to any one of [A1] to [A60], wherein the step of bringing the cyclic peptide into contact with the compound and the solvent is carried out at a constant temperature in 0°C to 110°C, and carried out for 1 hour to 8 days.
[A62] The method according to any one of [A1] to [A60], wherein the step of bringing the cyclic peptide into contact with the compound and the solvent is carried out at a constant temperature in 20°C to 90°C for 12 hours to 8 days.
[A62-1] The method according to any one of [A1] to [A60], wherein the step of bringing the cyclic peptide into contact with the compound and the solvent is carried out at a constant temperature in 30°C to 80°C for 12 hours to 8 days.
[A62-2] The method according to any one of [A1] to [A60], wherein the step of bringing the cyclic peptide into contact with the compound and the solvent is carried out repeating heating and cooling between a lower limit temperature of 10°C and an upper limit temperature of 100°C, 10 times or more and 100 times or less, and carried out for 1 hour to 8 days.
[A62-3] The method according to any one of [A1] to [A60], wherein the step of bringing the cyclic peptide into contact with the compound and the solvent is carried out repeating heating and cooling between a lower limit temperature of 30°C and an upper limit temperature of 100°C, 30 times or more and 100 times or less, and carried out for 1 hour to 8 days.
[A62-4] The method according to any one of [A1] to [A60], wherein the step of bringing the cyclic peptide into contact with the compound and the solvent is carried out repeating heating and cooling between a lower limit temperature of 50°C and an upper limit temperature of 90°C, 30 times or more and 100 times or less, and carried out for 2 hours to 8 days.
[A62-5] The method according to any one of [A1] to [A60], wherein the step of bringing the cyclic peptide into contact with the compound and the solvent is carried out repeating heating and cooling between a lower limit temperature of 50°C and an upper limit temperature of 90°C, 30 times or more and 100 times or less, and carried out for 2 hours to 8 days.
[A62-6] The method according to any one of [A1] to [A60], wherein the step of bringing the cyclic peptide into contact with the compound and the solvent is carried out repeating heating and cooling between a lower limit temperature of 50°C and an upper limit temperature of 90°C, 30 times or more and 100 times or less, and carried out for 12 hours to 8 days.
[A63] The method according to any one of [A1] to [A62-6], wherein the cyclic peptide contains a cyclic portion composed of 7 to 15 residues of natural amino acids and non-natural amino acids in total, with a total number of natural amino acids and non-natural amino acids being 9 to 18 residues.
[A63-1] The method according to any one of [A1] to [A62-6], wherein the cyclic peptide contains a cyclic portion composed of 8 to 14 residues of natural amino acids and non-natural amino acids in total, with a total number of natural amino acids and non-natural amino acids being 10 to 16 residues.
[A63-2] The method according to any one of [A1] to [A62-6], wherein the cyclic peptide contains a cyclic portion composed of 9 to 13 residues of natural amino acids and non-natural amino acids in total, with a total number of natural amino acids and non-natural amino acids being 10 to 14 residues.
[A63-3] The method according to any one of [A1] to [A62-6], wherein the cyclic peptide contains a cyclic portion composed of 10 to 13 residues of natural amino acids and non-natural amino acids in total, with a total number of natural amino acids and non-natural amino acids being 11 to 14 residues.
[A64] The method according to any one of [A1] to [A62-6], wherein the cyclic peptide contains a cyclic portion composed of 11 to 13 residues of natural amino acids and non-natural amino acids in total, with a total number of natural amino acids and non-natural amino acids being 11 to 14 residues.
[A65] The method according to any one of [A1] to [A62-6], wherein the cyclic peptide contains a cyclic portion composed of 11 residues of natural amino acids and non-natural amino acids in total, with a total number of natural amino acids and non-natural amino acids being 11 residues.
[A66] The method according to any one of [A1] to [A65], wherein the cyclic peptide contains at least 3 residues of N-substituted amino acids.
[A66-1] The method according to any one of [A1] to [A65], wherein the cyclic peptide contains at least 4 residues of N-substituted amino acids.
[A66-2] The method according to any one of [A1] to [A65], wherein the cyclic peptide contains at least 5 residues of N-substituted amino acids.
[A67] The method according to any one of [A1] to [A66-2], wherein the cyclic peptide contains at least 1 residue of a N-unsubstituted amino acid.
[A68] The method according to any one of [A1] to [A66-2], wherein the cyclic peptide contains at least 2 residues of N-unsubstituted amino acids.
[A69] The method according to any one of [A1] to [A68], wherein the cyclic peptide contains at least 3 residues of N-unsubstituted amino acids.
[A70] The method according to any one of [A66] to [A69], wherein the N-substituted amino acid is a N-alkylamino acid.
[A71] The method according to any one of [A66] to [A69], wherein the N-substituted amino acid is a N-methylamino acid or a N-ethylamino acid.
[A72] The method according to any one of [A66] to [A69], wherein the N-substituted amino acid is a N-methylamino acid.
[A73] The method according to any one of [A1] to [A72], wherein the cyclic peptide contains at least 1 residue of a β-amino acid.
[A74] The method according to any one of [A1] to [A73], wherein the cyclic portion of the cyclic peptide contains at least 1 residue of a β-amino acid.
[A75] The method according to any one of [A1] to [A74], wherein the cyclic peptide contains a cyclic portion composed of a 28- to 55-membered ring.
[A75-1] The method according to any one of [A1] to [A74], wherein the cyclic peptide contains a cyclic portion composed of a 28- to 49-membered ring.
[A75-2] The method according to any one of [A1] to [A74], wherein the cyclic peptide contains a cyclic portion composed of a 31- to 46-membered ring.
[A75-3] The method according to any one of [A1] to [A74], wherein the cyclic peptide contains a cyclic portion composed of a 34- to 43-membered ring.
[A76] The method according to any one of [A1] to [A75-3], wherein the cyclic peptide contains a cyclic portion composed of a 34- to 40-membered ring.
[A77] The method according to any one of [A1] to [A75-3], wherein the cyclic peptide contains a cyclic portion composed of a 34-membered ring.
[A77-1] The method according to [A77], wherein the cyclic portion composed of a 34-membered ring is a cyclic peptide composed of 10 residues of α-amino acids and 1 residue of an amino acid having a β-amino acid skeleton, with a total of 11 residues of amino acids.
[A77-2] The method according to any one of [A1] to [A77], wherein the cyclic peptide is a cyclic peptide with a structure represented by the following formula (I): 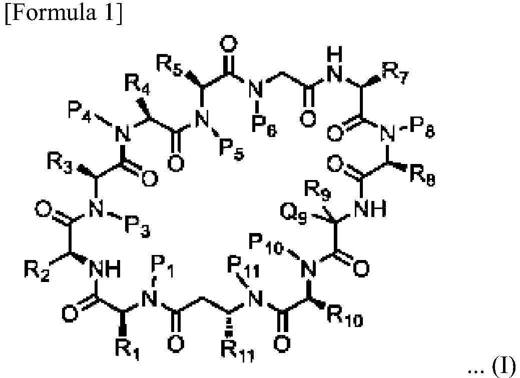 wherein, in the formula (I), P₁, P₃, P₅, P₆, P₁₀, and P₁₁ are each a C₁ to C₆ alkyl; P₄ is a C₁ to C₆ alkyl, or P₄ forms a 4- to 7-membered saturated heterocyclic ring together with the nitrogen atom to which P₄ is bonded, R₄, and the carbon atom to which R₄ is bonded; P₈ is a C₁ to C₆ alkyl, or P₈ forms a 4- to 7-membered saturated heterocyclic ring together with the nitrogen atom to which P₈ is bonded, R₈, and the carbon atom to which R₈ is bonded, the 4- to 7-membered saturated heterocyclic ring being optionally substituted by a C₁ to C₆ alkoxy; R₁, R₂, R₃, R₅, R₇, and R₁₀ are each a hydrogen atom, a C₁ to C₆ alkyl, a C₃ to C₆ cycloalkyl, or an aralkyl optionally having a substituent; R₄ is a hydrogen atom or a C₁ to C₆ alkyl, except when R₄ and P₄ form a 4- to 7-membered saturated heterocyclic ring; R₈ is a hydrogen atom or a C₁ to C₆ alkyl, except when R₈ and P₈ form a 4- to 7-membered saturated heterocyclic ring; R₉ forms a 3- to 7-membered saturated carbocyclic ring together with Q₉ and the carbon atom to which R₉ and Q₉ are bonded; and R₁₁ is a hydrogen atom, a C₁ to C₆ alkyl, a di-C₁ to C₆ alkylaminocarbonyl, or a 4- to 8-membered cyclic aminocarbonyl.
[A77-3] The method according to [A77-2], wherein P₁, P₃, P₅, P₆, P₁₀, and P₁₁ are each methyl or ethyl.
[A77-4] The method according to [A77-2] or [A77-3], wherein P₄ is methyl, or forms a 4-membered saturated heterocyclic ring together with the nitrogen atom to which P₄ is bonded, R₄, and the carbon atom to which R₄ is bonded.
[A77-5] The method according to any one of [A77-1] to [A77-4], wherein P₈ forms a 5-membered saturated heterocyclic ring together with the nitrogen atom to which P₈ is bonded, R₈, and the carbon atom to which R₈ is bonded, the 5-membered saturated heterocyclic ring being optionally substituted by a C₁ to C₆ alkoxy.
[A77-6] The method according to any one of [A77-1] to [A77-5], wherein R₁ and R₂ are each a C₁ to C₆ alkyl; R₃ is a hydrogen atom or a C₁ to C₆ alkyl; R₄ is a hydrogen atom, except when R₄ and P₄ form a 4- to 7-membered saturated heterocyclic ring; R₅ is a C₃ to C₆ cycloalkyl or benzyl optionally having a substituent; R₇ is phenylethyl optionally having a substituent; R₉ forms a 5-membered saturated carbocyclic ring together with Q₉ and the carbon atom to which R₉ and Q₉ are bonded; R₁₀ is a C₁ to C₆ alkyl or a C₃ to C₆ cycloalkyl; and R₁₁ is methyl, a di-C₁ to C₆ alkylaminocarbonyl, or a 6-membered cyclic aminocarbonyl.
[A77-7] The method according to any one of [A1] to [A77-6], wherein the cyclic peptide has a molecular weight (g/mol) of 1205 or more, 1206 or more, 1207 or more, 1208 or more, 1210 or more, 1220 or more, 1230 or more, 1250 or more, or 1300 or more, and 2800 or less, 2500 or less, 2000 or less, 1900 or less, 1800 or less, 1700 or less, or 1600 or less.
[A77-8] The method according to any one of [A1] to [A77-7], wherein the cyclic peptide has a molecular weight (g/mol) of 1300 or more and 1600 or less. [A78] The method according to any one of [A1] to [A77-8], wherein the cyclic peptide has a ClogP value of greater than 10.
[A79] The method according to any one of [A1] to [A77-8], wherein the cyclic peptide has a ClogP value of 11 or more, 12 or more, 13 or more, 14 or more, or 14.6 or more, and 25 or less, 24 or less, 23 or less, 22 or less, 21 or less, or 20 or less.
[A79-1] The method according to any one of [A1] to [A77-8], wherein the cyclic peptide has a ClogP value of 12 or more and 24 or less.
[A79-2] The method according to any one of [A1] to [A77-8], wherein the cyclic peptide has a ClogP value of 13 or more and 22 or less.
[A79-3] The method according to any one of [A1] to [A77-8], wherein the cyclic peptide has a ClogP value of 14 or more and 22 or less.
[A79-4] The method according to any one of [A1] to [A77-8], wherein the cyclic peptide has a ClogP value of 14.6 or more and 21 or less.
[A80] The method according to any one of [A1] to [A77-8], wherein the cyclic peptide has a ClogP value of 14.6 or more and 20 or less.
[A81] The method according to any one of [A1] to [A80], wherein the ClogP value of the cyclic peptide is greater than the ClogP value of cyclosporin A.
[A81-1] The method according to any one of [A1] to [A81], wherein the cyclic peptide does not contain a carboxy group.
[A81-2] The method according to any one of [A1] to [A81-1], wherein the cyclic peptide does not contain a hydroxy group.
[A81-3] The method according to any one of [A1] to [A81-2], wherein the cyclic peptide does not contain a phenolic hydroxy group.
[A81-4] The method according to any one of [A1] to [A81-3], wherein the cyclic peptide does not contain a pyridine ring.
[A81-5] The method according to any one of [A1] to [A81-4], wherein the cyclic peptide does not contain a xanthine ring moiety.
[A82] The method according to any one of [A1] to [A81-5], wherein the cocrystal further contains the solvent.
[A83] The method according to any one of [A21] to [A82], wherein the cocrystal further contains the solvent (A).
[A84] The method according to any one of [A1] to [A82], wherein the cocrystal further contains 2-methoxyethanol or 1,4-dioxane.
[A85] The method according to any one of [A21] to [A84], wherein the cocrystal further contains the solvent (B).
[A86] The method according to any one of [A1] to [A84], wherein the cocrystal further contains water.
[A87] The method according to any one of [A21] to [A82], wherein the cocrystal contains urea, the solvent (A), and the solvent (B).
[A88] The method according to any one of [A1] to [A82], wherein the cocrystal contains urea, 2-methoxyethanol or 1,4-dioxane, and water.
[A89] The method according to any one of [A21] to [A82], wherein the cocrystal contains nicotinamide, the solvent (A), and the solvent (B).
[A90] The method according to any one of [A1] to [A82], wherein the cocrystal contains nicotinamide, 2-methoxyethanol or 1,4-dioxane, and water.
[A91] The method according to any one of [A21] to [A82], wherein the cocrystal contains 2-furamide and the solvent (B).
[A92] The method according to any one of [A1] to [A82], wherein the cocrystal contains 2-furamide and water.
[A93] The method according to any one of [A21] to [A82], wherein the cocrystal contains 4-cyanopyridine and the solvent (B).
[A94] The method according to any one of [A1] to [A82], wherein the cocrystal contains 4-cyanopyridine and water.
[A95] A method for purifying a cyclic peptide, the method comprising producing a cocrystal containing a cyclic peptide and a compound by following the method according to any one of [A1] to [A94].
[A95-1] The method according to [A95], wherein the method for purifying the cyclic peptide is a method for reducing the content of an impurity in a mixture containing the cyclic peptide and the impurity, and the impurity is a peptide generated during a synthesis process of the cyclic peptide, which is an object to be purified.
[A95-2] The method according to [A95-1], wherein the impurity is a second cyclic peptide that is different from the cyclic peptide, which is an object to be purified.
[A95-3] The method according to [A95-2], wherein the impurity is a peptide having the number of amino acids twice the number of amino acids that the cyclic peptide, which is an object to be purified, has, a peptide having the number of amino acids three times the number of amino acids that the cyclic peptide, which is an object to be purified, has, or an isomer of the cyclic peptide, which is an object to be purified.
[A96] The method for purifying a cyclic peptide according to any one of [A95] to [A95-3], the method further comprising a step of reducing the content of the solvent (A) from a mixture containing the cocrystal.
[A97] The purification method according to [A96], wherein the step of reducing the content of the solvent (A) is a reduced pressure drying step.
[A98] The method for purifying a cyclic peptide according to any one of [A95] to [A97], the method further comprising a step of reducing the content of the compound with a melting point of 20°C or higher from a mixture containing the cocrystal.
[A99] The purification method according to [A98], wherein the step of reducing the content of the compound with a melting point of 20°C or higher comprises a liquid-liquid separation step using an organic solvent and water.
[B1] A method for determining a solvent, a compound, and crystallization conditions for use in a method for producing a cocrystal containing a cyclic peptide and a compound, the method comprising the following steps (a) to (c):
   (a) producing a cocrystal containing the cyclic peptide and the compound by following the method according to any one of [A1] to [A94];
   (b) analyzing the cocrystal by single crystal X-ray analysis and/or powder X-ray crystal diffraction; and
   (c) comparing the XRPD diffraction patterns of a substance that is to be the cocrystal and a reference substance.
[B2] A method for screening a cocrystal containing a cyclic peptide and a compound, the method comprising the following steps (a) and (b):
   (a) producing a cocrystal containing the cyclic peptide and the compound by following the method according to any one of [A1] to [A94]; and
   (b) analyzing the cocrystal by single crystal X-ray analysis and/or powder X-ray crystal diffraction.
[C1] A method for screening a method for producing a cocrystal containing a cyclic peptide and a compound, the method comprising the following steps (a) and (b):
   (a) producing a cocrystal containing the cyclic peptide and the compound by following the method according to any one of [A1] to [A94]; and
   (b) analyzing, when a cocrystal is obtained in (a), the cocrystal by single crystal X-ray analysis and/or powder X-ray crystal diffraction.
[D1] A method in which a cocrystal containing a cyclic peptide and a compound is produced, the method comprising a step of producing a cocrystal containing the cyclic peptide and the compound by following the method according to any one of [A1] to [A94].
[D2] A method for increasing the probability that a cocrystal containing a cyclic peptide and a compound is produced, the method comprising a step of producing a cocrystal containing the cyclic peptide and the compound by following the method according to any one of [A1] to [A94].
[E1] Use of urea, nicotinamide, 2-furamide, or 4-cyanopyridine in production of a cocrystal containing a cyclic peptide and urea, nicotinamide, 2-furamide, or 4-cyanopyridine.
[E2] Use of urea in production of a cocrystal containing a cyclic peptide and urea.
[E3] Use of nicotinamide in production of a cocrystal containing a cyclic peptide and nicotinamide.
[E4] Use of 2-furamide in production of a cocrystal containing a cyclic peptide and 2-furamide.
[E5] Use of 4-cyanopyridine in production of a cocrystal containing a cyclic peptide and 4-cyanopyridine.
[F1] A method for purifying a cyclic peptide, the method comprising a step of producing a cocrystal containing a cyclic peptide and a compound by following the method according to any one of [A1] to [A94], and a step of collecting the cocrystal by solid-liquid separation.
[F2] The method according to [F1], wherein the solid-liquid separation is centrifugation or filtration.
[F3] The method according to [F2], wherein the solid-liquid separation is filtration.
[F4] The method according to any one of [F1] to [F3], wherein the method for purifying a cyclic peptide is a method for reducing the content of an impurity in a mixture containing the cyclic peptide and the impurity, and the impurity is a peptide generated during a synthesis process of the cyclic peptide, which is an object to be purified.
[F5] The method according to [F4], wherein the impurity is a cyclic peptide that is different from the cyclic peptide, which is an object to be purified.
[F6] The method according to [F4] or [F5], wherein the impurity is a peptide having the number of amino acids twice the number of amino acids that the cyclic peptide, which is an object to be purified, has, a peptide having the number of amino acids three times the number of amino acids that the cyclic peptide, which is an object to be purified, has, or an isomer of the cyclic peptide, which is an object to be purified.
[G1] A cocrystal containing a cyclic peptide and a compound, which can be produced by following the method according to any one of [A1] to [A94].
[G2] A cocrystal containing a cyclic peptide and a compound, produced by following the method according to any one of [A1] to [A94].
[H1] A method for producing a cyclic peptide, the method comprising a step of producing cyclic peptide crystals containing urea.
[H2] A method for producing a cyclic peptide, the method comprising a step of producing cyclic peptide crystals containing nicotinamide.
[H3] A method for producing a cyclic peptide, the method comprising a step of producing cyclic peptide crystals containing 2-furamide.
[H4] A method for producing a cyclic peptide, the method comprising a step of producing cyclic peptide crystals containing 4-cyanopyridine.
[H5] The method according to any one of [H1] to [H4], wherein cyclic peptide crystals are produced by the method according to any one of [A1] to [A99], [B1], [C1], [D1], and [F1] to [F6].
[I1] The method according to any one of [A1] to [A62-6], wherein the cyclic peptide is (3S,9S,18S,21S,25S,28S,34S,36R)-9-(cyclohexylmethyl)-36-ethoxy-3-[2-[3-fluoro-4-(trifluoromethyl)phenyl]ethyl]-21,28-diisobutyl-7,10,13,16,22,26,29-heptamethyl-18-[(1S)-1-methylpropyl]-25-(piperidine-1-carbonyl)spiro[1,4,7,10,13,16,19,22,26,29,32-undecazabicyclo[32.3.0]heptatriacontane-31,1'-cyclopentane]-2,5,8,11,14,17,20,23,27,30,33-undecone.
[I1-1] The method according to [I1], wherein the cocrystal further contains the solvent.
[I1-2] The method according to any one of [I1] to [I1-1], wherein the cocrystal further contains the solvent (A).
[I1-3] The method according to any one of [I1] to [I1-2], wherein the cocrystal further contains 2-methoxyethanol or 1,4-dioxane.
[I1-4] The method according to any one of [I1] to [I1-3], wherein the cocrystal further contains the solvent (B).
[I1-5] The method according to any one of [I1] to [I1-4], wherein the cocrystal further contains water.
[I1-6] The method according to any one of [I1] to [I1-5], wherein the cocrystal contains urea, the solvent (A), and the solvent (B).
[I1-7] The method according to any one of [I1] to [I1-6], wherein the cocrystal contains urea, 2-methoxyethanol or 1,4-dioxane, and water.
[I2] The method according to any one of [A1] to [A62-6], wherein the cyclic peptide is (3S,9S,12S,17S,20S,23S,27S,30S,36S)-3-[2-[3-chloro-4-(trifluoromethyl)phenyl]ethyl]-9-(cyclohexylmethyl)-30-cyclopentyl-23-isobutyl-7,10,17,18,24,28,31-heptamethyl-20-[(1S)-1-methylpropyl]-27-(piperidine-1-carbonyl)spiro[1,4,7,10,15,18,21,24,28,31,34-undecazatricyclo[34.3.0.012,15]nonatriacontane-33,1'-cyclopentane]-2,5,8,11,16,19,22,25,29,32,35-undecone.
[I2-1] The method according to [I2], wherein the cocrystal further contains the solvent.
[I2-2] The method according to any one of [I2] to [I2-1], wherein the cocrystal further contains the solvent (A).
[I2-3] The method according to any one of [I2] to [I2-2], wherein the cocrystal further contains 2-methoxyethanol or 1,4-dioxane.
[I2-4] The method according to any one of [I2] to [I2-3], wherein the cocrystal further contains the solvent (B).
[I2-5] The method according to any one of [I2] to [I2-4], wherein the cocrystal further contains water.
[I2-6] The method according to any one of [I2] to [I2-5], wherein the cocrystal contains urea, the solvent (A), and the solvent (B).
[I2-7] The method according to any one of [I2] to [I2-6], wherein the cocrystal contains urea, 2-methoxyethanol or 1,4-dioxane, and water.
[I2-8] The method according to any one of [I2] to [I2-7], wherein the cocrystal contains nicotinamide, the solvent (A), and the solvent (B).
[I2-9] The method according to any one of [I2] to [I2-8], wherein the cocrystal contains nicotinamide, 2-methoxyethanol or 1,4-dioxane, and water.
[I2-10] The method according to any one of [I2] to [I2-9], wherein the cocrystal contains 2-furamide and the solvent (B).
[I2-11] The method according to any one of [I2] to [I2-10], wherein the cocrystal contains 2-furamide and water.
[I3] The method according to any one of [A1] to [A62-6], wherein the cyclic peptide is (3S,9S,12S,17S,20S,23S,27S,30S,36S)-3-[2-[3-chloro-4-(trifluoromethyl)phenyl]ethyl]-30-cyclopentyl-10-ethyl-23-isobutyl-7,17,18,24,28,3 1 -hexamethyl-20-[(1S)-1-methylpropyl]-27-(morpholine-4-carbonyl)-9-(p-tolylmethyl)spiro[1,4,7,10,15,18,21,24,28,31,34-undecazatricyclo[34.3.0.012,15]nonatriacontane-33,1'-cyclopentane]-2,5,8,11,16,19,22,25,29,32,35-undecone.
[I3-1] The method according to [13], wherein the cocrystal further contains the solvent.
[I3-2] The method according to any one of [I3] to [I3-1], wherein the cocrystal further contains the solvent (A).
[I3-3] The method according to any one of [I3] to [I3-2], wherein the cocrystal further contains 2-methoxyethanol or 1,4-dioxane.
[I3-4] The method according to any one of [I3] to [I3-3], wherein the cocrystal further contains the solvent (B).
[I3-5] The method according to any one of [I3] to [I3-4], wherein the cocrystal further contains water.
[I3-6] The method according to any one of [I3] to [I3-5], wherein the cocrystal contains urea, the solvent (A), and the solvent (B).
[I3-7] The method according to any one of [I3] to [I3-6], wherein the cocrystal contains urea, 2-methoxyethanol or 1,4-dioxane, and water.
[I3-8] The method according to any one of [I3] to [I3-7], wherein the cocrystal contains nicotinamide, the solvent (A), and the solvent (B).
[I3-9] The method according to any one of [I3] to [I3-8], wherein the cocrystal contains nicotinamide, 2-methoxyethanol or 1,4-dioxane, and water.
[I4] The method according to any one of [A1] to [A62-6], wherein the cyclic peptide is (3S,9S,125,17S,20S,23S,27S,30S,36S)-30-cyclopentyl-3-[2-[3,5-difluoro-4-(trifluoromethyl)phenyl]ethyl]-10-ethyl-23-isobutyl-N,N,7,17,18,24,28,31-octamethyl-20-[(1S)-1-methylpropyl]-2,5,8,11,16,19,22,25,29,32,35-undecaoxo-9-(p-tolylmethyl)spiro[1,4,7,10,15,18,21,24,28,31,34-undecazatricyclo[34.3.0.012,15]nonatriacontane-33,1'-cyclopentane]-27-carboxamide.
[I4-1] The method according to [I4], wherein the cocrystal further contains the solvent.
[I4-2] The method according to any one of [I4] to [I4-1], wherein the cocrystal further contains the solvent (A).
[I4-3] The method according to any one of [I4] to [14-2], wherein the cocrystal further contains 2-methoxyethanol or 1,4-dioxane.
[I4-4] The method according to any one of [I4] to [I4-3], wherein the cocrystal further contains the solvent (B).
[I4-5] The method according to any one of [I4] to [I4-4], wherein the cocrystal further contains water.
[I4-6] The method according to any one of [I4] to [I4-5], wherein the cocrystal contains urea, the solvent (A), and the solvent (B).
[I4-7] The method according to any one of [I4] to [I4-6], wherein the cocrystal contains urea, 2-methoxyethanol or 1,4-dioxane, and water.
[I5] The method according to any one of [A1] to [A62-6], wherein the cyclic peptide is (1S,4S,10S,13S,17S,20S,26S,28R,32S,38S,42Z)-20-cyclopentyl-28-ethoxy-32-[2-[3-methoxy-4-(trifluoromethyl)phenyl]ethyl]-N,N,2,14,18,21,24,36-octamethyl-10-[(1S)-1-methylpropyl]-3,9,12,15,19,22,25,31,34,37,45-undecaoxo-13-propyl-38-[[4-(trifluoromethyl)phenyl]methyl]spiro[2,8, 11,14,18,21,24,30,33,36,39-undecazatetracyclo[37.5.1.04,8.026,30]pentatetracont-42-ene-23,1'-cyclobutane]-17-carboxamide.
[I5-1] The method according to [I5], wherein the cocrystal further contains the solvent.
[I5-2] The method according to any one of [I5] to [I5-1], wherein the cocrystal further contains the solvent (A).
[I5-3] The method according to any one of [I5] to [I5-2], wherein the cocrystal further contains 2-methoxyethanol or 1,4-dioxane.
[I5-4] The method according to any one of [I5] to [I5-3], wherein the cocrystal further contains the solvent (B).
[I5-5] The method according to any one of [I5] to [I5-4], wherein the cocrystal further contains water.
[I5-6] The method according to any one of [I5] to [I5-5], wherein the cocrystal contains nicotinamide, the solvent (A), and the solvent (B).
[I5-7] The method according to any one of [I5] to [I5-6], wherein the cocrystal contains nicotinamide, 2-methoxyethanol or 1,4-dioxane, and water.
[I6] The method according to any one of [A1] to [A62-6], wherein the cyclic peptide is (2S,8S,12R,14S,20S,23S,27S,30S,36S,38Z)-20-cyclopentyl-8-[2-[3,5-difluoro-4-(trifluoromethyl)phenyl]ethyl]-12-ethoxy-27-isobutyl-N,N,4,19,22,26,32,35-octamethyl-30-[(1S)-1-methylpropyl]-3,6,9,15,18,21,25,28,31,34,42-undecaoxo-2-[[4-(trifluoromethyl)phenyl]methyl]spiro[1,4,7,10,16,19,22,26,29,32,35-undecazatricyclo[34.5.1.010,14]dotetracont-38-ene-17,1'-cyclopentane]-23-carboxamide.
[I6-1] The method according to [I6], wherein the cocrystal further contains the solvent.
[I6-2] The method according to any one of [I6] to [I6-1], wherein the cocrystal further contains the solvent (A).
[I6-3] The method according to any one of [I6] to [16-2], wherein the cocrystal further contains 2-methoxyethanol or 1,4-dioxane.
[I6-4] The method according to any one of [I6] to [I6-3], wherein the cocrystal further contains the solvent (B).
[I6-5] The method according to any one of [I6] to [I6-4], wherein the cocrystal further contains water.
[I6-6] The method according to any one of [I6] to [I6-5], wherein the cocrystal contains urea, the solvent (A), and the solvent (B).
[I6-7] The method according to any one of [I6] to [I6-6], wherein the cocrystal contains urea, 2-methoxyethanol or 1,4-dioxane, and water.
[I6-8] The method according to any one of [I6] to [16-7], wherein the cocrystal contains nicotinamide, the solvent (A), and the solvent (B).
[I6-9] The method according to any one of [I6] to [I6-8], wherein the cocrystal contains nicotinamide, 2-methoxyethanol or 1,4-dioxane, and water.
[I6-10] The method according to any one of [I6] to [I6-9], wherein the cocrystal contains 2-furamide and the solvent (B).
[I6-11] The method according to any one of [I6] to [I6-10], wherein the cocrystal contains 2-furamide and water.

### [Advantageous Effects of Invention]

According to one embodiment of the present invention, a method for producing a cocrystal of a cyclic peptide is provided. According to one embodiment of the present invention, a method for producing a cocrystal of a cyclic peptide containing a N-substituted amino acid residue is provided. According to one embodiment of the present invention, a method for producing a cocrystal of (3S,9S,12S,17S,20S,23S,27S,30S,36S)-30-cyclopentyl-3-[2-[3,5-difluoro-4-(trifluoromethyl)phenyl]ethyl]-10-ethyl-23-isobutyl-N,N,7,17,18,24,28,31-octamethyl-20-[(1S)-1-methylpropyl]-2,5,8,11,16,19,22,25,29,32,35-undecaoxo-9-(p-tolylmethyl)spiro[1,4,7,10,15,18,21,24,28,31,34-undecazatricyclo[34.3.0.012,15]nonatriacontane-33,1'-cyclopentane]-27-carboxamide is provided. According to one embodiment of the present invention, a method for producing a cocrystal of (1S,4S,10S,13S,17S,20S,26S,28R,32S,38S,42Z)-20-cyclopentyl-28-ethoxy-32-[2-[3-methoxy-4-(trifluoromethyl)phenyl]ethyl]-N,N,2,14,18,21,24,36-octamethyl-10-[(1S)-1-methylpropyl]-3,9,12,15,19,22,25,31,34,37,45-undecaoxo-13-propyl-38-[[4-(trifluoromethyl)phenyl]methyl]spiro[2,8,11,14,18,21,24,30,33,36,39-undecazatetracyclo[37.5.1.04,8.026,30]pentatetracont-42-ene-23,1'-cyclobutane]-17-carboxamide is provided.
According to one embodiment of the present invention, a method for producing a cocrystal of a cyclic peptide with excellent solubility in water is provided.

According to one embodiment of the present invention, a method for screening a cocrystal of a cyclic peptide is provided. According to one embodiment of the present invention, a method for screening a cocrystal of a cyclic peptide containing a N-substituted amino acid residue is provided.

According to one embodiment of the present invention, a method for screening a method for producing a cocrystal of a cyclic peptide is provided.

According to one embodiment of the present invention, a method for isolating and purifying a cyclic peptide as a cocrystal in order to produce the target cyclic peptide or salt thereof, or solvate thereof, without resorting to column chromatography is provided.

### [Brief Description of Drawings]

[Figure 1] Figure 1 shows the results of powder X-ray diffraction measurement of the cocrystal obtained in Example 2-1-1. The ordinate represents diffraction intensity, and the abscissa represents diffraction angle 2θ (°).
[Figure 2] Figure 2 shows the results of powder X-ray diffraction measurement of the hydrate crystal (Form C) of compound CP04. The ordinate represents diffraction intensity, and the abscissa represents diffraction angle 2θ (°).
[Figure 3] Figure 3 shows the results of powder X-ray diffraction measurement of the crystal of urea. The ordinate represents diffraction intensity, and the abscissa represents diffraction angle 2θ (°).
[Figure 4] Figure 4 shows the results of powder X-ray diffraction measurement of the cocrystal obtained in Example 2-1-2. The ordinate represents diffraction intensity, and the abscissa represents diffraction angle 2θ (°).
[Figure 5] Figure 5 shows the results of TG-DTA measurement of the cocrystal obtained in Example 2-1-2. The ordinate represents weight change and the abscissa represents temperature in (A), and the ordinate represents heat flow and the abscissa represents temperature in (B). The + mark and temperature shown on the graph of (B) indicate the melting point.
[Figure 6] Figure 6 shows the results of TG-DTA measurement of the hydrate crystal (Form C) of compound CP04. The ordinate represents weight change and the abscissa represents temperature in (A), and the ordinate represents heat flow and the abscissa represents temperature in (B).
[Figure 7] Figure 7 shows the results of TG-DTA measurement of urea. The ordinate represents weight change and the abscissa represents temperature in (A), and the ordinate represents heat flow and the abscissa represents temperature in (B). The + mark and temperature shown on the graph of (B) indicate the melting point.
[Figure 8] Figure 8 shows the crystal structure of the cocrystal of compound CP01 and urea described in Example 2-2-1. Urea is drawn with the Ball-and-Stick model and the others are drawn with the Capped Sticks model.
[Figure 9] Figure 9 shows the crystal structure of the cocrystal of compound CP01 and urea described in Example 2-2-2. Urea is drawn with the Ball-and-Stick model and the others are drawn with the Capped Sticks model.
[Figure 10] Figure 10 shows the crystal structure of the cocrystal of compound CP01 and urea described in Example 2-2-3. Urea is drawn with the Ball-and-Stick model and the others are drawn with the Capped Sticks model.
[Figure 11] Figure 11 shows the crystal structure of the cocrystal of compound CP02 and urea described in Example 2-3-1. Urea is drawn with the Ball-and-Stick model and the others are drawn with the Capped Sticks model.
[Figure 12] Figure 12 shows the crystal structure of the cocrystal of compound CP02 and urea described in Example 2-3-2. Urea is drawn with the Ball-and-Stick model and the others are drawn with the Capped Sticks model.
[Figure 13] Figure 13 shows the crystal structure of the cocrystal of compound CP02 and nicotinamide described in Example 2-3-3. Nicotinamide is drawn with the Ball-and-Stick model and the others are drawn with the Capped Sticks model.
[Figure 14] Figure 14 shows the crystal structure of the cocrystal of compound CP02 and 2-furamide described in Example 2-3-4. 2-Furamide is drawn with the Ball-and-Stick model and the others are drawn with the Capped Sticks model.
[Figure 15] Figure 15 shows the crystal structure of the cocrystal of Valinomycin and 4-cyanopyridine described in Example 2-4-2. 2-Furamide is drawn with the Ball-and-Stick model and the others are drawn with the Capped Sticks model.
[Figure 16] Figure 16 shows the results of powder X-ray diffraction measurement of compound CP03 obtained in Comparative Example 2-5-2. The ordinate represents diffraction intensity, and the abscissa represents diffraction angle 2θ (°).
[Figure 17] Figure 17 shows the results of TG-DTA measurement of compound CP03 obtained in Comparative Example 2-5-2. The ordinate represents weight change and the abscissa represents temperature in (A), and the ordinate represents heat flow and the abscissa represents temperature in (B). The + mark and temperature shown on the graph of (B) indicate the melting point.
[Figure 18] Figure 18 shows the results of diffraction measurement of the wet powder obtained in Example 2-5-4. The ordinate represents diffraction intensity, and the abscissa represents diffraction angle 2θ (°).
[Figure 19] Figure 19 shows the results of diffraction measurement of the wet powder obtained in Example 2-5-5. The ordinate represents diffraction intensity, and the abscissa represents diffraction angle 2θ (°).
[Figure 20] Figure 20 shows the crystal structure of the cocrystal of compound CP03 and nicotinamide described in Example 2-5-5. Nicotinamide is drawn with the Ball-and-Stick model and the others are drawn with the Capped Sticks model.
[Figure 21] Figure 21 shows the results of powder X-ray diffraction measurement of the wet powder obtained in Example 2-5-6. The ordinate represents diffraction intensity, and the abscissa represents diffraction angle 2θ (°).
[Figure 22] Figure 22 shows the results of powder X-ray diffraction measurement of the cocrystal of compound CP03 and urea obtained in Example 2-5-8. The ordinate represents diffraction intensity, and the abscissa represents diffraction angle 2θ (°).
[Figure 23] Figure 23 shows the results of TG-DTA measurement of the cocrystal of compound CP03 and urea obtained in Example 2-5-8. The ordinate represents weight change and the abscissa represents temperature in (A), and the ordinate represents heat flow and the abscissa represents temperature in (B). The + mark and temperature shown on the graph of (B) indicate the melting point.
[Figure 24] Figure 24 shows the results of powder X-ray diffraction measurement of the crystals obtained in Comparative Example 2-6-2. The ordinate represents diffraction intensity, and the abscissa represents diffraction angle 2θ (°).
[Figure 25] Figure 25 shows the results of TG-DTA measurement of the cocrystal obtained in Comparative Example 2-6-2. The ordinate represents weight change and the abscissa represents temperature in (A), and the ordinate represents heat flow and the abscissa represents temperature in (B). The + mark and temperature shown on the graph of (A) indicate the amount of reduction of volatile components, and the + marks and temperatures shown on the graph of (B) indicate the phase transition.
[Figure 26] Figure 26 shows the crystal structure of the cocrystal of CP05 and nicotinamide described in Example 2-6-3. Nicotinamide is drawn with the Ball-and-Stick model and the others are drawn with the Capped Stick model.
[Figure 27] Figure 27 shows the results of powder X-ray diffraction measurement of the cocrystal obtained in Example 2-6-4. The ordinate represents diffraction intensity, and the abscissa represents diffraction angle 2θ (°).
[Figure 28] Figure 28 shows the results of powder X-ray diffraction measurement of the crystal of nicotinamide. The ordinate represents diffraction intensity, and the abscissa represents diffraction angle 2θ (°).
[Figure 29] Figure 29 shows the results of TG-DTA measurement of the cocrystal obtained in Example 2-6-4. The ordinate represents weight change and the abscissa represents temperature in (A), and the ordinate represents heat flow and the abscissa represents temperature in (B). The + mark and temperature shown on the graph of (B) indicate the melting point.
[Figure 30] Figure 30 shows the results of TG-DTA measurement of nicotinamide. The ordinate represents weight change and the abscissa represents temperature in (A), and the ordinate represents heat flow and the abscissa represents temperature in (B). The + mark and temperature shown on the graph of (B) indicate the melting point.
[Figure 31] Figure 31 shows the results of powder X-ray diffraction measurement of the crystals obtained in Comparative Example 2-7-2. The ordinate represents diffraction intensity, and the abscissa represents diffraction angle 2θ (°).
[Figure 32] Figure 32 shows the crystal structure of the solvate crystal of CP06 described in Comparative Example 2-7-2. Acetone is drawn with the Ball-and-Stick model and the others are drawn with the Capped Stick model.
[Figure 33] Figure 33 shows the results of powder X-ray diffraction measurement of the cocrystal obtained in Example 2-7-4. The ordinate represents diffraction intensity, and the abscissa represents diffraction angle 2θ (°).
[Figure 34] Figure 34 shows the results of TG-DTA measurement of the cocrystal obtained in Example 2-7-4. The ordinate represents weight change and the abscissa represents temperature in (A), and the ordinate represents heat flow and the abscissa represents temperature in (B). The + mark and temperature shown on the graph of (B) indicate the melting point.
[Figure 35] Figure 35 shows the results of TG-DTA measurement of urea. The ordinate represents weight change and the abscissa represents temperature in (A), and the ordinate represents heat flow and the abscissa represents temperature in (B). The + mark and temperature shown on the graph of (B) indicate the melting point.
[Figure 36] Figure 36 shows the crystal structure of the cocrystal of CP06 and nicotinamide described in Example 2-7-6. Nicotinamide is drawn with the Ball-and-Stick model and the others are drawn with the Capped Stick model.
[Figure 37] Figure 37 shows the crystal structure of the cocrystal of CP06 and 2-furamide described in Example 2-7-7. 2-Furamide is drawn with the Ball-and-Stick model and the others are drawn with the Capped Stick model.

### [Description of Embodiments]

The term "room temperature" as used herein means a temperature of about 20°C to about 25°C.

The term "one or more" as used herein means the number of 1 or 2 or larger. When the term "one or more" is used in the context associated with a substituent of a group, the term means a number from 1 to the maximum number of substituents acceptable by the group. Specific examples of the term "one or more" include 1, 2, 3, 4, 5, 6, 7, 8, 9, 10, and/or larger numbers.

The term "one or more types" as used herein means one type or two or more types. Specific examples of "one or more types" include one type, two types, three types, four types, five types, six types, seven types, eight types, nine types, ten types, and/or a larger number of types.

As used herein, the term "to" that indicates a range includes values of both ends thereof. For example, "A to B" means the range of A or more and B or less.

As used herein, the term "about", when used in combination with a numeric value, means the value range of +10% and -10% of the numeric value.

The meaning of the term "and/or" as used herein includes any combination in which "and" and "or" are appropriately combined. Specifically, for example, the term "A, B, and/or C" includes the following seven variations; (i) A, (ii) B, (iii) C, (iv) A and B, (v) A and C, (vi) B and C, and (vii) A, B, and C.

The term "v/v%" as used herein represents % by volume (volume%). The term "v/v" as used herein represents volume ratio (volume/volume). For example, an acetone/water = 2/1 (v/v) solution means a solution containing acetone and water at a volume ratio of 2:1.

The term "v/w" as used herein represents ratio of volume (mL) to mass (g) (volume/weight). For example, adding 2 v/w of a certain liquid to a certain solid means adding the liquid to the solid at a ratio of 2 mL to 1 g.

The term "peptide" as used herein means one in which 2 or more amino acid residues are linked by an amide bond. Peptides having an ester bond in part of the main chain, such as depsipeptide, are also included in the term "peptide" herein.

The term "cyclic peptide" as used herein is a peptide having a cyclic structure composed of 4 or more amino acid residues. As an aspect of cyclization of the cyclic peptide, it may take any form, such as cyclization with a carbon-nitrogen bond such as an amide bond, cyclization with a carbon-oxygen bond such as an ester bond or ether bond, cyclization with a carbon-sulfur bond such as a thioether bond, cyclization with a carbon-carbon bond, or cyclization by heterocyclic construction. Among these, cyclization via a covalent bond such as an amide bond, a carbon-sulfur bond, or a carbon-carbon bond is preferred. Cyclization with an amide bond is more preferred, and the position of the carboxy group or amino group used for cyclization may be on the main chain or on a side chain. Most preferred is cyclization via an amide bond between a carboxy group on a side chain and the amino group of the main chain at the N-terminus.

The term "cyclization" of a peptide means formation of a cyclic portion containing 4 or more amino acid residues. The number of amino acids contained in the cyclic portion of the cyclic peptide herein is not particularly limited as long as it is 4 or more, and examples thereof include 4 to 16 residues, 8 to 16 residues, 9 to 15 residues, 10 to 16 residues, 11 to 15 residues, 11 to 13 residues, 11 to 12 residues, and 11 residues. It is preferably 9 to 15 residues, more preferably 10 to 14 residues, still more preferably 11 to 13 residues, and most preferably 11 residues. In another embodiment, examples thereof include 4 to 16 residues, 8 to 16 residues, 9 to 15 residues, 10 to 16 residues, 11 to 15 residues, 11 to 13 residues, 11 to 12 residues, and 11 residues, and it is preferably 9 to 15 residues, more preferably 10 to 14 residues, still more preferably 11 to 13 residues, and most preferably 11 residues. The method for converting a chain peptide to a cyclic peptide can be performed by carrying out an intramolecular bond formation reaction by the method described in Comprehensive Organic Transformations, A Guide to Functional Group Preparations, 3rd Edition (authored by R. C. Larock), March's Advanced Organic Chemistry: Reactions, Mechanisms, and Structure, 7th Edition (authored by M. B. Smith and J. March), or the like. It is also possible to further carry out a functional group conversion reaction after the bond formation reaction. Examples of the bond at the cyclization site of the cyclic peptide include a C(O)-N bond formed from a carboxylic acid and an amine, a C-O-C bond, a C(O)-O bond, and a C(S)-O bond via an oxygen atom, a C(O)-S bond, a C(S)-S bond, a C-S-S-C bond, a C-S-C bond, a C-S(O)-C bond, and a C-S(O₂)-C bond via a sulfur atom, and a C-N-C bond, a C=N-C bond, a N-C(O)-N bond, a N-C(S)N bond, and a C(S)-N bond via a nitrogen atom. Further examples thereof include a C-C bond formed by a coupling reaction catalyzed by a transition metal, such as the Suzuki reaction, the Heck reaction, and the Sonogashira reaction. Examples of the functional group conversion reaction that is further carried out after the bond formation reaction include an oxidation reaction or a reduction reaction. Specific examples thereof include a reaction in which a sulfur atom is oxidized and converted to a sulfoxide group or a sulfone group. Other examples thereof include a reduction reaction in which, among carbon-carbon bonds, a triple bond or a double bond is reduced and converted to a double bond or a single bond. Two amino acids may be bonded at the main chains of the amino acids to form a closed ring structure by a peptide bond, or a covalent bond may be formed between two amino acids via, for example, a bond between side chains or between a side chain and the main chain of the two amino acids.

As used herein, the term "cyclic portion" of the cyclic peptide means a ring-like portion formed by linkage of 4 or more amino acid residues.

The terms "cocrystal containing a cyclic peptide and a compound" and "cocrystal of a cyclic peptide" as used herein are defined as a crystal containing a cyclic peptide and a compound with a melting point of 20°C or higher, where the compound is a compound that does not contain a metal ion and does not ionically interact with the cyclic peptide. Accordingly, metal salts are not included in the compound, for example. The term "cocrystal containing a cyclic peptide and a compound" as used herein includes (i) a crystal composed of a cyclic peptide and a compound, (ii) a crystal composed of a cyclic peptide, a compound, and a solvent, and (iii) a mixture thereof. The "cocrystal containing a cyclic peptide and a compound" according to one embodiment of the present invention is preferably one selected from (i) to (iii) above, and more preferably (i) or (ii).

The solvate of a cyclic peptide as used herein is one in which the cyclic peptide and a solvent together form a molecular aggregate, and is not particularly limited as long as it is a solvate formed by a solvent. Examples of the solvate include a hydrate, an alcohol solvate (such as ethanol solvate, methanol solvate, 1-propanol solvate, and 2-propanol solvate), and not only a solvate formed with a single solvent such as formamide or dimethyl sulfoxide, but also a solvate formed with a plurality of solvents per one molecule of the cyclic peptide, or a solvate formed with a plurality of types of solvents per one molecule of the cyclic peptide.

The salt of a cyclic peptide as used herein is one in which the cyclic peptide forms a molecular aggregate together with a salt ionically bonded to an anion ionized from an acid or a cation ionized from a base, and is not particularly limited as long as it is a salt. Specific examples of the salt of the compound include hydrochloride; hydrobromide; hydroiodide; phosphate; phosphonate; sulfate; sulfonate such as methanesulfonate and p-toluenesulfonate; carboxylate such as acetate, citrate, malate, tartrate, succinate, salicylate, and adipate; alkali metal salts such as sodium salt and potassium salt; alkaline earth metal salts such as magnesium salt and calcium salt; and ammonium salts such as ammonium salt, alkylammonium salt, dialkylammonium salt, trialkylammonium salt, and tetraalkylammonium salt. These salts can be produced by, for example, bringing the compound into contact with an acid or a base.

The crystal of a solvate of a cyclic peptide as used herein means a crystal composed of the cyclic peptide and the solvent. In other words, the term "solvate of a cyclic peptide" as used herein is clearly distinguished from the term "cocrystal containing a cyclic peptide and a compound", which contains a cyclic peptide and a compound other than the solvent.

The term "amino acid" as used herein includes a natural amino acid and a non-natural amino acid. The term "amino acid" as used herein may mean an amino acid residue. The term "natural amino acid" as used herein refers to Gly, Ala, Ser, Thr, Val, Leu, Ile, Phe, Tyr, Trp, His, Glu, Asp, Gln, Asn, Cys, Met, Lys, Arg, and Pro (all in L-form except for Gly). Examples of the non-natural amino acid include, but are not particularly limited to, a β-amino acid, a D-type amino acid, a N-substituted amino acid, an α,α-disubstituted amino acid, an amino acid having a side chain different from that of natural amino acids, and a hydroxycarboxylic acid. As used herein, the amino acid accepts an arbitrary conformation. The selection of a side chain of the amino acid is not particularly limited, and the side chain is freely selected from, in addition to a hydrogen atom, for example, an alkyl group, an alkenyl group, an alkynyl group, an aryl group, a heteroaryl group, an aralkyl group, a heteroarylalkyl group, a cycloalkyl group, a spiro-bonded cycloalkyl group. A substituent may be added to each of the side chains. Such a substituent is not limited either, and can be one or two or more substituents each independently freely selected from any substituents including, for example, a halogen atom, an O atom, a S atom, a N atom, a B atom, a Si atom, or a P atom. That is, examples of the side chain include an alkyl group, an alkoxy group, an alkoxyalkyl group, an alkenyl group, an alkynyl group, an aryl group, a heteroaryl group, an aralkyl group, or a cycloalkyl group that is optionally substituted, or oxo, aminocarbonyl, and a halogen atom. In one non-limiting aspect, the amino acid as used herein may be a compound having a carboxy group and an amino group in the same molecule. Even in this case, a compound in which the nitrogen atom of an amino group and an arbitrary atom of a side chain together form a ring (such as proline, hydroxyproline, and azetidine-2-carboxylic acid) is also included in the amino acid as used herein.

As used herein, the term "optionally substituted" means that a group is optionally substituted by an arbitrary substituent. Furthermore, a substituent may be added to each of the substituents. Such a substituent is not limited either, and can be one or two or more substituents each independently freely selected from any substituents including, for example, a halogen atom, an oxygen atom, a sulfur atom, a nitrogen atom, a boron atom, a silicon atom, or a phosphorus atom. Examples of the substituent include alkyl, alkoxy, fluoroalkyl, fluoroalkoxy, oxo, aminocarbonyl, alkylsulfonyl, alkylsulfonylamino, cycloalkyl, aryl, heteroaryl, heterocyclyl, arylalkyl, heteroarylalkyl, halogen, nitro, amino, monoalkylamino, dialkylamino, cyano, carboxy, alkoxycarbonyl, and formyl.

The term "halogen" as used herein refers to F, Cl, Br, or I, more preferably refers to F or Cl, and still more preferably refers to F.

The "alkyl" as used herein is a monovalent group derived from an aliphatic hydrocarbon by removing any one hydrogen atom, and has a subset of hydrocarbyl or hydrocarbon group structures that do not contain a heteroatom (which refers to an atom other than carbon and hydrogen atoms) or an unsaturated carbon-carbon bond, and contain hydrogen and carbon atoms in the backbone. The alkyl includes not only a linear form but also a branched form. Preferred examples of the alkyl include alkyl having 1 to 20 carbon atoms (C₁-C₂₀; hereinafter, "Cₚ-C_{q}" means that the number of carbon atoms is p to q), preferably C₁-C₁₀ alkyl, and more preferably C₁-C₆ alkyl. Specific examples of the alkyl include methyl, ethyl, n-propyl, i-propyl, n-butyl, s-butyl, t-butyl, isobutyl (2-methylpropyl), n-pentyl, s-pentyl (1-methylbutyl), t-pentyl (1,1-dimethylpropyl), neopentyl (2,2-dimethylpropyl), isopentyl (3-methylbutyl), 3-pentyl (1-ethylpropyl), 1,2-dimethylpropyl, 2-methylbutyl, n-hexyl, 1,1,2-trimethylpropyl, 1,2,2-trimethylpropyl, 1,1,2,2-tetramethylpropyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, and 2-ethylbutyl.

As used herein, the term "alkynyl" is a monovalent group having at least one triple bond (two adjacent SP carbon atoms). The alkynyl includes not only a linear form but a branched form. Preferred examples of the alkynyl include C₂-C₁₀ alkynyl, and more preferably C₂-C₆ alkynyl. Specific examples thereof include ethynyl, 1-propynyl, propargyl, 3-butynyl, pentynyl, hexynyl, 3-phenyl-2-propynyl, 3-(2'-fluorophenyl)-2-propynyl, 2-hydroxy-2-propynyl, 3-(3-fluorophenyl)-2-propynyl, and 3-methyl-(5-phenyl)-4-pentynyl.

As used herein, the term "alkenyl" is a monovalent group having at least one double bond (two adjacent SP2 carbon atoms). Depending on the conformation of the double bond and a substituent (if present), the geometric morphology of the double bond can assume entgegen (E) or zusammen (Z) and cis or trans conformations. The alkenyl includes not only a linear form but a branched form. Preferred examples of the alkenyl include C₂-C₁₀ alkenyl, and more preferred examples thereof include C₂-C₆ alkenyl. Specific examples thereof include vinyl, allyl, 1-propenyl, 2-propenyl, 1-butenyl, 2-butenyl (including cis and trans), 3-butenyl, pentenyl, 3-methyl-2-butenyl, and hexenyl.

The term "aryl" as used herein means a monovalent aromatic hydrocarbon ring and an aromatic hydrocarbon ring group. Preferred examples of the aryl include C₆-C₁₀ aryl. Specific examples of the aryl include phenyl and naphthyl (for example, 1-naphthyl and 2-naphthyl).

The term "heteroaryl" as used herein means an aromatic cyclic monovalent group containing 1 to 5 heteroatoms in addition to a carbon atom, and an aromatic heterocyclic group. The ring may be a monocyclic ring or a condensed ring with another ring, and may be partially saturated. The number of atoms constituting the ring of heteroaryl is preferably 5 to 10 (5- to 10-membered heteroaryl), and more preferably 5 to 7 (5- to 7-membered heteroaryl). Specific examples of the heteroaryl include furyl, thienyl, pyrrolyl, imidazolyl, pyrazolyl, thiazolyl, isothiazolyl, oxazolyl, isoxazolyl, oxadiazolyl, thiadiazolyl, triazolyl, tetrazolyl, pyridyl, pyrimidyl, pyridazinyl, pyrazinyl, triazinyl, benzofuranyl, benzothienyl, benzothiadiazolyl, benzothiazolyl, benzoxazolyl, benzoxadiazolyl, benzimidazolyl, benzotriazolyl, indolyl, isoindolyl, indazolyl, azaindolyl, quinolyl, isoquinolyl, cinnolinyl, quinazolinyl, quinoxalinyl, benzodioxolyl, indolizinyl, and imidazopyridyl, pyrazolopyridyl, imidazopyridyl, triazolopyridyl, pyrrolopyrazinyl, and flopyridyl.

The term "aralkyl (arylalkyl)" as used herein means a group in which at least one hydrogen atom of the "alkyl" as defined above is replaced with the "aryl" as defined above. As the aralkyl, C₇-C₁₄ aralkyl is preferred, and C₇-C₁₀ aralkyl is more preferred. Specific examples of the aralkyl include benzyl, phenethyl, and 3-phenylpropyl.

The term "heteroarylalkyl" as used herein means a group in which at least one hydrogen atom of the "alkyl" as defined above is replaced with the "heteroaryl" as defined above. As the heteroarylalkyl, 5- to 10-membered heteroaryl-C₁-C₆ alkyl is preferred, and 5- to 10-membered heteroaryl-C₁-C₂ alkyl is more preferred. Specific examples of the heteroarylalkyl include 3-thienylmethyl, 4-thiazolylmethyl, 2-pyridylmethyl, 3-pyridylmethyl, 4-pyridylmethyl, 2-(2-pyridyl)ethyl, 2-(3-pyridyl)ethyl, 2-(4-pyridyl)ethyl, 2-(6-quinolyl)ethyl, 2-(7-quinolyl)ethyl, 2-(6-indolyl)ethyl, 2-(5-indolyl)ethyl, and 2-(5-benzofuranyl)ethyl.

The term "cycloalkyl" as used herein means a saturated or partially saturated cyclic monovalent aliphatic hydrocarbon group and includes a monocyclic ring, a bicyclo ring, and a spiro ring. Preferred examples of the cycloalkyl include C₃-C₈ cycloalkyl. Specific examples thereof include cyclopropyl, cyclobutyl, cyclopentyl, cyclohexyl, cycloheptyl, cyclooctyl, bicyclo[2.2.1]heptyl, and spiro[3.3]heptyl.

The term "alkoxy" as used herein means an oxy group to which the "alkyl" as defined above is bonded. Preferred examples of the alkoxy include C₁-C₆ alkoxy. Specific examples of the alkoxy include methoxy, ethoxy, 1-propoxy, 2-propoxy, n-butoxy, i-butoxy, s-butoxy, t-butoxy, pentyloxy, and 3-methylbutoxy.

The term "alkoxyalkyl" as used herein means a group in which one or more hydrogen of the "alkyl" as defined above are replaced with the "alkoxy" as defined above. As the alkoxyalkyl, C₁-C₆ alkoxy-C₁-C₆ alkyl is preferred, and C₁-C₆ alkoxy-C₁-C₂ alkyl is more preferred. Specific examples of the alkoxyalkyl include methoxymethyl, ethoxymethyl, 1-propoxymethyl, 2-propoxymethyl, n-butoxymethyl, i-butoxymethyl, s-butoxymethyl, t-butoxymethyl, pentyloxymethyl, 3-methylbutoxymethyl, 1-methoxyethyl, 2-methoxyethyl, and 2-ethoxyethyl.

As used herein, the term "amino" means -NH₂ in the narrow sense and means -NRR' in the broad sense. In this context, R and R' are each independently selected from hydrogen, alkyl, alkenyl, alkynyl, cycloalkyl, heterocyclyl, aryl, and heteroaryl, and preferred examples of the amino include -NH₂, mono-C₁-C₆ alkylamino, di-C₁-C₆ alkylamino, and 4- to 8-membered cyclic amino. Alternatively, R and R' form a ring together with the nitrogen atom to which they are bonded. More preferred examples of the amino include 4- to 8-membered cyclic amino.

The term "monoalkylamino" as used herein means a group of the "amino" as defined above in which R is hydrogen and R' is the "alkyl" as defined above. Preferred examples of the monoalkylamino include mono-C1-C6 alkylamino. Specific examples of the monoalkylamino include methylamino, ethylamino, n-propylamino, i-propylamino, n-butylamino, s-butylamino, and t-butylamino.

The term "dialkylamino" as used herein means a group of the "amino" as defined above in which R and R' are each independently the "alkyl" as defined above. Preferred examples of the dialkylamino include di-C₁-C₆ alkylamino. Specific examples of the dialkylamino include dimethylamino and diethylamino.

The term "alkylsulfonylamino" as used herein means a group in which sulfonyl is bonded to the "amino" as defined above. Preferred examples thereof include C₁-C₆ alkylsulfonyl-NH- and (C₁-C₆ alkylsulfonyl-)₂-N-. Specific examples of the aminoalkylsulfonyl include methylsulfonylamino, ethylsulfonylamino, bis(methylsulfonyl)amino, and bis(ethylsulfonyl)amino.

The term "aminocarbonyl" as used herein means a carbonyl group to which the "amino" as defined above is bonded. Preferred examples of the aminocarbonyl include -CONH₂, mono-C₁-C₆ alkylaminocarbonyl, di-C₁-C₆ alkylaminocarbonyl, and 4- to 8-membered cyclic aminocarbonyl. Specific examples of the aminocarbonyl include -CONH₂, dimethylaminocarbonyl, 1-azetidinylcarbonyl, 1-pyrrolidinylcarbonyl, 1-piperidinylcarbonyl, 1-piperazinylcarbonyl, 4-morpholinylcarbonyl, 3-oxazolidinylcarbonyl, 1,1-dioxidothiomorpholinyl-4-ylcarbonyl, and 3-oxa-8-azabicyclo[3.2.1]octan-8-ylcarbonyl.

The term "amino acid residue" as used herein means a monovalent or divalent radical derived from an amino acid and preferably bonded by a peptide bond.

The term "side chain of an amino acid" as used herein means, in the case of an α-amino acid, an atomic group bonded to carbon (α-carbon) to which an amino group and a carboxy group are bonded, other than the amino group and the carboxy group. For example, the methyl group of Ala is a side chain of the amino acid. In the case of a β-amino acid, an atomic group bonded to the α-carbon and/or the β-carbon, other than the amino group bonded to the β-carbon and the carboxy group bonded to the α-carbon, can be a side chain of the amino acid. Also, in the case of a γ-amino acid, an atomic group bonded to the α-carbon, the β-carbon, and/or the γ-carbon, other than the amino group bonded to the γ-carbon and the carboxy group bonded to the α-carbon, can be a side chain of the amino acid.

The term "main chain of an amino acid" as used herein means, in the case of an α-amino acid, a chain portion constituted by the amino group, the α-carbon, and the carboxy group; in the case of a β-amino acid, a chain portion constituted by the amino group, the β-carbon, the α-carbon, and the carboxy group; and in the case of a γ-amino acid, a chain portion constituted by the amino group, the γ-carbon, the β-carbon, the α-carbon, and the carboxy group. Also, the term "α-amino acid skeleton" means a chain portion constituted by the amino group, the α-carbon, and the carboxy group; the term "β-amino acid skeleton" means a chain portion constituted by the amino group, the β-carbon, the α-carbon, and the carboxy group; and the term "γ-amino acid skeleton" means a chain portion constituted by the amino group, the γ-carbon, the β-carbon, the α-carbon, and the carboxy group. As used herein, an amino acid having a "β-amino acid skeleton" as a whole or as a substructure may be referred to as an "amino acid having a β-amino acid skeleton". For example, aspartic acid has a chain portion constituted by the amino group, the β-carbon, the α-carbon, and the carboxy group (β-amino acid skeleton), and thus falls under the category of the "amino acid having a β-amino acid skeleton".

The term "main chain of a peptide" as used herein means a structure in which multiple amino acids are linked by an amide bond. The terms "main chain of a cyclic peptide" and "main chain of a cyclic portion" as used herein mean a structure contained in the cyclic portion of a cyclic peptide in which amino acid residues are bonded to adjacent amino acid residues in the chain via an amide bond. The "main chain of a peptide", the "main chain of a cyclic peptide", and the "main chain of a cyclic portion" may in part contain another bond, such as an ester bond, in place of an amide bond. Also, the "main chain of a cyclic peptide" and the "main chain of a cyclic portion" may include a bond exemplified herein as the bond at the cyclization site of a cyclic peptide, or a bond formed by the cyclization formation reaction of a peptide.

The term "N-substituted amino acid" as used herein means an amino acid in which the amino group contained in the amino acid is substituted, that is, represented by -NHR (R represents alkyl, alkenyl, alkynyl, aryl, heteroaryl, aralkyl, or cycloalkyl optionally having a substituent, and one or two non-adjacent methylene groups in any of these groups is optionally substituted by an oxygen atom, a carbonyl group (-CO-), or a sulfonyl group (-SO₂-); and a carbon chain bonded to the N atom and the carbon atom at position α may form a ring, as in proline.). The "N-substituted amino acid" as used herein may be a N-alkylamino acid, and preferred examples thereof include a N-C₁-C₆ alkylamino acid, more preferred examples thereof include a N-C₁-C₄ alkylamino acid, and most preferred examples thereof include a N-ethylamino acid or a N-methylamino acid although it is not limited to these. Preferred examples of the "N-substituted amino acid" as used herein include a N-substituted amino acid in which the amino group contained in the "main chain of an amino acid" is substituted.

The term "N-unsubstituted amino acid" as used herein means an amino acid in which an amino group contained in the amino acid is not substituted, that is, represented by -NH₂. Preferred examples of the "N-unsubstituted amino acid" as used herein include a N-unsubstituted amino acid in which the amino group contained in the "main chain of an amino acid" is not substituted.

In the case where an amino acid constituting a cyclic peptide as used herein is aspartic acid, or an amino acid in which one or more of hydrogen on the α-carbon and hydrogen on the β-carbon of aspartic acid are substituted, or an amino acid in which the amino group contained in its "main chain of the amino acid" is substituted, the carboxy group bonded to the α-carbon contained in the "main chain of the amino acid" may be contained in the "main chain of a cyclic peptide". On the other hand, a carboxy group bonded to the β-carbon contained in the "side chain of an amino acid" may be contained in the "main chain of a cyclic peptide", in which case the "β-amino acid skeleton" is contained in the "main chain of a cyclic peptide".

As used herein, the term "number of amino acids" or "number of amino acid residues" refers to the number of amino acid residues constituting a peptide, and means the number of amino acid units generated upon cleavage of amide bonds, ester bonds, and bonds of cyclized portions that link the amino acids. For example, the number of amino acids or the number of amino acid residues of the cyclic peptide in which the cyclic portion is composed of 10 amino acid residues and the linear portion is composed of 1 amino acid residue is 11.

The term "containing an amino acid in the cyclic portion of a cyclic peptide" as used herein means that the main chain of the cyclic portion of a cyclic peptide contains an amino acid residue.

The term "carbonyl group" as used herein refers to one of the functional groups constituting compounds, represented by the chemical formula -C(=O)-.

The term "ester" as used herein refers to a compound obtained by the condensation reaction of an oxo acid of an organic acid or inorganic acid with a compound containing a hydroxy group, such as an alcohol or phenol, and has a structure represented by -C(=O)-O-.

The term "anhydride" as used herein refers to a compound formed by dehydration condensation of two molecules of an oxo acid, and has a structure represented by -C(=O)-O-C(=O)-.

As used herein, the molecular weight of a cyclic peptide or a solvent is represented in g/mol, unless otherwise stated. Also, in any case where a crystal of a cyclic peptide is a crystal of the free form of the cyclic peptide, a crystal of a solvate of the cyclic peptide, or a mixture thereof, the "molecular weight of the cyclic peptide" is on the basis of the molecular weight of the free form of the cyclic peptide.

A cyclic peptide according to one embodiment of the present invention may have the following characteristic (i):
(i) a characteristic of containing a cyclic portion composed of 8 to 16 residues of amino acids in total. That is, the cyclic peptide according to one embodiment of the present invention may contain a cyclic portion composed of 8 to 16 residues of amino acids in total. The cyclic peptide according to one embodiment of the present invention may contain a cyclic portion composed of 8 to 16 residues, 9 to 15 residues, 10 to 14 residues, 11 to 13 residues, or 11 to 12 residues. The cyclic peptide of the present invention preferably contains a cyclic portion composed of 8 to 16 residues, more preferably contains a cyclic portion composed of 11 to 13 residues, and most preferably contains a cyclic portion composed of 11 to 12 residues.

A cyclic peptide according to one embodiment of the present invention may have the following characteristic (i):
(i) a characteristic of containing a cyclic portion composed of 8 to 16 residues of amino acids in total, with a total number of amino acids being 8 to 20 residues. That is, the cyclic peptide according to one embodiment of the present invention may contain a cyclic portion composed of 8 to 16 residues of amino acids in total, with a total number of amino acids being 8 to 20 residues. The cyclic peptide according to one embodiment of the present invention may contain a cyclic portion composed of 8 to 16 residues, 9 to 15 residues, 10 to 14 residues, 11 to 13 residues, or 11 to 12 residues of amino acids in total, with a total number of amino acids being 8 to 20 residues, 9 to 18 residues, 10 to 16 residues, 11 to 15 residues, 11 to 13 residues, or 11 to 12 residues. The cyclic peptide of the present invention preferably contains a cyclic portion composed of 8 to 16 residues, with a total number of amino acids being 8 to 20 residues, more preferably contains a cyclic portion composed of 11 to 13 residues, with a total number of amino acids being 11 to 15 residues, and most preferably contains a cyclic portion composed of 11 to 12 residues of amino acids in total, with a total number of amino acids being 11 to 12 residues.

In the cyclic peptide according to one embodiment of the present invention, the cyclic peptide contains a cyclic portion composed of 7 to 15 residues of natural amino acids and non-natural amino acids in total, with a total number of natural amino acids and non-natural amino acids being 9 to 18 residues.
The cyclic peptide preferably contains a cyclic portion composed of 8 to 14 residues of natural amino acids and non-natural amino acids in total, with a total number of natural amino acids and non-natural amino acids being 10 to 16 residues.
The cyclic peptide more preferably contains a cyclic portion composed of 9 to 13 residues of natural amino acids and non-natural amino acids in total, with a total number of natural amino acids and non-natural amino acids being 10 to 14 residues.
The cyclic peptide still more preferably contains a cyclic portion composed of 10 to 13 residues of natural amino acids and non-natural amino acids in total, with a total number of natural amino acids and non-natural amino acids being 11 to 14 residues.
The cyclic peptide still more preferably contains a cyclic portion composed of 11 to 13 residues of natural amino acids and non-natural amino acids in total, with a total number of natural amino acids and non-natural amino acids being 11 to 14 residues.
The cyclic peptide most preferably contains a cyclic portion composed of 11 residues of natural amino acids and non-natural amino acids in total, with a total number of natural amino acids and non-natural amino acids being 11 residues.

A cyclic peptide according to one embodiment of the present invention may further have, in addition to the characteristic (i) described above, the following characteristic (ii):
(ii) a characteristic of containing at least one N-substituted amino acid. That is, the cyclic peptide according to one embodiment of the present invention may contain a cyclic portion composed of 8 to 16 residues of amino acids in total, with a total number of amino acids being 8 to 20 residues, and also contains at least one N-substituted amino acid. The cyclic peptide according to one embodiment of the present invention may contain a cyclic portion composed of 8 to 16 residues, 9 to 15 residues, 10 to 14 residues, 11 to 13 residues, or 11 to 12 residues of amino acids in total, with a total number of amino acids being 8 to 20 residues, 9 to 18 residues, 10 to 16 residues, 11 to 15 residues, 11 to 13 residues, or 11 to 12 residues, and may also have a characteristic of containing at least 2, 3, 4, 5, or 6 N-substituted amino acids. The cyclic peptide according to one embodiment of the present invention preferably contains a cyclic portion composed of 8 to 16 residues of amino acids in total, with a total number of amino acids being 8 to 20 residues, and also contains at least one N-substituted amino acid. The cyclic peptide according to one embodiment of the present invention more preferably contains a cyclic portion composed of 11 to 13 residues of amino acids in total, with a total number of amino acids being 11 to 15 residues, and also contains at least 5 N-substituted amino acids. The cyclic peptide according to one embodiment of the present invention most preferably contains a cyclic portion composed of 11 to 12 residues of amino acids in total, with a total number of amino acids being 11 to 12 residues, and also contains at least 6 N-substituted amino acids.

A cyclic peptide according to one embodiment of the present invention may contain at least one N-substituted amino acid. The cyclic peptide according to one embodiment of the present invention may contain at least 2, 3, 4, 5, or 6 N-substituted amino acids. The cyclic peptide of the present invention preferably contains at least one N-substituted amino acid, more preferably contains at least 5 N-substituted amino acids, and most preferably contains at least 6 N-substituted amino acids.

A cyclic peptide according to one embodiment of the present invention may further have, in addition to the characteristics (i) and (ii) described above, the following characteristic (iii):
(iii) a characteristic of having a molecular weight (g/mol) of 1110 or more and 3000 or less.

A cyclic peptide according to one embodiment of the present invention may have a molecular weight (g/mol) of 1110 or more and 3000 or less. The cyclic peptide according to one embodiment of the present invention may have a molecular weight (g/mol) of 1204 or more, 1300 or more, or 1400 or more, and 2500 or less, 2000 or less, 1800 or less, 1700 or less, 1600 or less, or 1500 or less. The cyclic peptide according to one embodiment of the present invention preferably has a molecular weight (g/mol) of 1110 or more and 3000 or less, more preferably 1204 or more and 2000 or less, and most preferably 1300 or more and 1700 or less.

A cyclic peptide according to one embodiment of the present invention may contain a cyclic portion composed of 7 to 15 residues, 8 to 14 residues, 9 to 13 residues, 10 to 13 residues, 11 to 13 residues, 11 to 12 residues, or 11 residues of natural amino acids and non-natural amino acids in total, with a total number of natural amino acids and non-natural amino acids being 9 to 18 residues, 10 to 16 residues, 10 to 14 residues, 11 to 14 residues, 11 to 13 residues, 11 to 12 residues, or 11 residues. The cyclic peptide according to one embodiment of the present invention preferably contains a cyclic portion composed of 7 to 15 residues of natural amino acids and non-natural amino acids in total, with a total number of natural amino acids and non-natural amino acids being 9 to 18 residues. The cyclic peptide according to one embodiment of the present invention more preferably contains a cyclic portion composed of 11 to 13 residues of natural amino acids and non-natural amino acids in total, with a total number of natural amino acids and non-natural amino acids being 11 to 14 residues. The cyclic peptide according to one embodiment of the present invention most preferably contains a cyclic portion composed of 11 residues of natural amino acids and non-natural amino acids in total, with a total number of natural amino acids and non-natural amino acids being 11 residues.

A cyclic peptide according to one embodiment of the present invention may contain at least 3 residues, 4 residues, or 5 residues of N-substituted amino acids. The cyclic peptide according to one embodiment of the present invention preferably contains at least 3 residues of N-substituted amino acids. The cyclic peptide according to one embodiment of the present invention more preferably contains at least 4 residues of N-substituted amino acids. The cyclic peptide according to one embodiment of the present invention most preferably contains at least 5 residues of N-substituted amino acids.

A cyclic peptide according to one embodiment of the present invention may contain at least one residue, 2 residues, or 3 residues of N-unsubstituted amino acids. The cyclic peptide according to one embodiment of the present invention preferably contains at least one residue of N-unsubstituted amino acid. The cyclic peptide according to one embodiment of the present invention more preferably contains at least 2 residues of N-unsubstituted amino acids. The cyclic peptide according to one embodiment of the present invention most preferably contains at least 3 residues of N-unsubstituted amino acids.

The N-substituted amino acid contained in the cyclic peptide according to one embodiment of the present invention may be a N-alkylamino acid, may be a N-methylamino acid or a N-ethylamino acid in one other embodiment, and may be a N-methylamino acid in one other embodiment. The N-substituted amino acid contained in the cyclic peptide of the present invention is preferably a N-alkylamino acid, more preferably a N-methylamino acid or a N-ethylamino acid, and most preferably a N-methylamino acid.

A cyclic peptide according to one embodiment of the present invention may contain at least one β-amino acid. The cyclic peptide according to one embodiment of the present invention preferably contains at least one β-amino acid.

A cyclic portion of a cyclic peptide according to one embodiment of the present invention may contain at least one β-amino acid. In other words, the cyclic peptide according to one embodiment of the present invention may contain at least one β-amino acid skeleton in the cyclic portion. The cyclic peptide according to one embodiment of the present invention preferably contains at least one β-amino acid skeleton in the cyclic portion.

A cyclic peptide according to one embodiment of the present invention may contain a cyclic portion composed of a 28- to 55-, 28- to 49-, 31- to 46-, 34- to 43-, 34- to 40-, 34- to 37-, or 34-membered ring. The cyclic peptide according to one embodiment of the present invention preferably contains a cyclic portion composed of a 28- to 55-membered ring. The cyclic peptide according to one embodiment of the present invention more preferably contains a cyclic portion composed of a 34- to 40-membered ring. The cyclic peptide according to one embodiment of the present invention most preferably contains a cyclic portion composed of a 34-membered ring.

In a cyclic peptide according to one embodiment of the present invention, the cyclic portion composed of a 34-membered ring is a cyclic peptide composed of 10 residues of α-amino acids and 1 residue of an amino acid having a β-amino acid skeleton, with a total of 11 residues of amino acids.

A cyclic peptide according to one embodiment of the present invention is a cyclic peptide with a structure represented by the following formula (I): 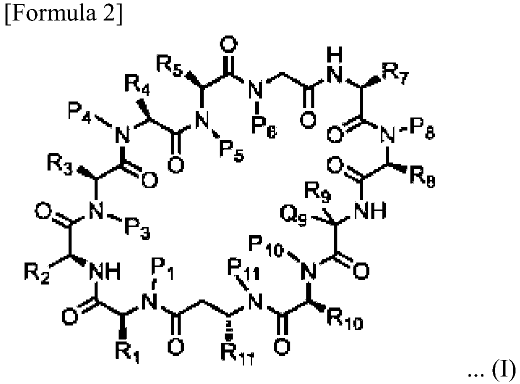 wherein, in the above formula (I), P₁, P₃, P₅, P₆, P₁₀, and P₁₁ are each a C₁ to C₆ alkyl; P₄ is a C₁ to C₆ alkyl, or P₄ forms a 4- to 7-membered saturated heterocyclic ring together with the nitrogen atom to which P₄ is bonded, R₄, and the carbon atom to which R₄ is bonded; P₈ is a C₁ to C₆ alkyl, or P₈ forms a 4- to 7-membered saturated heterocyclic ring together with the nitrogen atom to which P₈ is bonded, R₈, and the carbon atom to which is bonded, the 4- to 7-membered saturated heterocyclic ring being optionally substituted by a C₁ to C₆ alkoxy; R₁, R₂, R₃, R₅, R₇, and R₁₀ are each a hydrogen atom, a C₁ to C₆ alkyl, a C₃ to C₆ cycloalkyl, or an aralkyl optionally having a substituent; R₄ is a hydrogen atom or a C₁ to C₆ alkyl, except when R₄ and P₄ form a 4- to 7-membered saturated heterocyclic ring; R₈ is a hydrogen atom or a C₁ to C₆ alkyl, except when R₈ and P₈ form a 4- to 7-membered saturated heterocyclic ring; R₉ forms a 3- to 7-membered saturated carbocyclic ring together with Q₉ and the carbon atom to which R₉ and Q₉ are bonded; and R₁₁ is a hydrogen atom, a C₁ to C₆ alkyl, a di-C₁ to C₆ alkylaminocarbonyl, or a 4- to 8-membered cyclic aminocarbonyl.

In a cyclic peptide according to one embodiment of the present invention, P₁, P₃, P₅, P₆, P₁₀, and P₁₁ are each methyl or ethyl.

In a cyclic peptide according to one embodiment of the present invention, P₄ is methyl, or forms a 4-membered saturated heterocyclic ring together with the nitrogen atom to which P₄ is bonded, R₄, and the carbon atom to which R₄ is bonded.

In a cyclic peptide according to one embodiment of the present invention, P₈ forms a 5-membered saturated heterocyclic ring together with the nitrogen atom to which P₈ is bonded, R₈, and the carbon atom to which R₈ is bonded, the 5-membered saturated heterocyclic ring being optionally substituted by a C₁ to C₆ alkoxy.

In a cyclic peptide according to one embodiment of the present invention, R₁ and R₂ are each a C₁ to C₆ alkyl; R₃ is a hydrogen atom or a C₁ to C₆ alkyl; R₄ is a hydrogen atom, except when R₄ and P₄ form a 4- to 7-membered saturated heterocyclic ring; is a C₃ to C₆ cycloalkyl or benzyl optionally having a substituent; R₇ is phenylethyl optionally having a substituent; R₉ forms a 5-membered saturated carbocyclic ring together with Q₉ and the carbon atom to which R₉ and Q₉ are bonded; R₁₀ is a C₁ to C₆ alkyl or a C₃ to C₆ cycloalkyl; and R₁₁ is methyl, a di-C₁ to C₆ alkylaminocarbonyl, or a 6-membered cyclic aminocarbonyl.

A cyclic peptide according to one embodiment of the present invention may have a ClogP value of greater than 10. The cyclic peptide according to one embodiment of the present invention may have a ClogP value of 11 or more, 12 or more, 13 or more, 14 or more, or 14.6 or more, and 25 or less, 24 or less, 23 or less, 22 or less, 21 or less, or 20 or less. The cyclic peptide according to one embodiment of the present invention preferably has a ClogP value of 11 or more and 25 or less. The cyclic peptide according to one embodiment of the present invention more preferably has a ClogP value of 13 or more and 22 or less. The cyclic peptide according to one embodiment of the present invention most preferably has a ClogP value of 14.6 or more and 20 or less. In addition, the ClogP value of the cyclic peptide according to one embodiment of the present invention may be greater than the ClogP value of cyclosporin A.

The cyclic peptide according to one embodiment of the present invention preferably does not contain a carboxy group.

The cyclic peptide according to one embodiment of the present invention preferably does not contain a hydroxy group.

The cyclic peptide according to one embodiment of the present invention preferably does not contain a phenolic hydroxy group.

The cyclic peptide according to one embodiment of the present invention preferably does not contain a pyridine ring.

The cyclic peptide according to one embodiment of the present invention preferably does not contain a xanthine ring.

One embodiment of the present invention is a method for producing a cocrystal containing a cyclic peptide and a compound with a melting point of 20°C or higher, the method comprising a step of bringing the above cyclic peptide into contact with the above compound and a solvent. As described above, the term "cocrystal containing a cyclic peptide and a compound" as used herein is defined as a crystal containing a cyclic peptide and a compound with a melting point of 20°C or higher. As used herein, the above compound with a melting point of 20°C or higher contained in such a cocrystal is also described as "coformer".

A coformer according to one embodiment of the present invention may be a coformer other than metal salts. In other words, one embodiment of the present invention may be a method for producing a cocrystal containing a cyclic peptide and a compound with a melting point of 20°C or higher (excluding metal salts), the method comprising a step of bringing the above cyclic peptide into contact with the above compound and a solvent.

A coformer according to one embodiment of the present invention has a melting point of 20°C or higher. The coformer according to one embodiment of the present invention may have a melting point of 25°C or higher. The coformer according to one embodiment of the present invention may have a melting point of 26°C or higher, 27°C or higher, 28°C or higher, 30°C or higher, 40°C or higher, 50°C or higher, 60°C or higher, or 70°C or higher. The coformer according to one embodiment of the present invention preferably have a melting point of 25°C or higher, more preferably 28°C or higher, and most preferably 30°C or higher.

A coformer according to one embodiment of the present invention may be a compound with a molecular weight of 500 or less, 400 or less, 300 or less, 200 or less, 150 or less, or 125 or less. The coformer according to one embodiment of the present invention is preferably a compound with a molecular weight of 500 or less, more preferably a compound with a molecular weight of 200 or less, and most preferably a compound with a molecular weight of 150 or less.

A coformer according to one embodiment of the present invention may be a compound with a molecular weight of 50 or more, 55 or more, or 58 or more. The coformer according to one embodiment of the present invention is preferably a compound with a molecular weight of 50 or more, more preferably a compound with a molecular weight of 55 or more, and most preferably a compound with a molecular weight of 58 or more.

A coformer according to one embodiment of the present invention may be a compound represented by R¹-C(=O)-NR²R³ or R¹-C≡N, where R¹ is a C₁-C₄ alkyl, phenyl, 2-pyridyl, 3-pyridyl, 4-pyridyl, 2-furyl, -NH₂, -NH-OH, -C(=O)-NH₂, -CH₂-C(=O)-NH₂, or -(CH₂)₂-C(=O)-NH₂, and R² and R³ may be each independently hydrogen or a C₁-C₄ alkyl. The coformer according to one embodiment of the present invention is preferably a compound represented by R¹-C(=O)-NR²R³, where R¹ is a C₁-C₄ alkyl, phenyl, 2-pyridyl, 3-pyridyl, 4-pyridyl, 2-furyl, - NH₂, -NH-OH, -C(=O)-NH₂, -CH₂-C(=O)-NH₂, or -(CH₂)₂-C(=O)-NH₂, and R² and R³ are each independently hydrogen or a C₁-C₄ alkyl. In these cases, more preferably, R² and R³ are hydrogen. In these cases, most preferably, R¹ is 2-pyridyl, 3-pyridyl, 2-furyl, or -NH₂, and R² and R³ are hydrogen. In addition, the coformer according to one embodiment of the present invention is a compound represented by R¹-C≡N, where R¹ is 2-pyridyl, 3-pyridyl, or 4-pyridyl.

A coformer according to one embodiment of the present invention may be a compound selected from the group consisting of urea, nicotinamide, 2-furamide, and 4-cyanopyridine. The coformer according to one embodiment of the present invention is preferably urea, nicotinamide, or 2-furamide. The coformer according to one embodiment of the present invention is more preferably urea or nicotinamide. The coformer according to one embodiment of the present invention is most preferably urea. Alternatively, the coformer according to one embodiment of the present invention is most preferably nicotinamide.

One embodiment of the present invention is a method for producing a cocrystal containing a coformer and the above compound, the method comprising a step of bringing a cyclic peptide into contact with the above coformer and a solvent. The above solvent according to a production method of one embodiment of the present invention may be solvent (A) with a molecular weight of 30 or more and 170 or less, or a mixed solvent of solvent (A) with a molecular weight of 30 or more and 170 or less and solvent (B) with a molecular weight of 18 or more and 75 or less, the above solvent (A) is one or more types selected from the group consisting of an amide-based solvent, a sulfoxide-based solvent, an aromatic hydrocarbon-based solvent, a halogen-based solvent, an alcohol-based solvent, an ether-based solvent, an ester-based solvent, a nitrile-based solvent, a carboxylic acid-based solvent, and a ketone-based solvent, and the above solvent (B) may be one or more types selected from the group consisting of an alkyl alcohol-based solvent and water.

The above solvent (A) according to one embodiment of the present invention may be one or more types selected from the group consisting of an amide-based solvent, a sulfoxide-based solvent, an aromatic hydrocarbon-based solvent, a halogen-based solvent, an alcohol-based solvent, an ether-based solvent, an ester-based solvent, a nitrile-based solvent, a carboxylic acid-based solvent, and a ketone-based solvent. The above solvent (A) according to one embodiment of the present invention may be one or more types selected from the group consisting of an alcohol-based solvent, an ether-based solvent, a carboxylic acid-based solvent, and a ketone-based solvent. The above solvent (A) according to one embodiment of the present invention is preferably one or more types selected from the group consisting of an alcohol-based solvent, an ether-based solvent, a carboxylic acid-based solvent, and a ketone-based solvent. The above solvent (A) according to one embodiment of the present invention is more preferably an alcohol-based solvent. Alternatively, the above solvent (A) according to one embodiment of the present invention is more preferably an ether-based solvent. Alternatively, the above solvent (A) according to one embodiment of the present invention is more preferably a carboxylic acid-based solvent. Alternatively, the above solvent (A) according to one embodiment of the present invention is more preferably a ketone-based solvent.

The term "amide-based solvent" as used herein means a solvent that contains an amide bond in the molecule. Examples of the "amide-based solvent" or "amide-based solvent with a molecular weight of 30 or more and 170 or less" as used herein may include formamide, N-methylformamide, N,N-dimethylformamide, N,N-dimethylacetamide, 2-pyrrolidone, and N-methylpyrrolidone.

The term "sulfoxide-based solvent" as used herein means a solvent that falls under the category of sulfoxide. Examples of the "sulfoxide-based solvent" or "sulfoxide-based solvent with a molecular weight of 30 or more and 170 or less" as used herein may include dimethyl sulfoxide, phenyl methyl sulfoxide, and diethyl sulfoxide.

The term "hydrocarbon-based solvent" as used herein means a solvent that is composed of multiple carbon atoms and multiple hydrogen atoms.

The term "aromatic hydrocarbon-based solvent" as used herein means a hydrocarbon-based solvent that has one or more aromatic rings in the molecule. The "aromatic hydrocarbon-based solvent" as used herein is preferably a solvent that has one or more benzene rings in the molecule, and more preferably a solvent that has one benzene ring in the molecule. Examples of the "aromatic hydrocarbon-based solvent" or "aromatic hydrocarbon-based solvent with a molecular weight of 30 or more and 170 or less" as used herein may include benzene, toluene, xylene, ethylbenzene, tetralin, and cumene.

The term "halogen-based solvent" as used herein means a solvent that has one or more halogen atoms in the molecule. The "halogen-based solvent" as used herein is preferably a solvent that has one or more chlorine atoms and/or bromine atoms in the molecule, and more preferably a solvent that has one or more chlorine atoms in the molecule. Examples of the "halogen-based solvent" or "halogen-based solvent with a molecular weight of 30 or more and 170 or less" as used herein may include dichloromethane, chloroform, 1,2-dichloroethane, chlorobenzene, bromobenzene, and carbon tetrachloride.

The term "alcohol-based solvent" as used herein means a solvent that has one or more hydroxy groups bonded to a carbon atom in the molecule. Examples of the "alcohol-based solvent" or "alcohol-based solvent with a molecular weight of 30 or more and 170 or less" as used herein may include methanol, ethanol, 1-propanol, 2-propanol, 1-butanol, 1-pentanol, 2-methyl-1-propanol, 2-methyl-1-butanol, 2-methoxyethanol, 2-ethoxyethanol, 2,2,2-trifluoroethanol, 1,1,1,3,3,3-hexafluoro-2-propanol, phenol, and benzyl alcohol. An "alcohol-based solvent" or an "alcohol-based solvent with a molecular weight of 30 or more and 170 or less" according to one embodiment of the present invention is preferably one or more types selected from the group consisting of methanol, ethanol, 1-propanol, 2-propanol, 1-butanol, 1-pentanol, 2-methyl-1-propanol, 2-methyl-1-butanol, 2-methoxyethanol, 2-ethoxyethanol, 2,2,2-trifluoroethanol, 1,1,1,3,3,3-hexafluoro-2-propanol, phenol, and benzyl alcohol, more preferably methanol, ethanol, 1-propanol, 2-propanol, 1-butanol, 1-pentanol, 2-methyl-1-propanol, 2-methyl-1-butanol, 2-methoxyethanol, 2-ethoxyethanol, 2,2,2-trifluoroethanol, 1,1,1,3,3,3-hexafluoro-2-propanol, phenol, or benzyl alcohol, and most preferably 2-methoxyethanol or 2-ethoxyethanol.

The term "ether-based solvent" as used herein means a solvent that has one or more ether bonds in the molecule. Examples of the "ether-based solvent" or "ether-based solvent with a molecular weight of 30 or more and 170 or less" as used herein may include diethyl ether, tetrahydrofuran, 2-methyltetrahydrofuran, cyclopentyl methyl ether, 4-methyltetrahydropyran, 1,3-dioxolane, 1,4-dioxane, 1,2-dimethoxyethane, diisopropyl ether, anisole, and t-butyl methyl ether. An "ether-based solvent" or an "ether-based solvent with a molecular weight of 30 or more and 170 or less" according to one embodiment of the present invention is preferably one or more types selected from the group consisting of diethyl ether, tetrahydrofuran, 2-methyltetrahydrofuran, cyclopentyl methyl ether, 4-methyltetrahydropyran, 1,3-dioxolane, 1,4-dioxane, 1,2-dimethoxyethane, diisopropyl ether, anisole, and t-butyl methyl ether, more preferably diethyl ether, tetrahydrofuran, 2-methyltetrahydrofuran, cyclopentyl methyl ether, 4-methyltetrahydropyran, 1,3-dioxolane, 1,4-dioxane, 1,2-dimethoxyethane, diisopropyl ether, anisole, or t-butyl methyl ether, and most preferably 1,4-dioxane.

The term "ester-based solvent" as used herein means a solvent that has one or more ester bonds in the molecule. Examples of the "ester-based solvent" or "ester-based solvent with a molecular weight of 30 or more and 170 or less" as used herein may include ethyl formate, methyl acetate, ethyl acetate, methyl propionate, n-butyl acetate, propyl acetate, isopropyl acetate, isobutyl acetate, pentyl acetate, and γ-valerolactone.

The term "nitrile-based solvent" as used herein means a solvent that has one or more cyano groups bonded to a carbon atom in the molecule. Examples of the "nitrile-based solvent" or "nitrile-based solvent with a molecular weight of 30 or more and 170 or less" as used herein may include acetonitrile, benzonitrile, and propionitrile.

The term "carboxylic acid-based solvent" as used herein means a solvent that has one or more carboxy groups in the molecule. Examples of the "carboxylic acid-based solvent" or "carboxylic acid-based solvent with a molecular weight of 30 or more and 170 or less" as used herein may include formic acid, acetic acid, and propionic acid. A "carboxylic acid-based solvent" or a "carboxylic acid-based solvent with a molecular weight of 30 or more and 170 or less" according to one embodiment of the present invention is preferably one or more types selected from the group consisting of formic acid, acetic acid, and propionic acid, more preferably formic acid, acetic acid, or propionic acid, and most preferably acetic acid.

The term "ketone-based solvent" as used herein means a solvent represented by R₁-C(=O)-R₂, where R₁ and R₂ are each independently an alkyl group, or R₁ and R₂ together form an alkylene group. Examples of the "ketone-based solvent" or "ketone-based solvent with a molecular weight of 30 or more and 170 or less" as used herein may include acetone, methyl ethyl ketone, methyl isobutyl ketone, methyl butyl ketone, cyclohexanone, diethyl ketone, and cyclopentanone. A "ketone-based solvent" or a "ketone-based solvent with a molecular weight of 30 or more and 170 or less" according to one embodiment of the present invention is preferably one or more types selected from the group consisting of acetone, methyl ethyl ketone, methyl isobutyl ketone, methyl butyl ketone, cyclohexanone, diethyl ketone, and cyclopentanone, more preferably acetone, methyl ethyl ketone, methyl isobutyl ketone, methyl butyl ketone, cyclohexanone, diethyl ketone, or cyclopentanone, and most preferably acetone.

The above solvent (B) according to one embodiment of the present invention may be one or more types selected from the group consisting of an alkyl alcohol-based solvent and water. The above solvent (B) according to one embodiment of the present invention is preferably an alkyl alcohol-based solvent or water. The above solvent (B) according to one embodiment of the present invention is more preferably an alkyl alcohol-based solvent. Alternatively, the above solvent (B) according to one embodiment of the present invention is more preferably water.

The term "alkyl alcohol-based solvent" as used herein means a solvent that is composed of one alkyl group and one hydroxy group. Examples of the "alkyl alcohol-based solvent" or "alkyl alcohol-based solvent with a molecular weight of 18 or more and 75 or less" as used herein may include methanol, ethanol, 1-propanol, 2-propanol, 1-butanol, and 2-methyl-1-propanol. An "alkyl alcohol-based solvent" or an "alkyl alcohol-based solvent with a molecular weight of 18 or more and 75 or less" according to one embodiment of the present invention is preferably one or more types selected from the group consisting of methanol, ethanol, 1-propanol, 2-propanol, 1-butanol, and 2-methyl-1-propanol, more preferably methanol, ethanol, 1-propanol, 2-propanol, 1-butanol, or 2-methyl-1-propanol, and most preferably ethanol.

The volume ratio (v/v) between the above solvent (A) and the above solvent (B) in the solvent according to one embodiment of the present invention may be 1:0 to 1:10. The volume ratio (v/v) between the above solvent (A) and the above solvent (B) in the solvent according to one embodiment of the present invention may be 1:0 to 1:8, 1:0 to 1:5, 1:0 to 1:4, 1:0 to 1:3, 1:1 to 1:4, or 1:1 to 1:3. The volume ratio (v/v) between the above solvent (A) and the above solvent (B) in the solvent according to one embodiment of the present invention is preferably 1:0 to 1:10, more preferably 1:0 to 1:4, and most preferably 1:1 to 1:3.

The above solvent (A) and the above solvent (B) in the solvent according to one embodiment of the present invention may have a melting point of 25°C or lower. The above solvent (A) and the above solvent (B) in the solvent according to one embodiment of the present invention preferably have a melting point of 25°C or lower, more preferably lower than 20°C, and most preferably lower than 18°C.

The molecular weight of the above solvent (A) in the solvent according to one embodiment of the present invention is 30 or more and 170 or less. The molecular weight of the above solvent (A) in the solvent according to one embodiment of the present invention may be 40 or more and 150 or less, 45 or more and 130 or less, 50 or more and 110 or less, or 55 or more and 95 or less. The molecular weight of the above solvent (A) in the solvent according to one embodiment of the present invention is preferably 40 or more and 150 or less, still more preferably 50 or more and 110 or less, and most preferably 55 or more and 95 or less.

The molecular weight of the alkyl alcohol-based solvent in the above solvent (B) of the solvent according to one embodiment of the present invention is 18 or more and 75 or less. The molecular weight of the alkyl alcohol-based solvent in the above solvent (B) of the solvent according to one embodiment of the present invention may be 30 or more and 60 or less, 35 or more and 55 or less, or 40 or more and 50 or less. The molecular weight of the alkyl alcohol-based solvent in the above solvent (B) of the solvent according to one embodiment of the present invention is preferably 30 or more and 60 or less, more preferably 35 or more and 55 or less, and most preferably 40 or more and 50 or less.

In one embodiment of the present invention, the step of bringing the cyclic peptide into contact with the compound (coformer) and the solvent can be performed by following, for example, the following method. The cyclic peptide and the coformer can be dissolved in the solvent and stirred with shaking for a predetermined time under predetermined temperature conditions to bring them into contact with the solvent, thereby producing a cocrystal containing the above cyclic peptide and the above coformer.

In one embodiment of the present invention, in the above step of bringing the cyclic peptide into contact with the compound and the solvent, the concentration of the cyclic peptide may be 1 mg to 700 mg/mL. In one embodiment of the present invention, in the above step of bringing the cyclic peptide into contact with the compound and the solvent, the concentration of the cyclic peptide may be 5 mg to 700 mg/mL, 10 mg to 1000 mg/mL, 10 mg to 500 mg/mL, 10 mg to 100 mg/mL, 10 mg to 50 mg/mL, 100 mg to 400 mg/mL, 100 mg to 200 mg/mL, or 500 mg to 1000 mg/mL. The concentration of the cyclic peptide according to one embodiment of the present invention is preferably 10 mg to 500 mg/mL, more preferably 10 mg to 400 mg/mL, still more preferably 10 mg to 100 mg/mL, and most preferably 10 mg to 50 mg/mL.

In one embodiment of the present invention, the cyclic peptide used in the above step of bringing the cyclic peptide into contact with the compound and the solvent may be a freeze-dried product.

In one embodiment of the present invention, the cyclic peptide used in the above step of bringing the cyclic peptide into contact with the compound and the solvent is a cyclic peptide contained in a composition containing the above cyclic peptide, and the weight ratio of the above cyclic peptide in the above mixture may be 50% or more. In one embodiment of the present invention, the cyclic peptide used in the above step of bringing the cyclic peptide into contact with the compound and the solvent is a cyclic peptide contained in a mixture containing the above cyclic peptide, and the weight ratio of the above cyclic peptide in the above composition may be 60% or more, 70% or more, 80% or more, 85% or more, 90% or more, 95% or more, or 97% or more. In one embodiment of the present invention, the weight ratio of the above cyclic peptide in the above mixture is preferably 70% or more, more preferably 85% or more, and most preferably 95% or more.

In one embodiment of the present invention, the cyclic peptide used in the above step of bringing the cyclic peptide into contact with the compound and the solvent is a cyclic peptide contained in a composition containing the above cyclic peptide, and the area of a peak of HPLC at 225 nm may account for 50% or more of the total area of all peaks detected in HPLC analysis of the above composition. In one embodiment of the present invention, the cyclic peptide used in the above step of bringing the cyclic peptide into contact with the compound and the solvent is a cyclic peptide contained in a mixture containing the above cyclic peptide, and the area of a peak of HPLC at 225 nm may account for 60% or more, 70% or more, 80% or more, 85% or more, 90% or more, 95% or more, or 97% or more of the total area of all peaks detected in HPLC analysis of the above mixture. In one embodiment of the present invention, the weight ratio of the above cyclic peptide in the above mixture is preferably 70% or more, more preferably 85% or more, and most preferably 95% or more.

In one embodiment of the present invention, the cyclic peptide used in the above step of bringing the cyclic peptide into contact with the compound and the solvent may be 0.5 mg to 200 kg. In one embodiment of the present invention, the cyclic peptide used in the above step of bringing the cyclic peptide into contact with the compound and the solvent may be 1 mg to 1 g. In one embodiment of the present invention, the cyclic peptide used in the above step of bringing the cyclic peptide into contact with the compound and the solvent may be 10 g to 200 kg, 100 g to 100 kg, 0.5 mg to 10 g, 1 mg to 1 g, 1 mg to 100 mg, 1 mg to 10 mg, or 1 mg to 5 mg. In one embodiment of the present invention, the cyclic peptide used in the above step of bringing the cyclic peptide into contact with the compound and the solvent is preferably 1 mg to 1 g, more preferably 1 mg to 100 mg, and most preferably 1 mg to 5 mg.

In one embodiment of the present invention, the above step of bringing the cyclic peptide into contact with the compound and the solvent may not comprise adding a seed crystal.

In one embodiment of the present invention, the above step of bringing the cyclic peptide into contact with the compound and the solvent may comprise adding a seed crystal. The seed crystal may be a cocrystal containing the cyclic peptide and the compound (coformer) produced by a production method according to one embodiment of the present disclosure, or it may be a cocrystal containing the cyclic peptide and the compound (coformer) produced by another method.

In one embodiment of the present invention, a solid-liquid separation step may be further comprised after the above step of bringing the cyclic peptide into contact with the compound and the solvent. Comprising a solid-liquid separation step after the above step of bringing the cyclic peptide into contact with the compound and the solvent is not limited to the case where the solid-liquid separation step is carried out immediately after the above step of bringing the cyclic peptide into contact with the compound and the solvent, and also includes the case where the solid-liquid separation step is carried out after one or more steps are carried out immediately after the above step of bringing the cyclic peptide into contact with the compound and the solvent. The above solid-liquid separation is preferably centrifugation or filtration, and more preferably filtration. The filtration step is a step of filtering the solvent containing the cocrystal containing the cyclic peptide and the compound (coformer) to collect the cocrystal by solid-liquid separation. The filtration step can be carried out by, for example, filtering the solvent containing the cocrystal containing the cyclic peptide and the compound (coformer) through filter paper or the like, collecting by filtration the cocrystal containing the cyclic peptide and the compound (coformer), and the like.

In one embodiment of the present invention, the above step of bringing the cyclic peptide into contact with the compound and the solvent may comprise bringing into contact with beads. In one embodiment of the present invention, the above step of bringing the cyclic peptide into contact with the compound and the solvent may be carried out under grinding conditions using tungsten beads. In one embodiment of the present invention, the above step of bringing the cyclic peptide into contact with the compound and the solvent may be carried out under stirring or shaking conditions.

In one embodiment of the present invention, the above cocrystal may have one or more diffraction peaks in powder X-ray diffraction using CuKα radiation.

In one embodiment of the present invention, the above step of bringing the cyclic peptide into contact with the compound and the solvent may be carried out at a temperature of -10°C to 120°C for 30 minutes to 12 weeks. In one embodiment of the present invention, the above step of bringing the cyclic peptide into contact with the compound and the solvent may be carried out at a constant temperature in 0°C to 110°C, 10°C to 100°C, 15°C to 90°C, or 20°C to 80°C, or carried out repeating heating and cooling between a lower limit temperature of 10°C, 20°C, 30°C, 40°C, 45°C, 50°C, or 55°C and an upper limit temperature of 100°C, 90°C, 85°C, 80°C, 75°C, 70°C, or 60°C, 10 times or more, 20 times or more, or 30 times or more, and 1000 times or less, 500 times or less, or 100 times or less, and carried out for 1 hour to 6 weeks, 2 hours to 4 weeks, 4 hours to 2 weeks, or 6 hours to 8 days. In one embodiment of the present invention, the above step of bringing the cyclic peptide into contact with the compound and the solvent is preferably carried out at a temperature of -10°C to 120°C for 30 minutes to 12 weeks, and more preferably carried out at a constant temperature in 20°C to 90°C for 12 hours to 8 days. Alternatively, in one embodiment of the present invention, the above step of bringing the cyclic peptide into contact with the compound and the solvent is more preferably carried out repeating heating and cooling 30 times or more and 100 times or less, and carried out for 12 hours to 8 days.

In one embodiment of the present invention, the cocrystal to be produced containing the cyclic peptide and the compound (coformer) may further contain the solvent.

In one embodiment of the present invention, the cocrystal to be produced containing the cyclic peptide and the compound (coformer) may further contain the above solvent (A). In this case, the above solvent (A) may be 2-methoxyethanol or 1,4-dioxane. In other words, in one embodiment of the present invention, the cocrystal to be produced containing the cyclic peptide and the compound (coformer) may further contain 2-methoxyethanol or 1,4-dioxane.

In one embodiment of the present invention, the cocrystal to be produced containing the cyclic peptide and the compound (coformer) may further contain the above solvent (B). In this case, the above solvent (B) may be water. In other words, in one embodiment of the present invention, the cocrystal to be produced containing the cyclic peptide and the compound (coformer) may further contain water.

In one embodiment of the present invention, the above coformer may be urea and the cocrystal to be produced may further contain the above solvent (A) and the above solvent (B). In other words, in one embodiment of the present invention, the cocrystal to be produced may contain urea, the above solvent (A), and the above solvent (B). In this case, the above solvent (A) may be 2-methoxyethanol or 1,4-dioxane, and the above solvent (B) may be water. In other words, in one embodiment of the present invention, the cocrystal to be produced may contain urea, 2-methoxyethanol or 1,4-dioxane, and water.

In one embodiment of the present invention, the above coformer may be nicotinamide and the cocrystal to be produced may further contain the above solvent (A) and the above solvent (B). In other words, in one embodiment of the present invention, the cocrystal to be produced may contain nicotinamide, the above solvent (A), and the above solvent (B). In this case, the above solvent (A) may be 2-methoxyethanol or 1,4-dioxane and the above solvent (B) may be water. In other words, in one embodiment of the present invention, the cocrystal to be produced may contain nicotinamide, 2-methoxyethanol or 1,4-dioxane, and water.

In one embodiment of the present invention, the above coformer may be 2-furamide and the cocrystal to be produced may further contain the above solvent (B). In other words, in one embodiment of the present invention, the cocrystal to be produced may contain 2-furamide and the above solvent (B). In this case, the above solvent (B) may be water. In other words, in one embodiment of the present invention, the cocrystal to be produced may contain 2-furamide and water.

In one embodiment of the present invention, the above coformer may be 4-cyanopyridine and the cocrystal to be produced may further contain the above solvent (B). In other words, in one embodiment of the present invention, the cocrystal to be produced may contain 4-cyanopyridine and the above solvent (B). In this case, the above solvent (B) may be water. In other words, in one embodiment of the present invention, the cocrystal to be produced may contain 4-cyanopyridine and water.

In one embodiment of the present disclosure, production of the cocrystal containing the cyclic peptide and the coformer can be confirmed by, for example, powder X-ray diffraction measurement, single crystal X-ray structural analysis, thermogravimetry-differential thermal analysis (TG-DTA), and the like. As an example of specific evaluation, it may be evaluated that the cocrystal containing the cyclic peptide and the coformer has been produced in the case where the diffraction pattern of the obtained (co)crystal in powder X-ray diffraction measurement or single crystal X-ray structural analysis differs from both the diffraction patterns when the same measurement is carried out on each of the crystal of the cyclic peptide and the crystal of the coformer. Also, as an example of specific evaluation, it may be evaluated that the cocrystal containing the cyclic peptide and the coformer has been produced in the case where the melting point of the obtained (co)crystal in TG-DTA differs from the melting points when the same measurement is carried out on each of the crystal of the cyclic peptide and the crystal of the coformer.

One other embodiment of the present invention is a method for purifying a cyclic peptide, the method comprising producing a cocrystal containing a cyclic peptide and a compound by following the production method according to one embodiment of the present invention. In one embodiment, the above method for purifying a cyclic peptide is a method for reducing the content of an impurity in a mixture containing the above cyclic peptide and the impurity, and the impurity may be a peptide generated during a synthesis process of the above cyclic peptide, which is an object to be purified. In this case, the above impurity may be a cyclic peptide that is different from the above cyclic peptide, which is an object to be purified, and it may be a peptide having the number of amino acids twice the number of amino acids that the above cyclic peptide, which is an object to be purified, has, a peptide having the number of amino acids three times the number of amino acids that the above cyclic peptide, which is an object to be purified, has, or an isomer of the above cyclic peptide, which is an object to be purified.

In one embodiment, the above method for purifying a cyclic peptide may further comprise a step of reducing the content of the solvent (A) from a mixture containing the above cocrystal. In this case, the above step of reducing the content of the solvent (A) may be a reduced pressure drying step.

In one embodiment, the above method for purifying a cyclic peptide may further comprise a step of reducing the content of the compound with a melting point of 20°C or higher (coformer) from a mixture containing the above cocrystal. In this case, the above step of reducing the content of the compound with a melting point of 20°C or higher may comprise a liquid-liquid separation step using an organic solvent and water.

In one embodiment, the above method for purifying a cyclic peptide may be a method for purifying a cyclic peptide, the method comprising a step of producing a cocrystal containing a cyclic peptide and a compound (coformer) by following the production method according to one embodiment of the present invention, and a step of collecting the above cocrystal by solid-liquid separation. In this case, the above solid-liquid separation may be, for example, centrifugation or filtration, it is preferably centrifugation or filtration, and more preferably filtration. In one embodiment, the above method for purifying a cyclic peptide may be a method for reducing the content of an impurity in a mixture containing the above cyclic peptide and the impurity and the impurity may be a peptide generated during a synthesis process of the above cyclic peptide, which is an object to be purified. In this case, the above impurity may be a cyclic peptide that is different from the above cyclic peptide, which is an object to be purified, and it may be a peptide having the number of amino acids twice the number of amino acids that the above cyclic peptide, which is an object to be purified, has, a peptide having the number of amino acids three times the number of amino acids that the above cyclic peptide, which is an object to be purified, has, or an isomer of the above cyclic peptide, which is an object to be purified.

In one embodiment, purification of the cyclic peptide by the above method for purifying a cyclic peptide can be confirmed by, for example, powder X-ray diffraction measurement, single crystal X-ray structural analysis, thermogravimetry-differential thermal analysis (TG-DTA), chromatographic analysis such as HPLC and LC/MS analysis, and the like. As an example of specific evaluation, it may be evaluated that the cyclic peptide has been purified in the case where the diffraction pattern of the obtained (co)crystal in powder X-ray diffraction measurement or single crystal X-ray structural analysis differs from both the diffraction patterns when the same measurement is carried out on each of the crystal of the cyclic peptide and the crystal of the coformer, or in the case where the diffraction peak intensity corresponding to the diffraction pattern when the same measurement is carried out on the crystal of the cyclic peptide is reduced compared to that before carrying out purification by following the above method for purifying a cyclic peptide. Also, as an example of specific evaluation, it may be evaluated that the cyclic peptide has been purified in the case where the obtained (co)crystal has a single melting point in TG-DTA that is different from the case where the same measurement is carried out on each of the crystal of the cyclic peptide and the crystal of the coformer. In addition, as an example of specific evaluation, it may be evaluated that the cyclic peptide has been purified in the case where, when LC/MS analysis is carried out before and after purification, the proportion of peak area derived from the cyclic peptide relative to the total area of peaks detected in the LC/MS analysis after purification is greater than the proportion when the same analysis is carried out before purification.

One other embodiment of the present invention is a method for screening a cocrystal containing a cyclic peptide and a compound (coformer), the method comprising the following steps (a) to (c):
(a) producing a cocrystal containing the above cyclic peptide and the above compound by following the production method according to one embodiment of the present invention;
(b) analyzing the above cocrystal by single crystal X-ray analysis and/or powder X-ray crystal diffraction; and
(c) comparing the XRPD diffraction patterns of a substance that is to be the cocrystal and a reference substance. In this case, the conditions for single crystal X-ray analysis and powder X-ray crystal diffraction are not particularly limited as long as the diffraction patterns of the above cyclic peptide, the above compound, and the above cocrystal can be measured, and they can be carried out under conditions normally carried out by those skilled in the art.

One other embodiment of the present invention is a method for screening a method for producing a cocrystal containing a cyclic peptide and a compound (coformer), the method comprising the following steps (a) and (b):
(a) producing a cocrystal containing the above cyclic peptide and the above compound by following the production method according to one embodiment of the present invention; and
(b) analyzing, when a cocrystal is obtained in (a), the above cocrystal by single crystal X-ray analysis and/or powder X-ray crystal diffraction. In this case, the conditions for single crystal X-ray analysis and powder X-ray crystal diffraction are not particularly limited as long as the diffraction patterns of the above cyclic peptide, the above compound, and the above cocrystal can be measured, and they can be carried out under conditions normally carried out by those skilled in the art.

One other embodiment of the present invention is a method for increasing the probability that a cocrystal containing a cyclic peptide and a compound (coformer) is produced, the method comprising a step of producing a cocrystal containing the above cyclic peptide and the above compound by following the production method according to one embodiment of the present invention.

One other embodiment of the present invention is use of urea, nicotinamide, 2-furamide, or 4-cyanopyridine in production of a cocrystal containing a cyclic peptide and urea, nicotinamide, 2-furamide, or 4-cyanopyridine. One other embodiment of the present invention is use of urea in production of a cocrystal containing a cyclic peptide and urea. One other embodiment of the present invention is use of nicotinamide in production of a cocrystal containing a cyclic peptide and nicotinamide. One other embodiment of the present invention is use of 2-furamide in production of a cocrystal containing a cyclic peptide and 2-furamide. One other embodiment of the present invention is use of 4-cyanopyridine in production of a cocrystal containing a cyclic peptide and 4-cyanopyridine. In these cases, the production of a cocrystal containing a cyclic peptide and urea, nicotinamide, 2-fluamide, or 4-cyanopyridine may be carried out by following the production method according to one embodiment of the present invention, and is preferably carried out by following the production method according to one embodiment of the present invention.

One other embodiment of the present invention is a cocrystal containing a cyclic peptide and a compound (coformer), produced by following the production method according to one embodiment of the present invention.

### [Examples]

The contents of the present invention will be further described by the following Examples, but the present invention is not limited to their contents. Except those as specifically described, starting substances, starting raw materials, solvents, and reagents were obtained from commercial suppliers, or synthesized using known methods.

### [Example 1] Synthesis of cyclic peptide

Among cyclic peptides CP01 to CP04 (they are also referred to simply as compounds CP01 to CP04.) shown in Table 1, CP01, CP02, and CP04 were synthesized by the same method as described in International Publication No. WO 2013/100132, International Publication No. WO 2018/225864, or International Publication No. WO 2021/90855, and the final products were obtained as dry products. Specifically, compound 2118 in International Publication No. WO 2021/90855 corresponds to compound CP01, compound 1787 corresponds to compound CP02, and compound 1217 corresponds to compound CP04. The synthesis method for compound CP03 will be described in (Example 1-1).

Also, while there is a description of the hydrate crystal (Form C) of compound CP04 herein, it corresponds to the hydrate crystal (Form C) described in International Publication No. WO 2022/234864.

Note that Valinomycin (CAS No.: 2001-95-8) as used herein was purchased from Nacalai Tesque, Inc. or Fermentek Ltd.

The structural formulas of compounds CP01 to CP04 and Valinomycin are shown in Table 1.

Among cyclic peptides (they are also referred to simply as compounds CP05 and CP06.) shown in Table 2, CP05 and CP06 were synthesized by the same method as described in International Publication No. WO 2022/234853 or International Publication No. WO 2023/214576, and the final products were obtained as dry products. Specifically, compound 2230 in International Publication No. WO 2022/234853 and International Publication No. WO 2023/214576 corresponds to compound CP05, and compound 1105 corresponds to compound CP06.

**[Table 1]**

| Compound No. | Structural formula |
|---|---|
| CP01 | 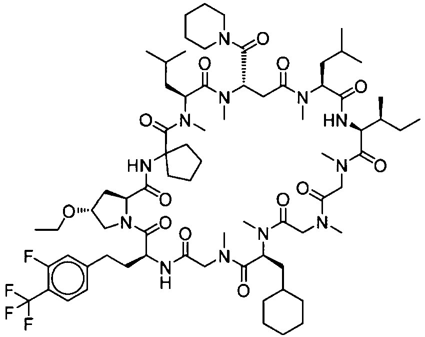 |
| CP02 | 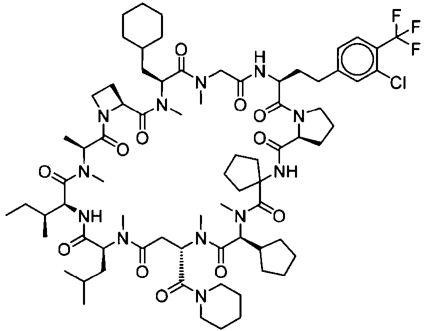 |
| CP03 | 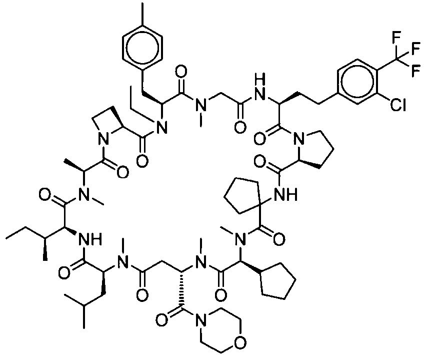 |
| CP04 | 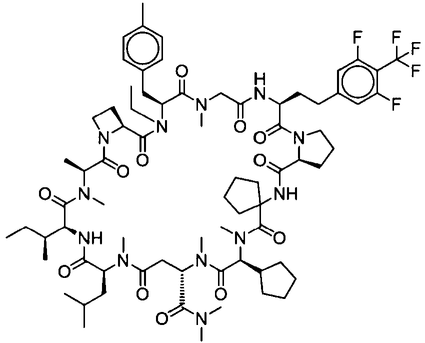 |
| Valinomycin | 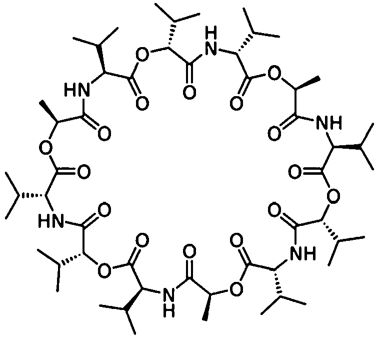 |

**[Table 2]**

| Compound No. | Structural formula |
|---|---|
| CP05 | 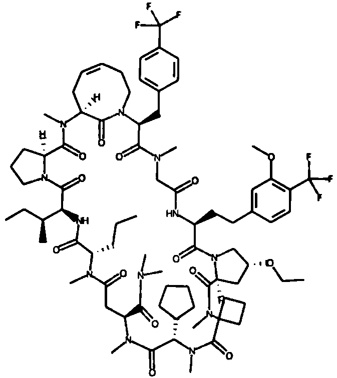 |
| CP06 | 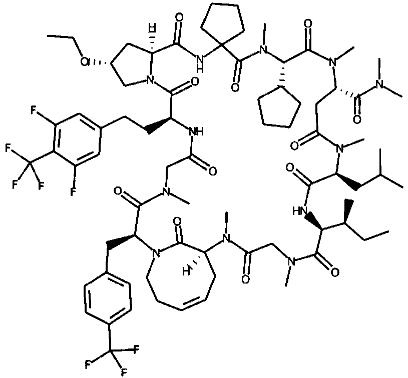 |

The molecular weights of compounds CP01 to CP06 and Valinomycin are as follows.
Compound CP01: 1443.8
Compound CP02: 1454.2
Compound CP03: 1478.2
Compound CP04: 1437.7
Compound CP05: 1553.8
Compound CP06: 1547.7
Valinomycin: 1111.3

The chemical names of compounds CP01 to CP06 and Valinomycin are as follows.
Compound CP01: (3S,9S,18S,21S,25S,28S,34S,36R)-9-(cyclohexylmethyl)-36-ethoxy-3-[2-[3-fluoro-4-(trifluoromethyl)phenyl]ethyl]-21,28-diisobutyl-7,10,13,16,22,26,29-heptamethyl-18-[(1S)-1-methylpropyl]-25-(piperidine-1-carbonyl)spiro[1,4,7,10,13,16,19,22,26,29,32-undecazabicyclo[32.3.0]heptatriacontane-31,1'-cyclopentane]-2,5,8,11,14,17,20,23,27,30,33-undecone
Compound CP02: (3S,9S,12S,17S,20S,23S,27S,30S,36S)-3-[2-[3-chloro-4-(trifluoromethyl)phenyl]ethyl]-9-(cyclohexylmethyl)-30-cyclopentyl-23-isobutyl-7,10,17,18,24,28,31-heptamethyl-20-[(1S)-1-methylpropyl]-27-(piperidine-1-carbonyl)spiro[1,4,7,10,15,18,21,24,28,31,34-undecazatricyclo[34.3.0.012,15]nonatriacontane-33,1'-cyclopentane]-2,5,8,11,16,19,22,25,29,32,35-undecone
Compound CP03: (3S,9S,12S,17S,20S,23S,27S,30S,36S)-3-[2-[3-chloro-4-(trifluoromethyl)phenyl]ethyl]-30-cyclopentyl-10-ethyl-23-isobutyl-7,17,18,24,28,31-hexamethyl-20-[(1S)-1-methylpropyl]-27-(morpholine-4-carbonyl)-9-(p-tolylmethyl)spiro[1,4,7,10,15,18,21,24,28,31,34-undecazatricyclo[34.3.0.012,15]nonatriacontane-33,1'-cyclopentane]-2,5,8,11,16,19,22,25,29,32,35-undecone
Compound CP04: (3S,9S,12S,17S,20S,23S,27S,30S,36S)-30-cyclopentyl-3-[2-[3,5-difluoro-4-(trifluoromethyl)phenyl]ethyl]-10-ethyl-23-isobutyl-N,N,7,17,18,24,28,31-octamethyl-20-[(1S)-1-methylpropyl]-2,5,8,11,16,19,22,25,29,32,35-undecaoxo-9-(p-tolylmethyl)spiro[1,4,7,10,15,18,21,24,28,31,34-undecazatricyclo[34.3.0.012,15]nonatriacontane-33,1'-cyclopentane]-27-carboxamide
Compound CP05: (1S,4S,10S,13S,17S,20S,26S,28R,32S,38S,42Z)-20-cyclopentyl-28-ethoxy-32-[2-[3-methoxy-4-(trifluoromethyl)phenyl]ethyl]-N,N,2,14,18,21,24,36-octamethyl-10-[(1S)-1-methylpropyl]-3,9,12,15,19,22,25,31,34,37,45-undecaoxo-13-propyl-38-[[4-(trifluoromethyl)phenyl]methyl]spiro[2,8,11,14,18,21,24,30,33,36,39-undecazatetracyclo[37.5.1.04,8.026,30]pentatetracont-42-ene-23,1'-cyclobutane]-17-carboxamide
Compound CP06: (2S,8S,12R,14S,20S,23S,27S,30S,36S,38Z)-20-cyclopentyl-8-[2-[3,5-difluoro-4-(trifluoromethyl)phenyl]ethyl]-12-ethoxy-27-isobutyl-N,N,4,19,22,26,32,35-octamethyl-30-[(1S)-1-methylpropyl]-3,6,9,15,18,21,25,28,31,34,42-undecaoxo-2-[[4-(trifluoromethyl)phenyl]methyl]spiro[1,4,7,10,16,19,22,26,29,32,35-undecazatricyclo[34.5.1.010,14]dotetracont-38-ene-17,1'-cyclopentane]-23-carboxamide

The measurement conditions for liquid chromatography-mass spectrometry (LC/MS) used in Synthesis Examples are shown in Table 2.

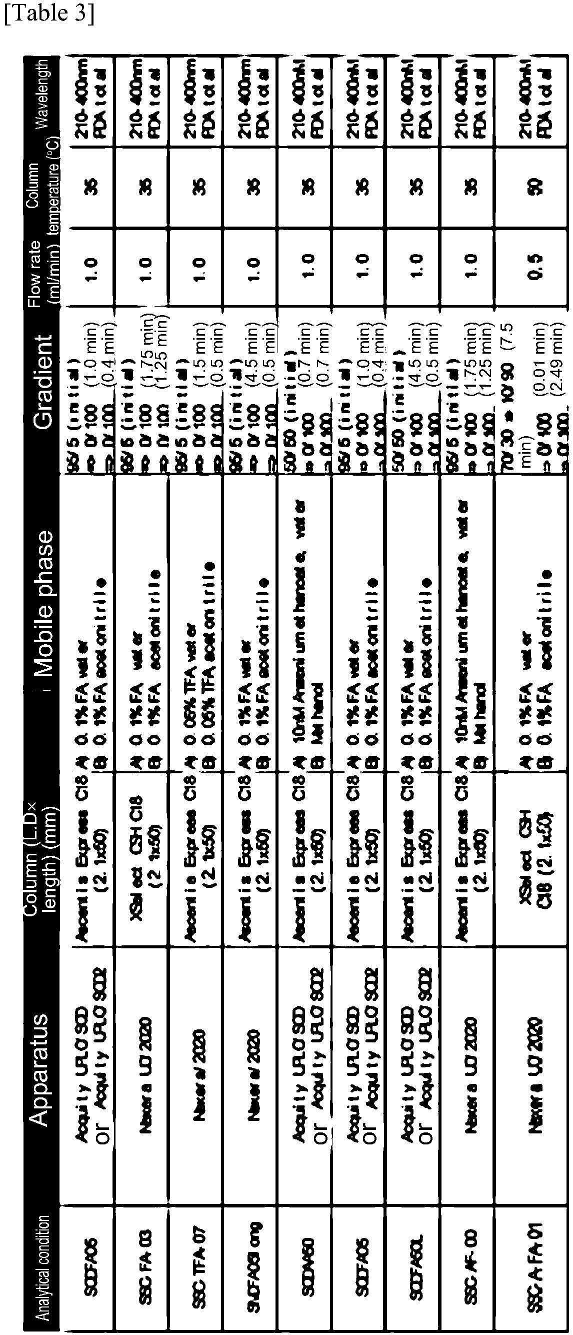

### (Example 1-1) Synthesis of cyclic peptide CP03

### [Synthesis Example 1: Synthesis of compound 2]

Compound 2 was synthesized according to the following scheme.

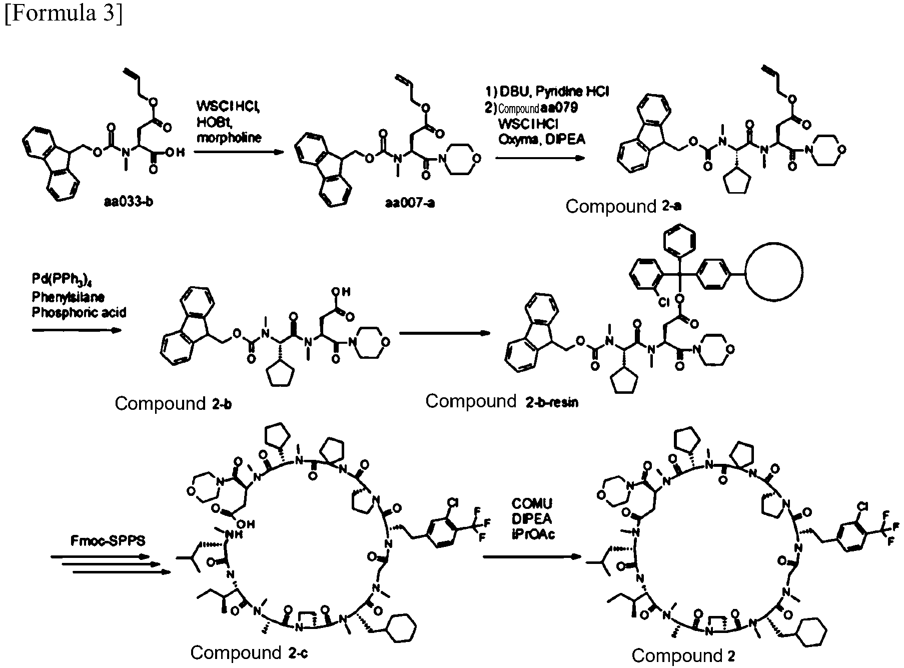

### (1) Synthesis of compound aa007-a

Under a nitrogen atmosphere, to a solution of compound aa033-b ((2S)-2-[9H-fluoren-9-ylmethoxycarbonyl(methyl)amino]-4-oxo-4-prop-2-enoxybutanoic acid, Fmoc-MeAsp(OAl)-OH) (87.94 g, 215 mmol) in dimethylformamide (DMF) (430 ml) produced by the method described in International Publication No. WO 2021/090855, 1-hydroxybenzotriazole (HOBt) (31.9 g, 236 mmol) and 1-ethyl-3-(3-dimethylaminopropyl)carbodiimide hydrochloride (WSCI·HCl) (49.4 g, 258 mmol) were added at room temperature, and the mixture was stirred for 30 minutes. Then, the reaction solution was cooled to 0°C, morpholine (20.44 mL, 236 mmol) was added dropwise, and the mixture was stirred at 0°C for 45 minutes. To the reaction solution, water (180 mL) was added, and the mixture was stirred at room temperature for 1 hour. Further, water (180 mL) was added, and the mixture was stirred at room temperature for 105 minutes. The precipitated solid was collected by filtration and dried under reduced pressure to obtain compound aa007-a (86.83 g, yield 84%).
LCMS(ESI) m/z = 479 (M + H)⁺
Retention time: 2.57 minutes (analytical condition SMDFA05long)

### (2) Synthesis of compound 2-a

To a solution of compound aa079 ((2S)-2-cyclopentyl-2-[9H-fluoren-9-ylmethoxycarbonyl(methyl)amino]acetic acid) (22.6 g, 59.6 mmol) produced by the method described in International Publication No. WO 2021/090855 and cyano(hydroxyimino)ethyl acetate (Oxyma) (10.69 g, 75 mmol) in DMF (203 mL), WSCI·HCl (16.83 g, 88 mmol) was added at room temperature, and the mixture was stirred for 30 minutes to obtain solution A.

Under a nitrogen atmosphere, to a solution of compound aa007-a (30 g, 62.7 mmol) in DMF (203 mL), diazabicycloundecene (DBU) (9.45 mL, 62.7 mmol) was added dropwise at room temperature, and the mixture was stirred for 5 minutes. Pyridine hydrochloride (7.97 g, 69 mmol) was added thereto, and the mixture was stirred for 5 minutes. To the obtained reaction solution, solution A and N,N-diisopropylethylamine (DIPEA) (10.95 mL, 62.7 mmol) were added, and the mixture was stirred under a nitrogen atmosphere at room temperature for 2.5 hours. The reaction solution was diluted with ethyl acetate (300 mL), washed twice with hydrochloric acid (1 mol/L, 300 mL), and the obtained aqueous phase was extracted twice with ethyl acetate (300 mL). All organic phases were mixed, and washed sequentially with water (300 mL), twice with a mixed solution of saturated aqueous sodium hydrogen carbonate solution and water (1:1, 300 mL), and further with a mixed solution of saturated saline and water (1:1, 300 mL). Then, the obtained organic phase was dried over sodium sulfate and the solvent was distilled off under reduced pressure. To the obtained residue, dichloromethane (DCM) (300 mL) was added, and the solids were removed by filtration. The solvent of the obtained solution was distilled off under reduced pressure, and the obtained residue was purified by silica gel column chromatography (hexane/ethyl acetate) to obtain compound 2-a (23.8 g, yield 61.5%).
LCMS(ESI) m/z = 640.4 (M+Na)⁺
Retention time: 0.97 minutes (analytical condition SQDFA05)

### (3) Synthesis of compound 2-b

Under a nitrogen atmosphere, to a solution of compound 2-a (23.8 g, 38.5 mmol) in DCM (77 mL), tetrakis(triphenylphosphine)palladium(0) (0.445 g, 0.385 mmol) was added at room temperature, phenylsilane (3.32 mL, 27 mmol) was further added, and the mixture was stirred for 30 minutes. The reaction solution was diluted with methyl tert-butyl ether (MTBE) (240 mL) and extracted with a mixed solution of saturated aqueous sodium hydrogen carbonate solution and water (1:1, 240 mL). The obtained organic phase was extracted with water (50 mL). All aqueous phases were mixed, DCM (240 mL) was added to the mixture, and phosphoric acid (13.44 ml, 231 mmol) was added dropwise thereto. The organic phase was separated, and the aqueous phase was extracted with DCM (240 mL). The obtained organic phases were mixed, washed with a mixed solution of saturated saline and water (1:1, 240 mL), and then dried over sodium sulfate, and the solvent was distilled off under reduced pressure to obtain compound 2-b (21.35 g, 96% yield).
LCMS(ESI) m/z = 578.4 (M+H)⁺
Retention time: 0.80 minutes (analytical condition SQDFA05)

### (4) Synthesis of compound 2-b-resin

A reaction vessel with a filter was charged with 2-chlorotrityl chloride resin (1.36 mmol/g, 46.2 g, 62.8 mmol), and DCM (462 mL) was added. The vessel was shaken at room temperature for 45 minutes, and the solvent was then discharged from the filter. A solution of compound 2-b (21.35 g, 37 mmol), methanol (11.96 mL, 296 mmol), and DIPEA (30.9 mL, 177 mmol) in DCM (323 mL) was added to the reaction vessel. The vessel was shaken at room temperature for 60 minutes, and the solution was discharged from the filter. Subsequently, a solution of methanol (44.85 mL, 1.1 mol) and DIPEA (30.9 mL, 177 mmol) in DCM (323 mL) was added to the reaction vessel. The vessel was shaken at room temperature for 90 minutes, and the solution was discharged from the filter. DCM (323 mL) was added to the reaction vessel, the vessel was shaken for 5 minutes, and the solvent was discharged from the filter. This washing operation of resin was repeated four more times, and the obtained resin was dried under reduced pressure to obtain compound 2-b-resin (59.1 g). The carrying amount was calculated to be 0.433 mmol/g by the quantitative method of resin described in International Publication No. WO 2013/100132, International Publication No. WO 2018/225864, or International Publication No. WO 2021/90855.

### (5) Synthesis of compound 2-c

Subsequent extensions of Fmoc-cLeu-OH, Fmoc-Pro-OH, Fmoc-Hph (4-CF3-3-Cl)-OH (compound aa132, produced by the method described in International Publication No. WO 2021/090855), Fmoc-MeGly-OH, Fmoc-MeCha-OH, Fmoc-Aze(2)-OH, Fmoc-MeAla-OH, Fmoc-Ile-OH, and Fmoc-MeLeu-OH were performed by the Fmoc solid-phase synthesis method.

### (5-1) Extension of Fmoc-cLeu-OH

Compound 2-b-resin (0.433 mmol/g, 59 g, 25.5 mmol) was added to a reaction vessel with a filter. DCM (600 mL) was added to this solid-phase reaction vessel, and after shaking at room temperature for 5 minutes, the solvent was discharged from the frit. DMF (420 mL) was added to this solid-phase reaction vessel, and after shaking at room temperature for 5 minutes, the solvent was discharged from the frit. This washing step of resin with DMF was repeated one more time. A DMF solution of DBU (2 v/v%, 420 mL) was added to this solid-phase reaction vessel, and after shaking at room temperature for 10 minutes, the solvent was discharged from the frit.

DMF (420 mL) was added to this solid-phase reaction vessel, and after shaking at room temperature for 5 minutes, the solution was discharged from the frit. A solution of triethylamine hydrochloride (7.03 g, 51.1 mmol) in DCM (420 mL) was added to this solid-phase reaction vessel, and after shaking at room temperature for 5 minutes, the solution was discharged from the frit. DCM (420 mL) was added to this solid-phase reaction vessel, and after shaking at room temperature for 5 minutes, the solution was discharged from the frit. DMF (420 mL) was added to this solid-phase reaction vessel, and after shaking at room temperature for 5 minutes, the solvent was discharged from the frit. This washing step of resin with DMF was repeated one more time.

A solution of Fmoc-cLeu-OH (35.9 g, 102 mmol) and Oxyma (9.08 g, 63.9 mmol) in DMF (180 ml) and a solution of N,N'-diisopropylcarbodiimide (DIC) in DMF (10 v/v%, 216 mL) were mixed at room temperature, and after 2 minutes, the resulting mixture was added to the solid-phase reaction vessel obtained by the above. This solid-phase reaction vessel was shaken at 50°C for 24 hours, and the solution was then discharged from the frit. DMF (420 mL) was added to this solid-phase reaction vessel, and after shaking at room temperature for 5 minutes, the solvent was discharged from the frit. This washing step of resin with DMF was repeated four more times.

### (5-2) Extension of Fmoc-Pro-OH

A solution of DBU in DMF (2 v/v%, 420 mL) was added to the resulting solid-phase reaction vessel, and after shaking at room temperature for 10 minutes, the solution was discharged from the frit.

DMF (420 mL) was added to this solid-phase reaction vessel, and after shaking at room temperature for 5 minutes, the solution was discharged from the frit. A solution of triethylamine hydrochloride (7.03 g, 51.1 mmol) in DCM (420 mL) was added to this solid-phase reaction vessel, and after shaking at room temperature for 5 minutes, the solution was discharged from the frit. DCM (420 mL) was added to this solid-phase reaction vessel, and after shaking at room temperature for 5 minutes, the solution was discharged from the frit. DMF (420 mL) was added to this solid-phase reaction vessel, and after shaking at room temperature for 5 minutes, the solution was discharged from the frit. This washing step of resin with DMF was repeated one more time.

A solution of Fmoc-Pro-OH (17.24 g, 51.1 mmol) and 1-hydroxy-7-azabenzotriazole (HOAt) (4.35 g, 31.9 mmol) in DMF (240 mL) was mixed with DIC (11.54 mL, 74.1 mmol) at room temperature, and after 2 minutes, the mixture was added to the solid-phase reaction vessel obtained by the above. This solid-phase reaction vessel was shaken at 30°C for 17 hours, and the solution was then discharged from the frit. DMF (420 mL) was added to this solid-phase reaction vessel, and after shaking at room temperature for 5 minutes, the solvent was discharged from the frit. This washing step of resin with DMF was repeated four more times. DCM (420 mL) was further added to this solid-phase reaction vessel, and after shaking at room temperature for 5 minutes, the solvent was discharged from the frit. This washing step of resin was repeated four more times. The obtained resin was dried under reduced pressure to obtain 63.1 g of resin.

### (5-3) Extension of Fmoc-Hph(4-CF3-3-Cl)-OH (compound aa132)

DCM (600 mL) was added to the solid-phase reaction vessel obtained by the above, and after shaking at room temperature for 5 minutes, the solvent was discharged from the frit. DMF (420 mL) was further added to this solid-phase reaction vessel, and after shaking at room temperature for 5 minutes, the solvent was discharged from the frit. This washing step of resin with DMF was repeated one more time. A DMF solution of DBU (2 v/v%, 420 mL) was added thereto, and after shaking at room temperature for 10 minutes, the solution was discharged from the frit.

DMF (420 mL) was added to this solid-phase reaction vessel, and after shaking at room temperature for 5 minutes, the solution was discharged from the frit. A solution of triethylamine hydrochloride (7.03 g, 51.1 mmol) in DCM (420 mL) was added to this solid-phase reaction vessel, and after shaking at room temperature for 5 minutes, the solution was discharged from the frit. DCM (420 mL) was added to this solid-phase reaction vessel, and after shaking at room temperature for 5 minutes, the solution was discharged from the frit. DMF (420 mL) was added to this solid-phase reaction vessel, and after shaking at room temperature for 5 minutes, the solvent was discharged from the frit. This washing step of resin with DMF was repeated one more time.

A solution of Fmoc-Hph(4-CF3-3-Cl)-OH (compound aa132) (25.7 g, 51.1 mmol) and HOAt (4.35 g, 31.9 mmol) in DMF (240 mL) was mixed with DIC (11.54 mL, 74.1 mmol) at room temperature, and after 2 minutes, the mixture was added to the solid-phase reaction vessel obtained by the above. This solid-phase reaction vessel was shaken at 30°C for 21 hours, and the solution was then discharged from the frit. DMF (420 mL) was added to this solid-phase reaction vessel, and after shaking at room temperature for 5 minutes, the solvent was discharged from the frit. This washing step of resin with DMF was repeated four more times.

### (5-4) Extension of Fmoc-MeGly-OH

A solution of DBU in DMF (2 v/v%, 420 mL) was added to the resulting solid-phase reaction vessel, and after shaking at room temperature for 10 minutes, the solution was discharged from the frit.

DMF (420 mL) was added to this solid-phase reaction vessel, and after shaking at room temperature for 5 minutes, the solution was discharged from the frit. A solution of triethylamine hydrochloride (7.03 g, 51.1 mmol) in DCM (420 mL) was added to this solid-phase reaction vessel, and after shaking at room temperature for 5 minutes, the solution was discharged from the frit. DCM (420 mL) was added to this solid-phase reaction vessel, and after shaking at room temperature for 5 minutes, the solution was discharged from the frit. DMF (420 mL) was added to this solid-phase reaction vessel, and after shaking at room temperature for 5 minutes, the solvent was discharged from the frit. This washing step of resin with DMF was repeated one more time.

A solution of Fmoc-MeGly-OH (15.91 g, 51.1 mmol) and HOAt (4.35 g, 31.9 mmol) in DMF (240 mL) was mixed with DIC (11.54 mL, 74.1 mmol) at room temperature, and after 2 minutes, the resulting mixture was added to the solid-phase reaction vessel obtained by the above. This solid-phase reaction vessel was shaken at 30°C for 32 hours, and the solution was then discharged from the frit. DMF (420 mL) was added to this solid-phase reaction vessel, and after shaking at room temperature for 5 minutes, the solution was discharged from the frit. This washing step of resin with DMF was repeated four more times.

### (5-5) Extension of Fmoc-MeCha-OH

A solution of DBU in DMF (2 v/v%, 420 mL) was added to the resulting solid-phase reaction vessel, and after shaking at room temperature for 10 minutes, the solution was discharged from the frit.

DMF (420 mL) was added to this solid-phase reaction vessel, and after shaking at room temperature for 5 minutes, the solution was discharged from the frit. A solution of triethylamine hydrochloride (7.03 g, 51.1 mmol) in DCM (420 mL) was added to this solid-phase reaction vessel, and after shaking at room temperature for 5 minutes, the solution was discharged from the frit. DCM (420 mL) was added to this solid-phase reaction vessel, and after shaking at room temperature for 5 minutes, the solution was discharged from the frit. DMF (420 mL) was added to this solid-phase reaction vessel, and after shaking at room temperature for 5 minutes, the solvent was discharged from the frit. This washing step of resin with DMF was repeated one more time.

A solution of Fmoc-MeCha-OH (20.82 g, 51.1 mmol) and HOAt (4.35 g, 31.9 mmol) in DMF (240 mL) was mixed with DIC (11.54 mL, 74.1 mmol) at room temperature, and after 2 minutes, the resulting mixture was added to the solid-phase reaction vessel obtained by the above. This solid-phase reaction vessel was shaken at 30°C for 12 hours, and the solution was then discharged from the frit. DMF (420 mL) was added to this solid-phase reaction vessel, and after shaking at room temperature for 5 minutes, the solvent was discharged from the frit. This washing step of resin with DMF was repeated four more times. DCM (420 mL) was further added to this solid-phase reaction vessel, and after shaking at room temperature for 5 minutes, the solvent was discharged from the frit. This washing step of resin with DCM was repeated four more times. The obtained resin was dried under reduced pressure to obtain 72.4 g of resin.

### (5-6) Extension of Fmoc-Aze(2)-OH

DCM (600 mL) was added to the solid-phase reaction vessel obtained by the above, and after shaking at room temperature for 5 minutes, the solvent was discharged from the frit. DMF (420 mL) was further added to this solid-phase reaction vessel, and after shaking at room temperature for 5 minutes, the solvent was discharged from the frit. This washing step of resin with DMF was repeated one more time. A DMF solution of DBU (2 v/v%, 420 mL) was added thereto, and after shaking at room temperature for 10 minutes, the solution was discharged from the frit.

DMF (420 mL) was added to this solid-phase reaction vessel, and after shaking at room temperature for 5 minutes, the solution was discharged from the frit. A solution of triethylamine hydrochloride (7.03 g, 51.1 mmol) in DCM (420 mL) was added to this solid-phase reaction vessel, and after shaking at room temperature for 5 minutes, the solution was discharged from the frit. DCM (420 mL) was added to this solid-phase reaction vessel, and after shaking at room temperature for 5 minutes, the solution was discharged from the frit. DMF (420 mL) was added to this solid-phase reaction vessel, and after shaking at room temperature for 5 minutes, the solvent was discharged from the frit. This washing step of resin with DMF was repeated one more time.

A solution of Fmoc-Aze(2)-OH (16.52 g, 51.1 mmol) and HOAt (4.35 g, 31.9 mmol) in DMF (240 mL) was mixed with DIC (11.54 mL, 74.1 mmol) at room temperature and after 2 minutes, the resulting mixture was added to the solid-phase reaction vessel obtained by the above. This solid-phase reaction vessel was shaken at 30°C for 21 hours, and the solution was then discharged from the frit. DMF (420 mL) was added to this solid-phase reaction vessel, and after shaking at room temperature for 5 minutes, the solvent was discharged from the frit. This washing step of resin with DMF was repeated four more times. DCM (420 mL) was further added to this solid-phase reaction vessel, and after shaking at room temperature for 5 minutes, the solvent was discharged from the frit. This washing step of resin with DCM was repeated four more times. The obtained resin was dried under reduced pressure to obtain 73.1 g of resin.

### (5-7) Extension of Fmoc-MeAla-OH

DCM (600 mL) was added to the solid-phase reaction vessel obtained by the above, and after shaking at room temperature for 5 minutes, the solvent was discharged from the frit. DMF (420 mL) was further added to this solid-phase reaction vessel, and after shaking at room temperature for 5 minutes, the solvent was discharged from the frit. This washing step of resin with DMF was repeated one more time. A DMF solution of DBU (2 v/v%, 420 mL) was added thereto, and after shaking at room temperature for 10 minutes, the solution was discharged from the frit.

DMF (420 mL) was added to this solid-phase reaction vessel, and after shaking at room temperature for 5 minutes, the solution was discharged from the frit. A solution of triethylamine hydrochloride (7.03 g, 51.1 mmol) in DCM (420 mL) was added to this solid-phase reaction vessel, and after shaking at room temperature for 5 minutes, the solution was discharged from the frit. DCM (420 mL) was added to this solid-phase reaction vessel, and after shaking at room temperature for 5 minutes, the solution was discharged from the frit. DMF (420 mL) was added to this solid-phase reaction vessel, and after shaking at room temperature for 5 minutes, the solvent was discharged from the frit. This washing step of resin with DMF was repeated one more time.

A solution of Fmoc-MeAla-OH (16.63 g, 51.1 mmol) and HOAt (4.35 g, 31.9 mmol) in DMF (240 mL) was mixed with DIC (11.54 mL, 74.1 mmol), and after 2 minutes, the resulting mixture was added to the solid-phase reaction vessel obtained by the above. This solid-phase reaction vessel was shaken at 30°C for 16 hours, and the solution was then discharged from the frit. DMF (420 mL) was added to this solid-phase reaction vessel, and after shaking at room temperature for 5 minutes, the solvent was discharged from the frit. This washing step of resin with DMF was repeated four more times. DCM (420 mL) was further added to this solid-phase reaction vessel, and after shaking at room temperature for 5 minutes, the solvent was discharged from the frit. This washing step of resin with DCM was repeated four more times. The obtained resin was dried under reduced pressure to obtain 76.4 g of resin.

### (5-8) Extension of Fmoc-Ile-OH

DCM (600 mL) was added to the solid-phase reaction vessel obtained by the above, and after shaking at room temperature for 5 minutes, the solvent was discharged from the frit. DMF (420 mL) was further added to this solid-phase reaction vessel, and after shaking at room temperature for 5 minutes, the solvent was discharged from the frit. This washing step of resin with DMF was repeated one more time. A DMF solution of DBU (2 v/v%, 420 mL) was added thereto, and after shaking at room temperature for 10 minutes, the solution was discharged from the frit.

DMF (420 mL) was added to this solid-phase reaction vessel, and after shaking at room temperature for 5 minutes, the solution was discharged from the frit. A solution of triethylamine hydrochloride (7.03 g, 51.1 mmol) in DCM (420 mL) was added to this solid-phase reaction vessel, and after shaking at room temperature for 5 minutes, the solution was discharged from the frit. DCM (420 mL) was added to this solid-phase reaction vessel, and after shaking at room temperature for 5 minutes, the solution was discharged from the frit. DMF (420 mL) was added to this solid-phase reaction vessel, and after shaking at room temperature for 5 minutes, the solvent was discharged from the frit. This washing step of resin with DMF was repeated one more time.

A solution of Fmoc-Ile-OH (36.1 g, 102 mmol) and HOAt (8.69 g, 63.9 mmol) in DMF (180 mL) was mixed with a solution of DIC (10 v/v%) in DMF (216 mL), and after 2 minutes, the resulting mixture was added to the solid-phase reaction vessel obtained by the above. This solid-phase reaction vessel was shaken at 40°C for 8 hours, then at 30°C for 14 hours. Thereafter, the solution was discharged from the frit. DMF (420 mL) was added to this solid-phase reaction vessel, and after shaking at room temperature for 5 minutes, the solvent was discharged from the frit. This washing step of resin with DMF was repeated three more times. DCM (420 mL) was added to this solid-phase reaction vessel, and after shaking at room temperature for 5 minutes, the solvent was discharged from the frit. This washing step of resin with DCM was repeated one more time. Toluene (420 mL) was further added to this solid-phase reaction vessel, and after shaking at room temperature for 5 minutes, the solvent was discharged from the frit. This washing step of resin with toluene was repeated one more time.

### (5-9) Extension of Fmoc-MeLeu-OH

A solution of DBU in toluene (2 v/v%, 420 mL) was added to the solid-phase reaction vessel obtained by the above, and after shaking at room temperature for 10 minutes, the solution was discharged from the frit.

Toluene (420 mL) was added to this solid-phase reaction vessel, and after shaking at room temperature for 5 minutes, the solvent was discharged from the frit. This washing step of resin with toluene was repeated one more time. DCM (420 mL) was further added to this solid-phase reaction vessel, and after shaking at room temperature for 5 minutes, the solvent was discharged from the frit. This washing step of resin with DCM was repeated one more time. A solution of Fmoc-MeLeu-OH (37.6 g, 102 mmol), [ethylcyano(hydroxyimino)acetat-O²]tri-1-pyrrolidinylphosphonium hexafluorophosphate (PyOxym) (53.9 g, 102 mmol), and DIPEA (26.8 mL, 153 mmol) in DCM (300 mL) was added thereto, and the vessel was shaken at 30°C for 2 hours. Thereafter, the solution was discharged from the frit. DMF (420 mL) was added to this solid-phase reaction vessel, and after shaking at room temperature for 5 minutes, the solvent was discharged from the frit. This washing step of resin with DMF was repeated four more times. DCM (420 mL) was further added to this solid-phase reaction vessel, and after shaking at room temperature for 5 minutes, the solvent was discharged from the frit. This washing step of resin with DCM was repeated four more times. The obtained resin was dried under reduced pressure to obtain 80.9 g of resin. Of the obtained resin, 40.4 g (equivalent to 13 mmol, converted from the carrying amount of compound 2-b-resin) was transferred to another solid-phase reaction vessel with a filter, and the following reaction was carried out.

### (6) Synthesis of compound 2-c (excision of peptide from resin)

To the solid-phase reaction vessel containing 40.4 g (equivalent to 13 mmol, converted from the carrying amount of compound 2-b-resin) of the resin obtained by the above, DCM (300 mL) was added, and after shaking at room temperature for 5 minutes, the solvent was discharged from the frit. DMF (210 mL) was further added to this solid-phase reaction vessel, and after shaking at room temperature for 5 minutes, the solvent was discharged from the frit. This washing step of resin with DMF was repeated one more time. A DMF solution of DBU (2 v/v%, 210 mL) was added thereto, and after shaking at room temperature for 10 minutes, the solution was discharged from the frit. DMF (210 mL) was added to this solid-phase reaction vessel, and after shaking at room temperature for 5 minutes, the solvent was discharged from the frit. This washing step of resin with DMF was repeated four more times. DCM (210 mL) was further added to this solid-phase reaction vessel, and after shaking at room temperature for 5 minutes, the solvent was discharged from the frit. This washing step of resin with DCM was repeated four more times.

To the solid-phase reaction vessel containing the resin obtained by the above, a mixed solution of 2,2,2-trifluoroethanol (TFE) (270 mL), DCM (270 mL), and DIPEA (4.01 mL, 23 mmol) was added, and the vessel was shaken at room temperature for 2 hours. Thereafter, the solution was collected from the frit. To this solid-phase reaction vessel, a mixed solution of TFE (150 mL) and DCM (150 mL) was added, and after shaking at room temperature for 20 minutes, the solution was collected from the frit. To this solid-phase reaction vessel, a mixed solution of TFE (150 mL) and DCM (150 mL) was further added, and after shaking at room temperature for 20 minutes, the solution was collected from the frit. All the collected solutions were mixed and the solvent was distilled off under reduced pressure to obtain compound 2-c as a crude product (18.9 g).
LCMS(ESI) m/z = 1474.0 (M+H)⁺
Retention time: 0.69 minutes (analytical condition SQDFA05)

### (7) Synthesis of compound 2 (cyclization and purification of peptide)

Compound 2-c (9.6 g) obtained by the above was dissolved in a mixed solution of isopropyl acetate (1246 mL) and DIPEA (1.959 mL, 11.21 mmol), (1-cyano-2-ethoxy-2-oxoethylideneaminooxy)dimethylamino-morpholino-carbenium hexafluorophosphate (COMU) (4 g, 9.35 mmol) was added, and the mixture was stirred at room temperature for 14 hours. Thereafter, the reaction solution was washed with a mixed solution of saturated aqueous ammonium chloride solution (350 mL) and water (350 mL), and then further washed with saturated saline (700 ml). The obtained organic phase was dried over sodium sulfate, and the solvent was distilled off under reduced pressure to obtain about 8 g of residue. Furthermore, the same operation was performed on compound 2-c (9.3 g). All the residues obtained were purified by reverse-phase silica gel column chromatography using acetonitrile (containing 0.1% formic acid)/water (containing 0.1% formic acid) as eluents to obtain a crude product (8.1 g). Of the obtained crude product, 7.9 g was purified by silica gel column chromatography (DCM/methanol) to obtain compound 2 (6.9 g, 37%). Liquid chromatography-mass spectrometry (LC/MS) was performed on the obtained compound 2 under the measurement conditions: SSC-FA-03, the result of which showed a retention time of 1.856 minutes and a mass spectrum value of 1453.8 (M-H)-.

### [Synthesis Example 2: Synthesis of compound CP03]

Compound CP03 (23 g, 30.5%) was obtained by a synthesis method similar to the synthesis of compound 2 using a resin (120 g) produced by the same method as compound 2-b-resin as a raw material. Liquid chromatography-mass spectrometry (LC/MS) was performed on the obtained compound CP03 under the measurement conditions: SQDFA05, the result of which showed a retention time of 1.01 minutes and a mass spectrum value of 1478.3 (M+H)+.

Note that, for example, with respect to compound CP03, there are descriptions of "compound CP03" and "crude product of compound CP03" herein. While the former is a purified product that has been purified using reversed-phase silica gel column chromatography, silica gel column chromatography, or the like, the latter is a product of solvent distillation under reduced pressure at a stage prior to the purification.

### [Example 2] Production of cocrystal containing cyclic peptide and compound with melting point of 20°C or higher

### (Example 2-1-1)

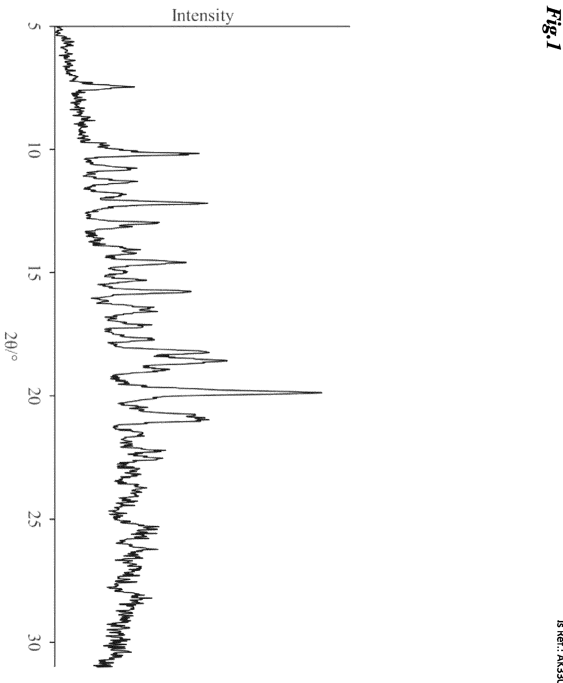

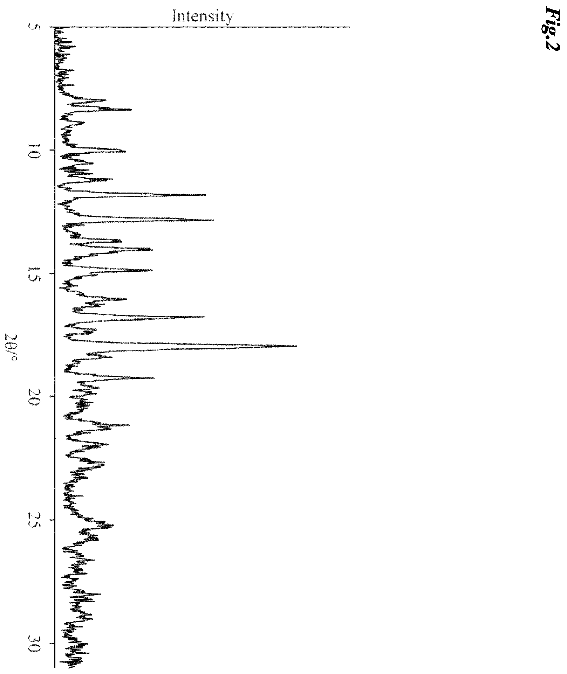

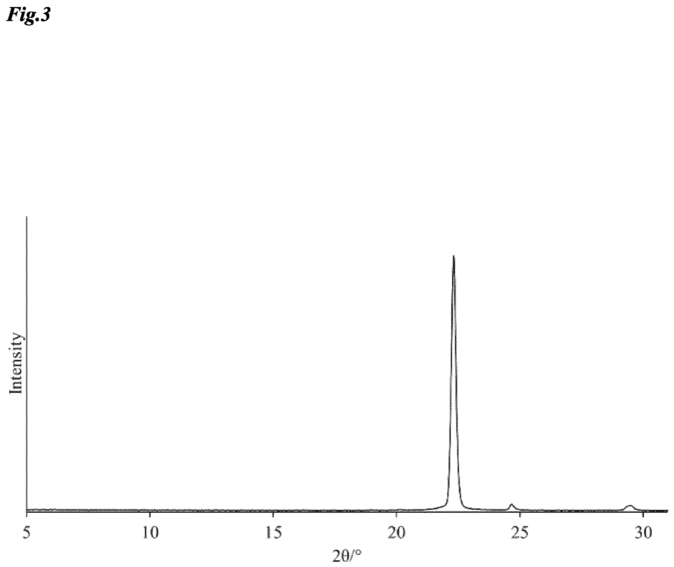

To compound CP04 (102.6 mg) was added 2-methoxyethanol (400 µL), and the mixture was stirred. Urea (20.7 mg) was added. After 25 minutes, 2-methoxyethanol (400 µL) was added and the mixture was further stirred for 70 minutes, the suspension was filtered, and the wet powder was subjected to powder X-ray diffraction measurement. Drying at room temperature and under normal pressure for 21 hours yielded 61.2 mg of cocrystal. The results of powder X-ray diffraction for the cocrystal are shown in Figure 1. The diffraction pattern was confirmed to be different from the diffraction pattern of the hydrate crystal (Form C) of the raw material compound CP04 described in Figure 2, and furthermore, also different from that of urea described in Figure 3. Also, when the content analysis for compound CP04 and urea was carried out by the internal standard method using ultra-high performance liquid chromatography in the manner shown in Examples 3-4 and 3-5, the contents of compound CP04 and urea were found to be 81.8% and 5.4%, respectively.

### (Example 2-1-2)

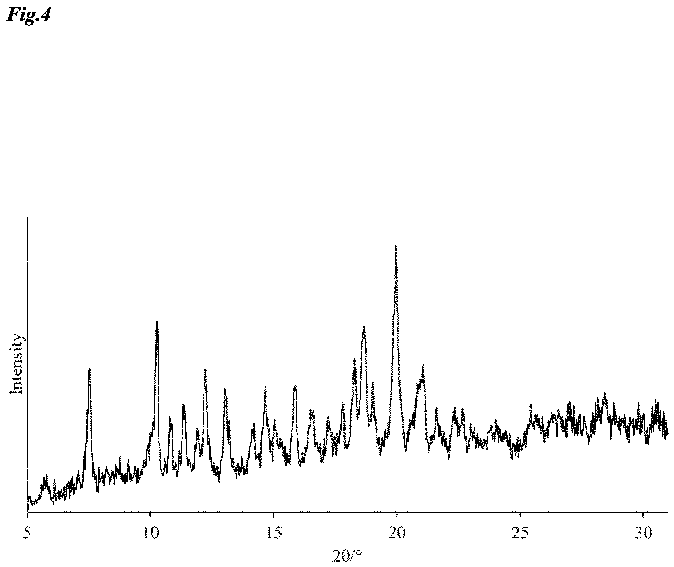

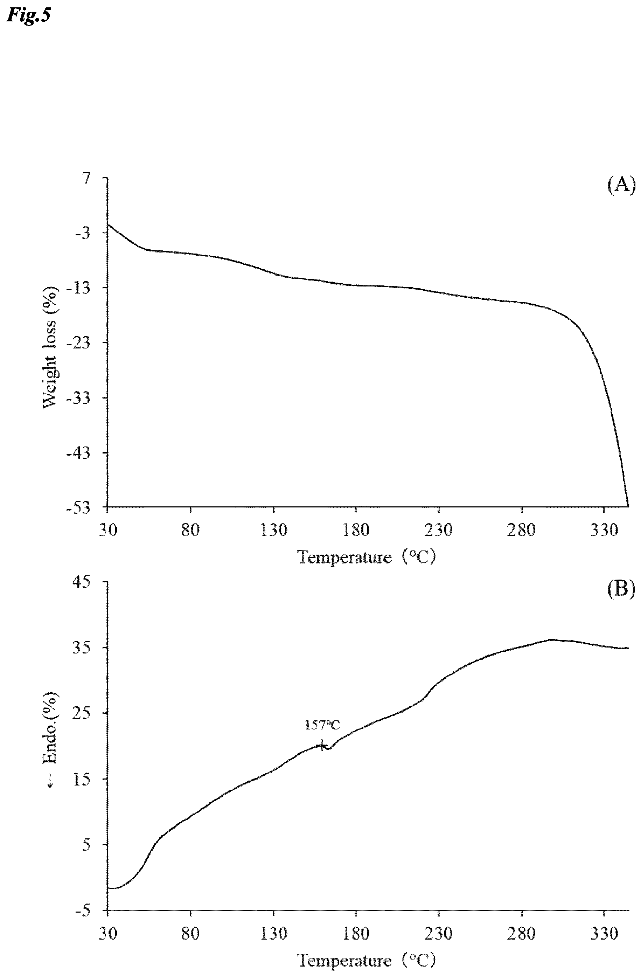

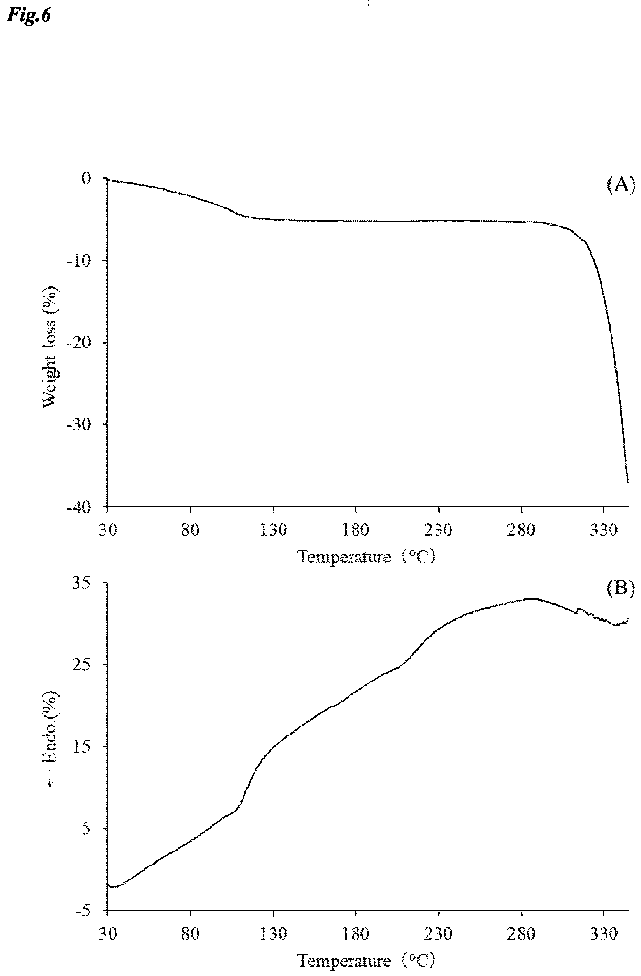

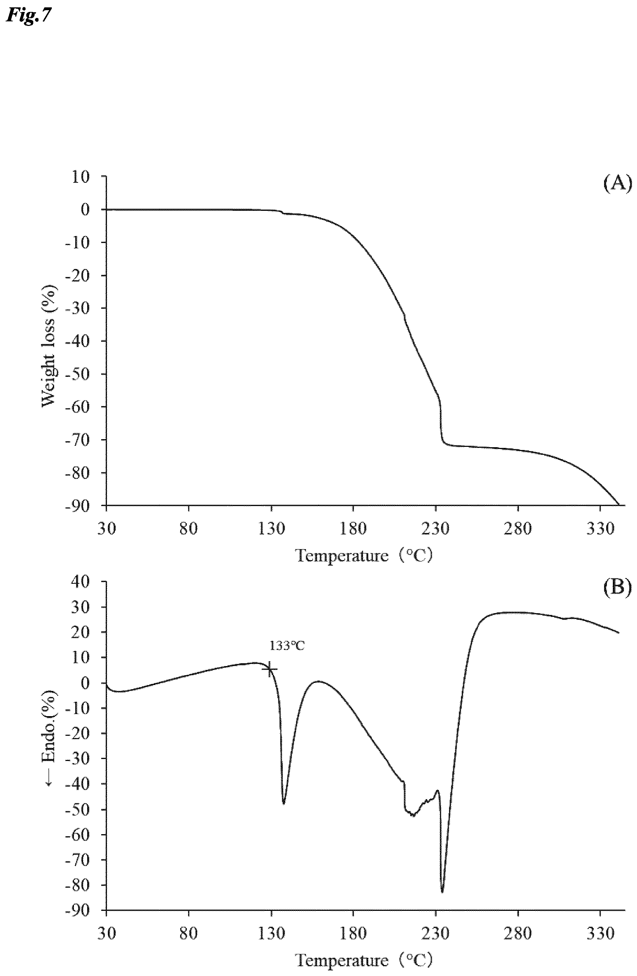

To compound CP04 (40.7 mg), 2-methoxyethanol (320 µL) was added, and urea (7.9 mg) was further added and the mixture was stirred. The seed crystals obtained in Example 2-1-1 were added and the mixture was further stirred. After 2 hours, the suspension was filtered and the wet powder was subjected to powder X-ray diffraction measurement. Drying at room temperature and under normal pressure for 16 hours yielded 22.1 mg of the cocrystal. The results of powder X-ray diffraction for the cocrystal are shown in Figure 4. Comparison with the pattern described in Figure 1 confirmed that the crystal equivalent to Example 2-1-1 was obtained. Also, the results of TG-DTA measurement are shown in Figure 5. The cocrystal was confirmed to have a single melting point that is different from that of the hydrate crystal (Form C) of compound CP04 shown in Figure 6 and urea shown in Figure 7.

### (Comparative Example 2-1-3)

To compound CP04 (9.9 mg) was added 2-methoxyethanol (80 µL), and the cocrystal obtained in Example 2-1-1 (about 0.1 mg) was added as seed crystals. The mixture was stirred for 29 days, but no crystal was obtained.

### (Comparative Example 2-1-4)

To urea (19.2 mg) was added 2-methoxyethanol (800 µL), and the mixture was heated and dissolved. After cooling to room temperature, the cocrystal obtained in Example 2-1-1 (about 0.1 mg) was added as seed crystals. The mixture was stirred for 29 days, but no crystal was obtained.

### (Example 2-2-1)

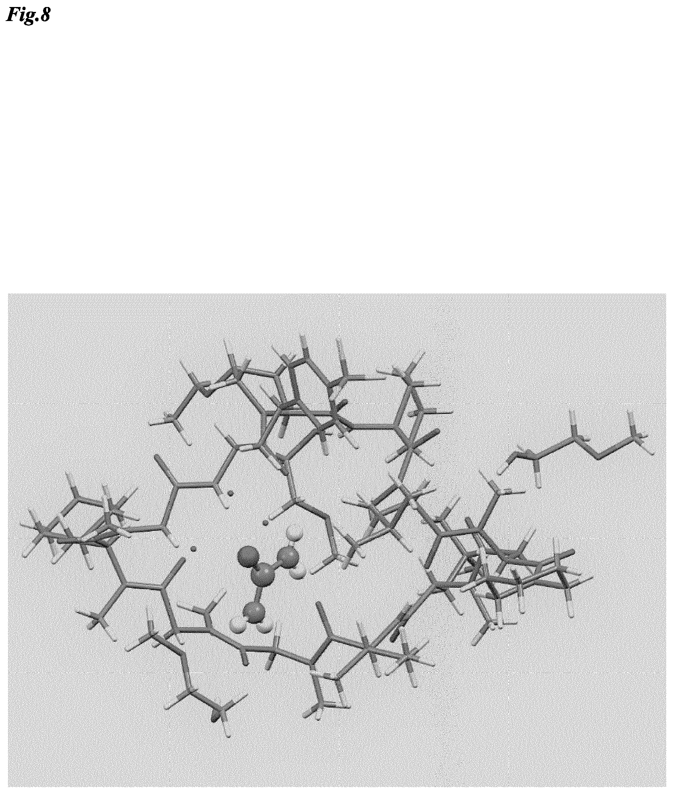

To compound CP01 (306.7 mg) was added 1,4-dioxane (1.53 mL). To this solution (20 µL) was added a 100 mg/mL aqueous urea solution (15 µL), and the mixture was freeze-dried for 3 days. To the obtained powder was added 15 µL of a 2-methoxyethanol/water = 3/1 (v/v) solution, and the mixture was shaken for 9 days to obtain a crystal. The results of single crystal X-ray structural analysis are shown in Figure 8. It was confirmed by the present analysis that the cocrystal of CP01 and urea was formed.

### (Example 2-2-2)

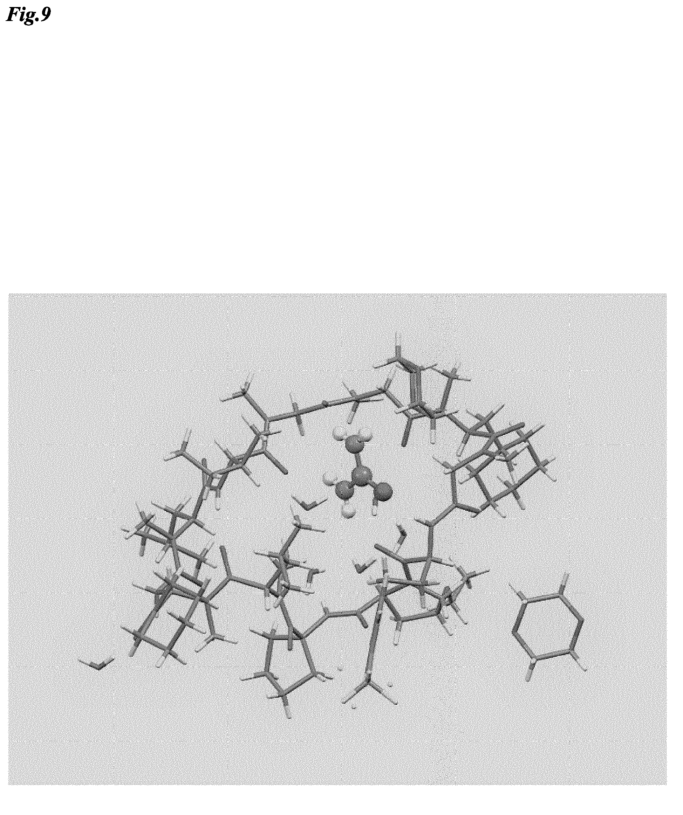

To compound CP01 (306.7 mg) was added 1,4-dioxane (1.53 mL). To this solution (20 µL) was added a 100 mg/mL aqueous urea solution (15 µL), and the mixture was freeze-dried for 3 days. To the obtained powder, a 1,4-dioxane/water = 1/1 (v/v) solution (15 µL) was added, and the mixture was shaken for 9 days to obtain a crystal. The results of single crystal X-ray structural analysis are shown in Figure 9. It was confirmed by the present analysis that the cocrystal of CP01 and urea was formed.

### (Example 2-2-3)

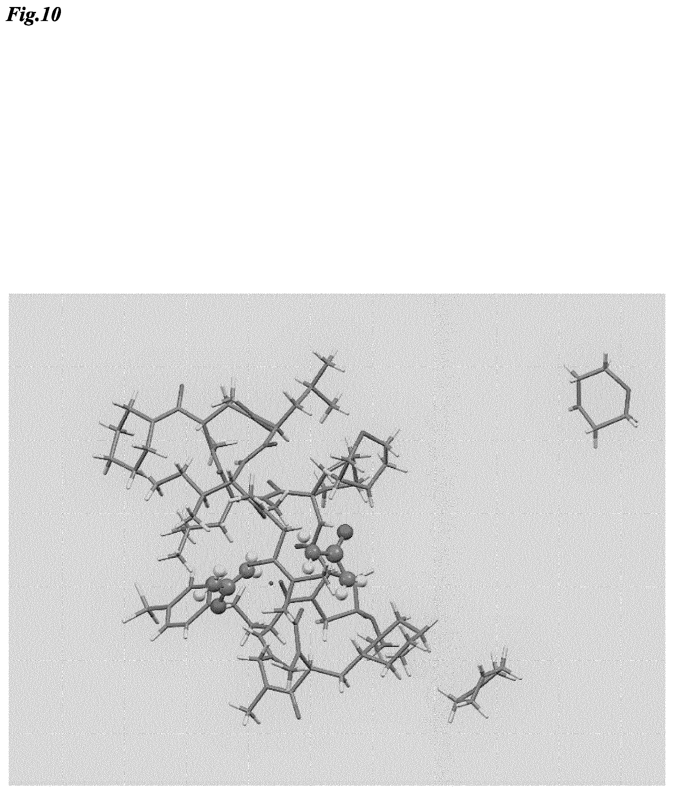

To compound CP01 (306.7 mg) was added 1,4-dioxane (1.53 mL). To this solution (20 µL) was added a 100 mg/mL aqueous urea solution (15 µL), and the mixture was freeze-dried for 3 days. To the obtained powder was added 15 µL of a morpholine/water = 3/1 (v/v) solution, and the mixture was shaken for 9 days to obtain a crystal. The results of single crystal X-ray structural analysis are shown in Figure 10. It was confirmed by the present analysis that the cocrystal of CP01 and urea was formed.

### (Example 2-3-1)

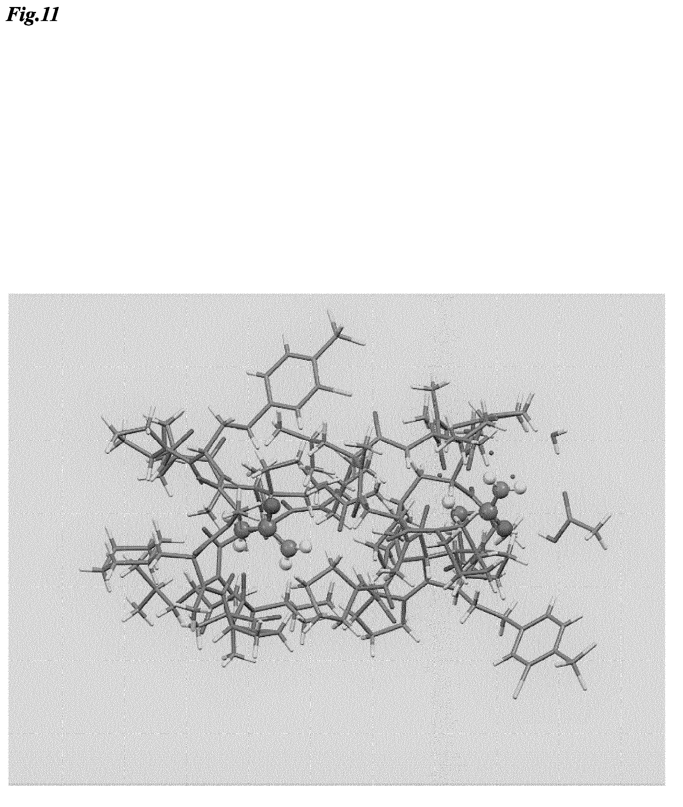

To compound CP02 (606.2 mg) was added 1,4-dioxane (3.03 mL). To this solution (20 µL) was added a 100 mg/mL aqueous urea solution (15 µL), and the mixture was freeze-dried for 3 days. To the obtained powder was added an acetic acid/water = 1/1 (v/v) solution (15 µL), and the mixture was shaken for 9 days to obtain a crystal. The results of single crystal X-ray structural analysis are shown in Figure 11. It was confirmed by the present analysis that the cocrystal of CP02 and urea was formed.

### (Example 2-3-2)

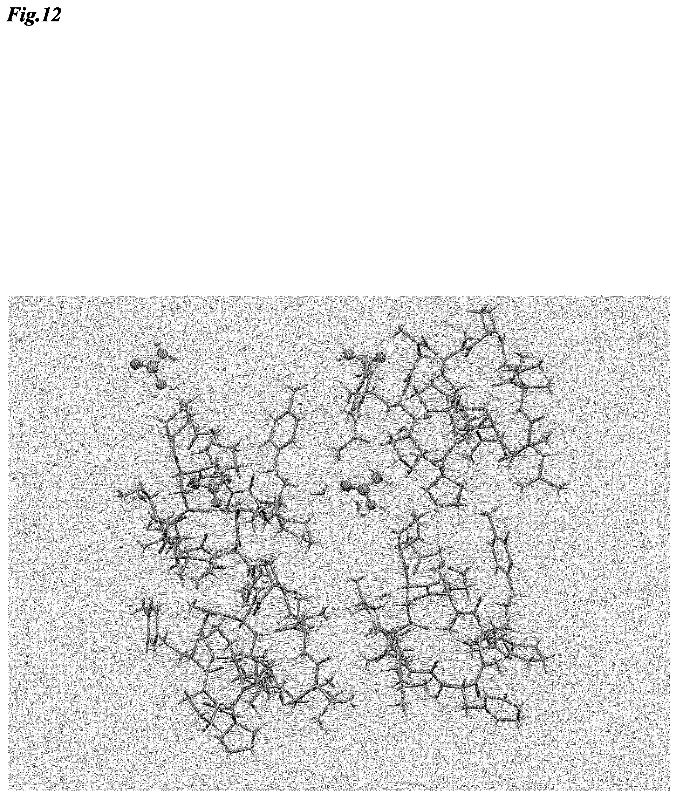

To compound CP02 (606.2 mg) was added 1,4-dioxane (3.03 mL). To this solution (20 µL) was added a 100 mg/mL aqueous urea solution (15 µL), and the mixture was freeze-dried for 3 days. To the obtained powder was added an acetone/water = 1/1 (v/v) solution (15 µL), and the mixture was shaken for 9 days to obtain a crystal. The results of single crystal X-ray structural analysis are shown in Figure 12. It was confirmed by the present analysis that the cocrystal of CP02 and urea was formed.

### (Example 2-3-3)

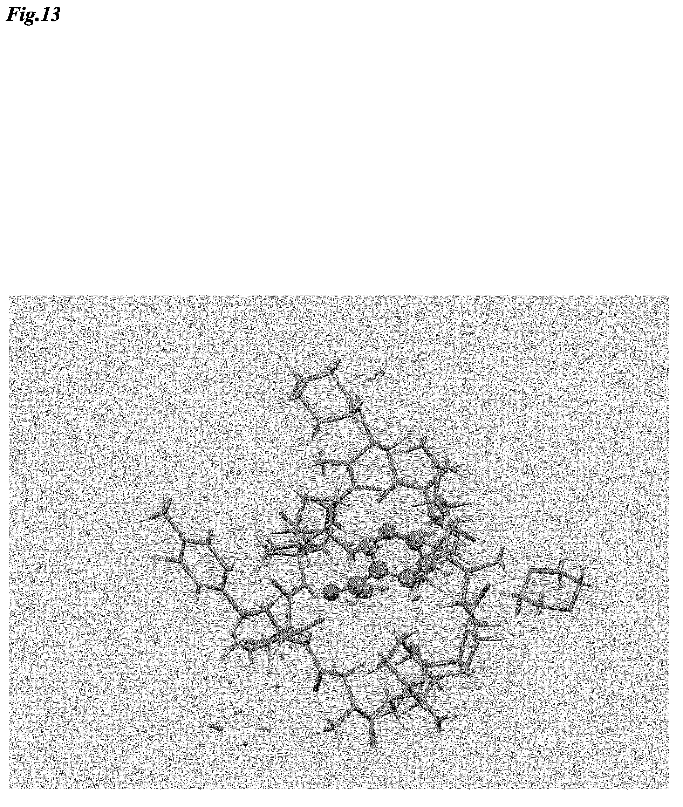

To compound CP02 (606.2 mg) was added 1,4-dioxane (3.03 mL). To this solution (20 µL) was added a 100 mg/mL aqueous nicotinamide solution (15 µL), and the mixture was freeze-dried for 3 days. To the obtained powder, a 1,4-dioxane/water = 1/1 (v/v) solution (15 µL) was added, and the mixture was shaken for 9 days to obtain a crystal. The results of single crystal X-ray structural analysis are shown in Figure 13. It was confirmed by the present analysis that the cocrystal of CP02 and nicotinamide was formed.

### (Example 2-3-4)

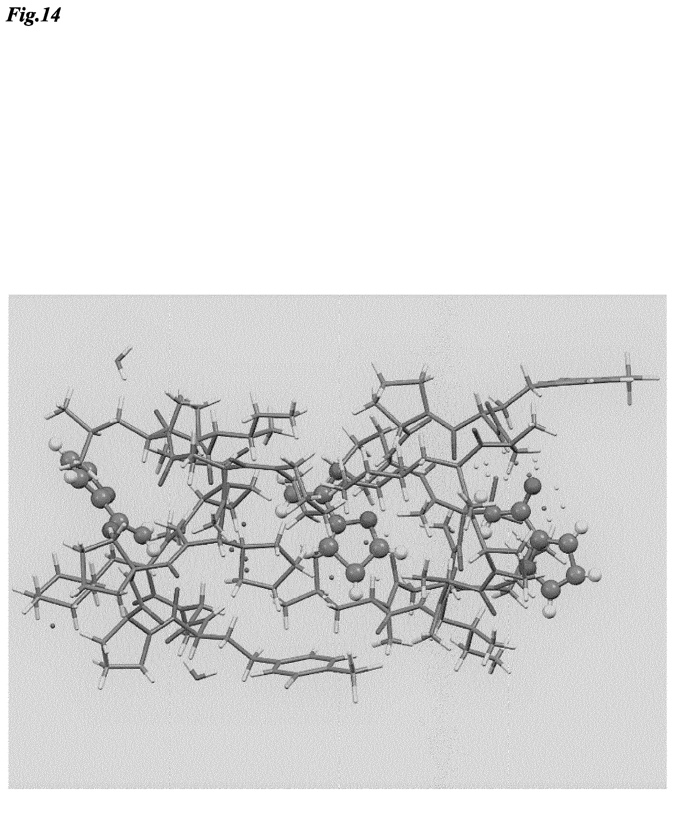

To compound CP02 (606.2 mg) was added 1,4-dioxane (3.03 mL). To this solution (20 µL) was added a 100 mg/mL solution of 2-furamide in dimethyl sulfoxide (15 µL), and the mixture was freeze-dried for 3 days. To the obtained powder was added a 2-ethoxyethanol/water = 1/1 (v/v) solution (15 µL), and the mixture was shaken for 9 days to obtain a crystal. The results of single crystal X-ray structural analysis are shown in Figure 14. It was confirmed by the present analysis that the cocrystal of CP02 and 2-furamide was formed.

### (Example 2-4-1)

Valinomycin (10.3 mg) and 4-cyanopyridine (4.7 mg) were mixed, to which an ethanol/water = 3/1 (v/v) solution (5 µL) was added. Furthermore, two tungsten beads with a diameter of 6.0 mm were added, and grinding was performed using a tabletop bead-type grinding apparatus ShakeMaster Neo (BMS) at 1500 rpm for 5 minutes to obtain a cocrystal.

### (Example 2-4-2)

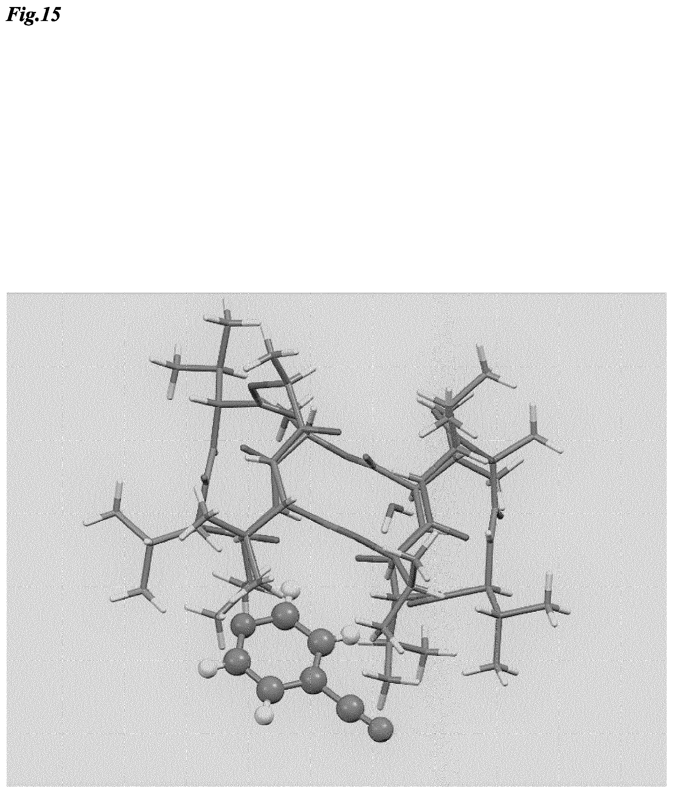

Valinomycin (19.7 mg) and 4-cyanopyridine (5.9 mg) were mixed, to which ethanol (60 µL) was added. The sample was dissolved by heating to 80°C and water (20 µL) was added. After cooling to 60°C, the cocrystal obtained in Example 2-4-1 (about 0.1 mg) was added as seed crystals, and further cooling to 50°C yielded a crystal. The results of single crystal X-ray structural analysis are shown in Figure 15. It was confirmed by the present analysis that the cocrystal of Valinomycin and 4-cyanopyridine was formed.

### (Comparative Example 2-5-1)

To amorphous compound CP03 (5.0 mg) was added 5 µL of triglyme, and the mixture was heated to 80°C and stirred for 17 hours to obtain a crystal.

### (Comparative Example 2-5-2)

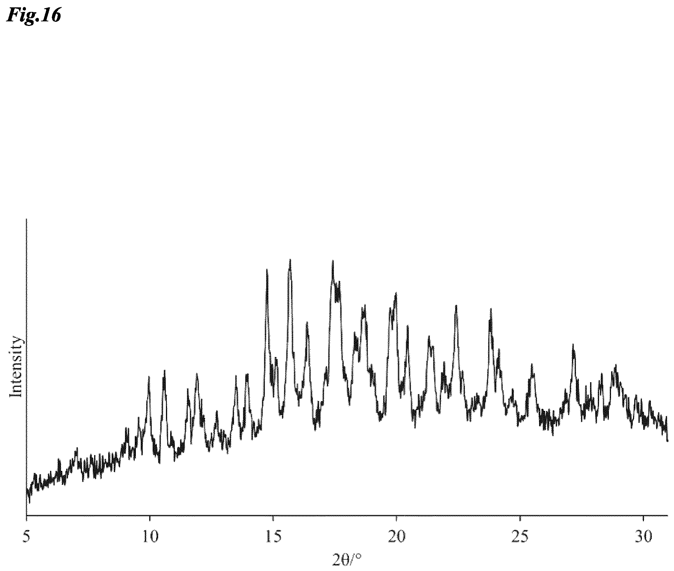

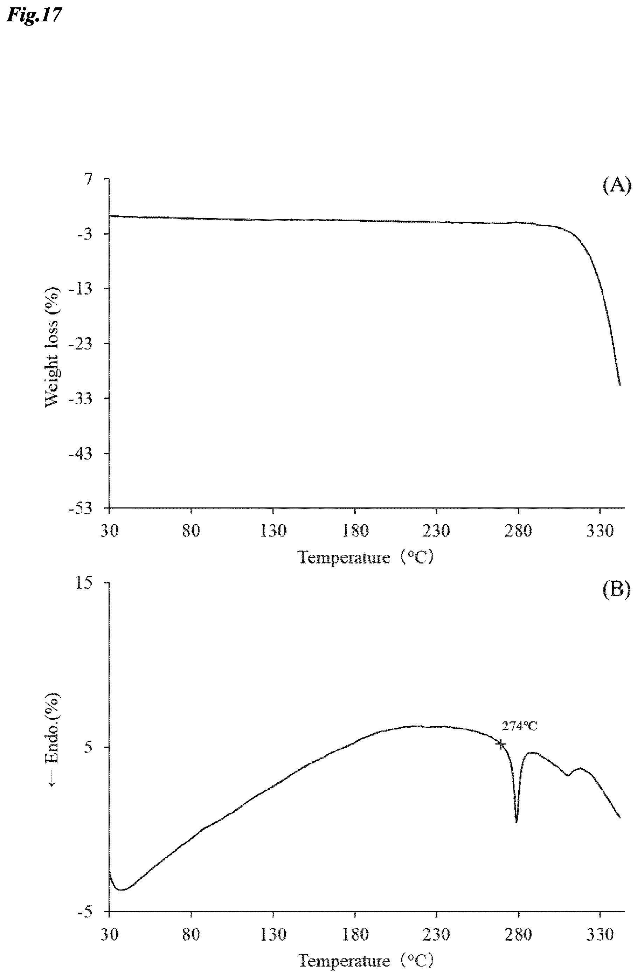

To amorphous compound CP03 (182.0 mg) was added a polyethylene glycol 400/ethanol = 9/1 (v/v) mixed solution (480 µL), and the mixture was heated to 80°C and dissolved. After 55 minutes, the crystal obtained in Comparative Example 2-5-1 (about 0.1 mg) was added as seed crystals, and after further 25 minutes, the solution was cooled to room temperature. After 20 minutes, water (480 µL) was added. After further 25 minutes, water (960 µL) was added. After further 85 minutes, water (2880 µL) was added. After 215 minutes, the suspension was filtered and the obtained powder was subjected to powder X-ray diffraction. The results are shown in Figure 16. Also, TG-DTA measurement was carried out after vacuum drying. The results are shown in Figure 17.

### (Example 2-5-3)

Amorphous compound CP03 (10 mg) and nicotinamide (10 mg) were mixed, to which an ethanol/water = 1/1 (v/v) solution (5 µL) was added. Furthermore, two tungsten beads with a diameter of 6.0 mm were added, and grinding was performed using a tabletop bead-type grinding apparatus ShakeMaster Neo (BMS) at 1500 rpm for 5 minutes to prepare seed crystals of a cocrystal.

### (Example 2-5-4)

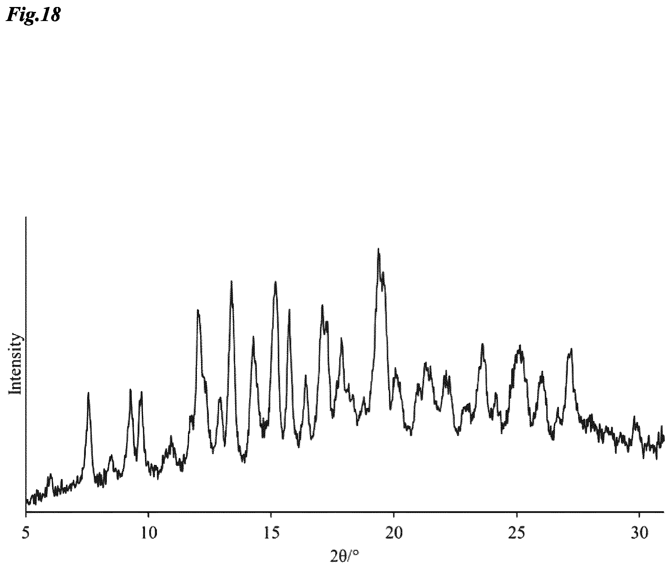

To amorphous compound CP03 (29.0 mg) was added 2 v/w of a nicotinamide solution (ethanol/water = 1/1 (v/v), 333 mg/mL) and the seed crystals of a cocrystal prepared in Example 2-5-3 (about 0.1 mg). After stirring at room temperature, precipitation of a crystal was observed in about 10 minutes. Thereafter, the wet powder was subjected to powder X-ray diffraction measurement. The measurement results are shown in Figure 18. The obtained crystal was confirmed to be a new crystal that is different from the crystal of compound CP03 described in Figure 16 and the crystal of urea described in Figure 3.

### (Example 2-5-5)

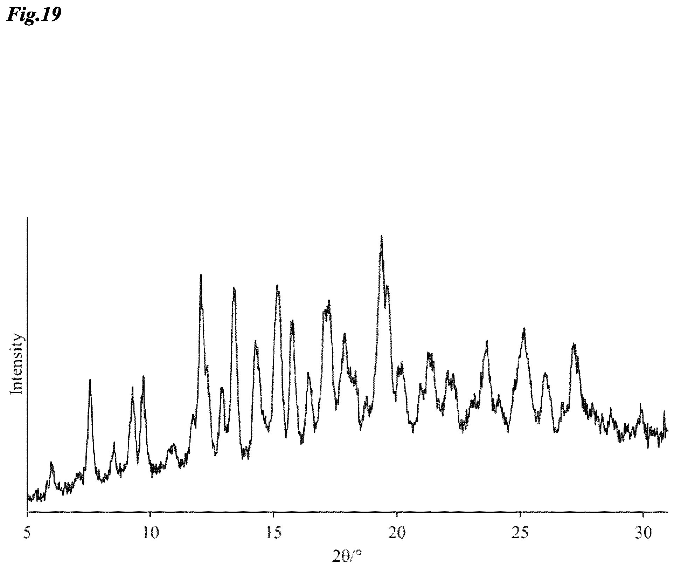

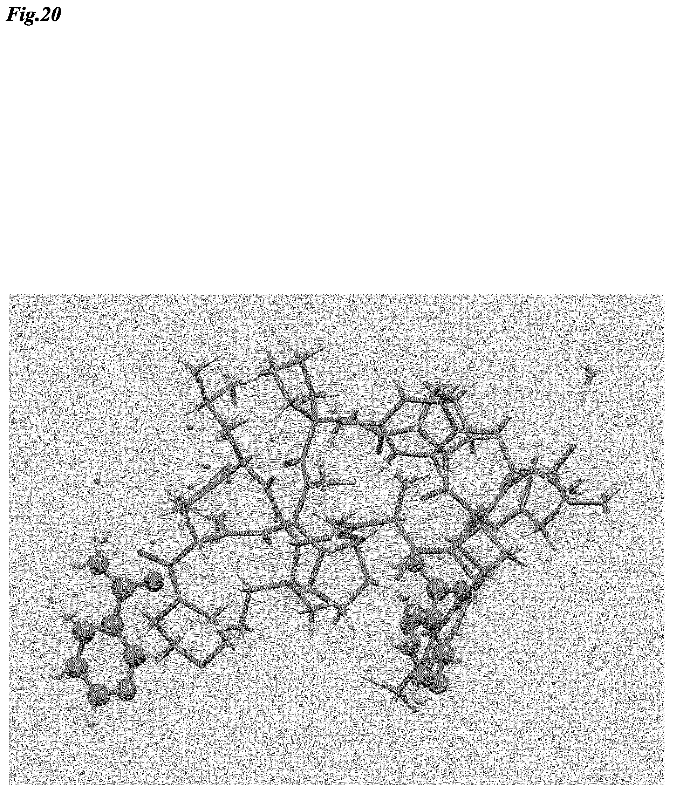

To compound CP03 (93.0 mg) was added 2 v/w of a nicotinamide solution (ethanol/water = 1/1 (v/v), 312 mg/mL), and the mixture was heated to 80°C and dissolved. After cooling to room temperature, the cocrystal obtained in Example 2-5-4 (about 0.1 mg) was added as seed crystals and the solution was shaken for 3 hours and 35 minutes, and the wet powder was subjected to powder X-ray diffraction measurement. The results are shown in Figure 19. The present results confirmed that the crystal form was the same as in Example 2-5-4. By filtration and drying under reduced pressure, 91.8 mg of the crystal was obtained. The results of single crystal X-ray structural analysis are shown in Figure 20. It was confirmed that the cocrystal of CP03 and nicotinamide was formed.

### (Example 2-5-6)

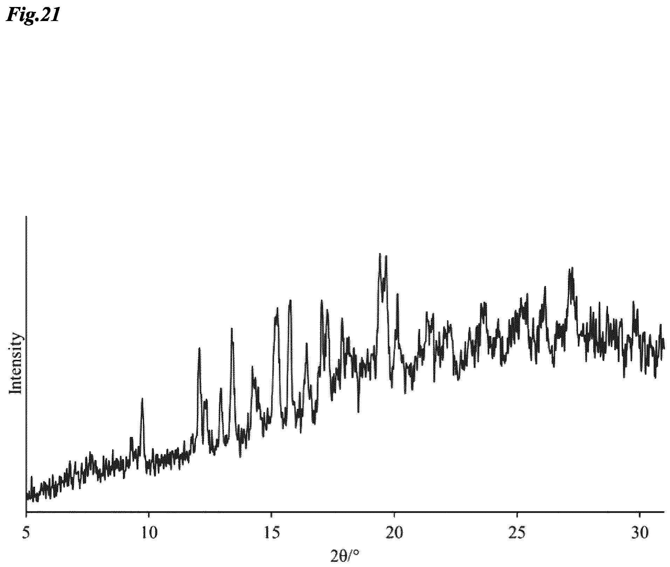

To the crude product of compound CP03 (30.4 mg) was added 2 v/w of a nicotinamide solution (ethanol/water = 1/1 (v/v), 316 mg/mL), and the mixture was heated at 80°C and dissolved. After cooling to room temperature, a cocrystal obtained by the same production method as in Example 2-5-4 (about 0.1 mg) was added as seed crystals, and the solution was shaken for 2 hours and 30 minutes. Furthermore, 8 v/w of a nicotinamide solution (ethanol/water = 4/6 (v/v), 50 mg/mL) was added, and the mixture was shaken at room temperature for 24 hours. The wet powder was subjected to powder X-ray diffraction measurement. The results are shown in Figure 21. The present measurement confirmed that the crystal form was the same as the crystal obtained in Example 2-5-4. Thereafter, by filtration and drying under reduced pressure, 21.6 mg of the crystal was obtained. When the purity of the crude product of compound CP03 used and the obtained cocrystal was confirmed by ultra-high performance liquid chromatography (detailed analytical conditions are described in Example 3-7), the respective area ratios were found to be 88.5% and 97.7%, confirming that the purity of the cyclic peptide was improved by the cocrystallization and filtration.

### (Example 2-5-7)

To amorphous compound CP03 (24.8 mg) was added 2 v/w of a saturated urea solution in ethanol/water = 1/1 (v/v). The mixture was heated to 70°C, stirred for 30 minutes, and then cooled to room temperature. Further stirring at room temperature for 2 hours and 30 minutes yielded a crystal.

### (Example 2-5-8)

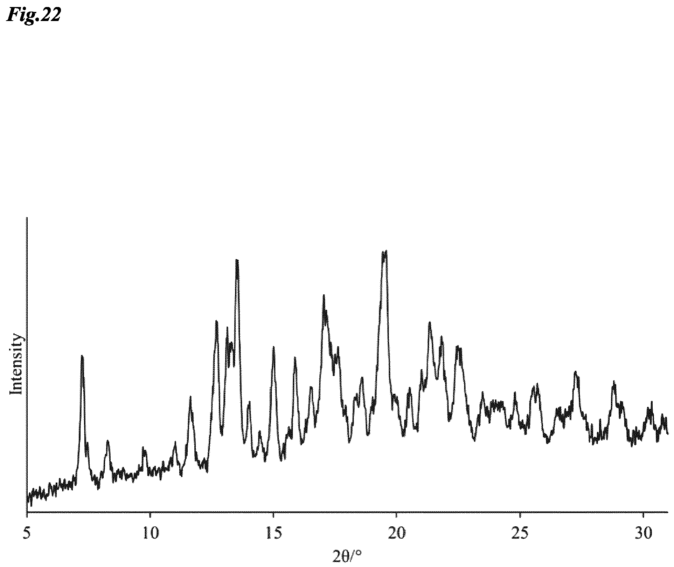

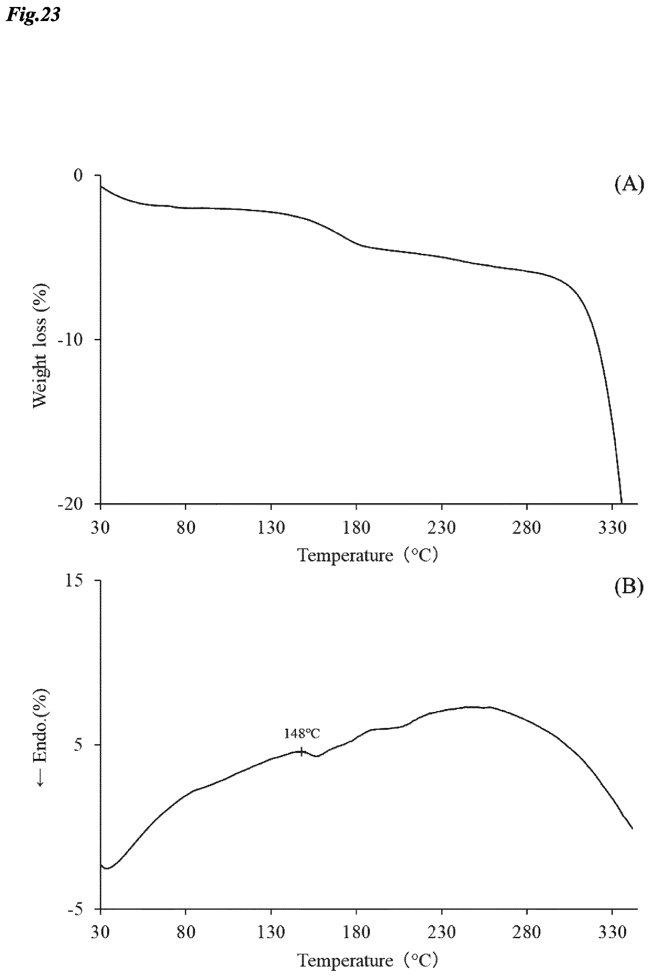

To amorphous compound CP03 (35.7 mg) were added an ethanol/water = 1/1 (v/v) solution (2 v/w) and a saturated urea in ethanol/water = 1/1 (v/v) solution (2 v/w). Furthermore, the cocrystal obtained in Example 2-5-7 (about 0.1 mg) was added as seed crystals, and the solution was stirred at room temperature for 2 hours and 30 minutes. After filtration, rinsing with an ethanol/water = 1/1 (v/v) solution (about 0.1 mL) yielded 13.6 mg of a crystal. The results of powder X-ray diffraction are shown in Figure 22. The obtained cocrystal was confirmed to have a diffraction pattern that is different from that of compound CP03 described in Figure 16 and urea described in Figure 3. Also, the results of TG-DTA measurement are shown in Figure 23. The obtained cocrystal was confirmed to exhibit a single melting point at about 148°C, which is different from that of compound CP03 shown in Figure 17 and urea shown in Figure 7.

### (Comparative Example 2-6-1)

To compound CP05 (77.4 mg) was added dimethyl sulfoxide (5 v/w). This solution (15 µL) was freeze-dried for 3 days. To the obtained powder, a 1,4-dioxane/n-heptane = 1/4 (v/v) solution (15 µL) was added, and the mixture was shaken for 7 days to obtain a crystal.

### (Comparative Example 2-6-2)

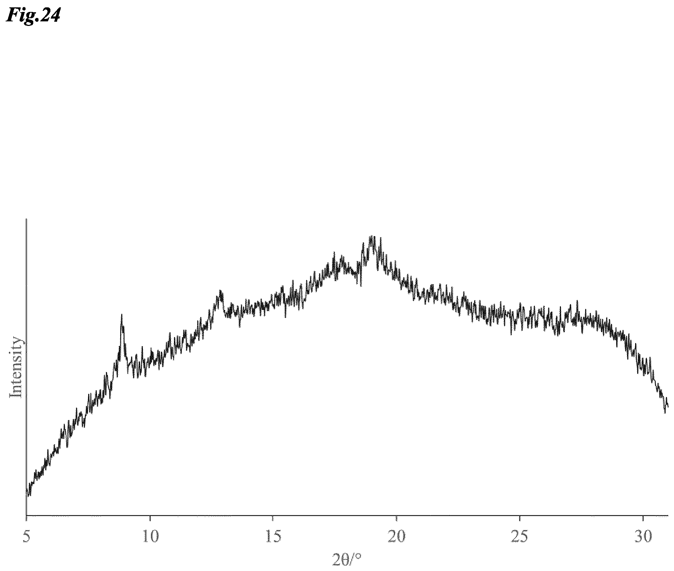

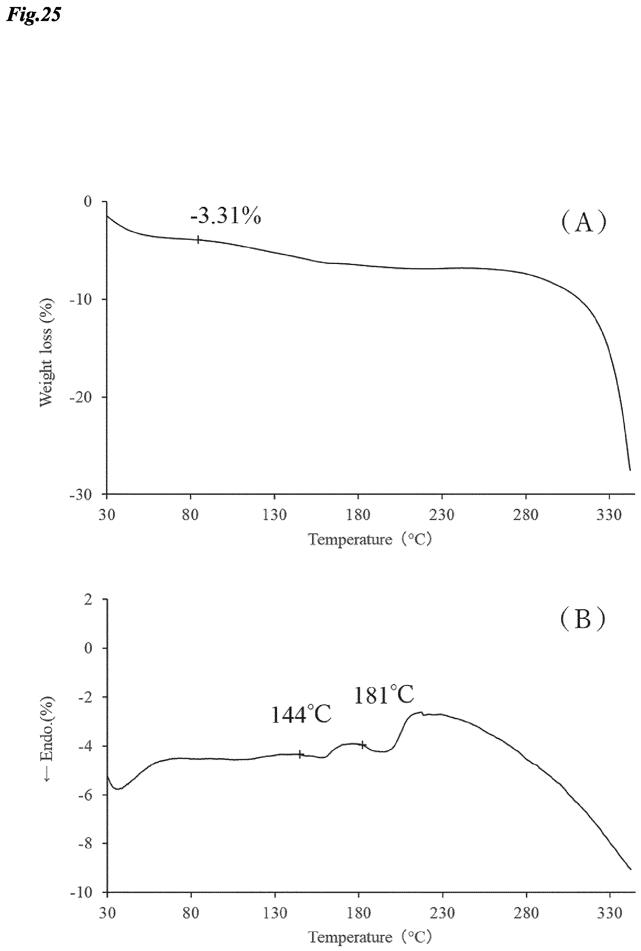

To compound CP05 (10.9 mg) was added 1,4-dioxane (3 v/w). n-Heptane of 1 v/w was added, and the crystal obtained in Comparative Example 2-6-1 (about 0.1 mg) was further added as seed crystals. Thereafter, n-heptane (5 v/w) was added, and the mixture was stirred at room temperature to obtain a crystal. The results of powder X-ray diffraction are shown in Figure 24. Also, the results of TG-DTA measurement are shown in Figure 25.

### (Example 2-6-3)

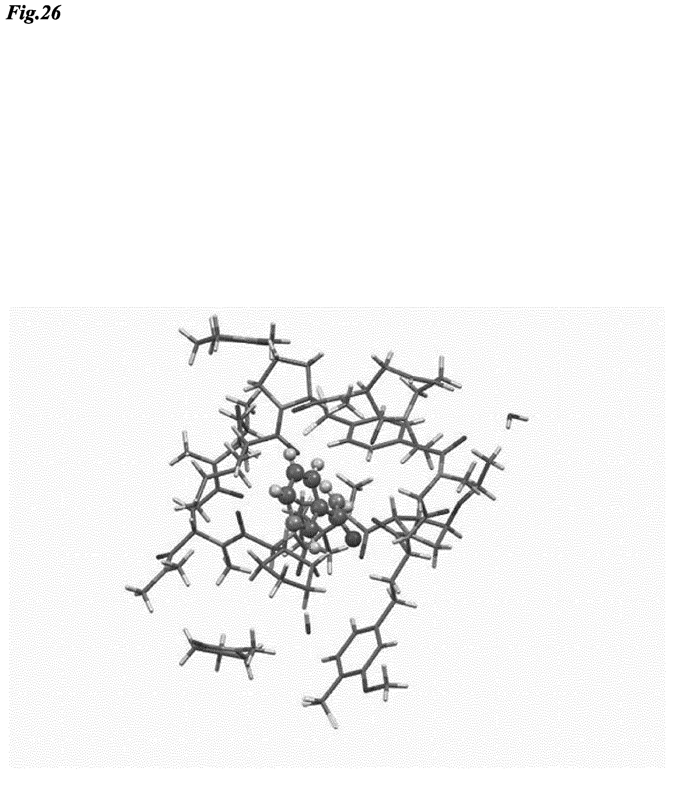

To compound CP05 (364.8 mg) was added 1,4-dioxane (5 v/w). To this solution (15 µL) was added a 100 mg/mL aqueous nicotinamide solution (15 µL), and the mixture was freeze-dried for 3 days. To the obtained powder, a N-methylpyrrolidone/water = 3/1 (v/v) solution (15 µL) was added, and the mixture was shaken for 7 days to obtain a crystal. The results of single crystal X-ray structural analysis are shown in Figure 26. It was confirmed by the present analysis that the cocrystal of CP05 and nicotinamide was formed.

### (Example 2-6-4)

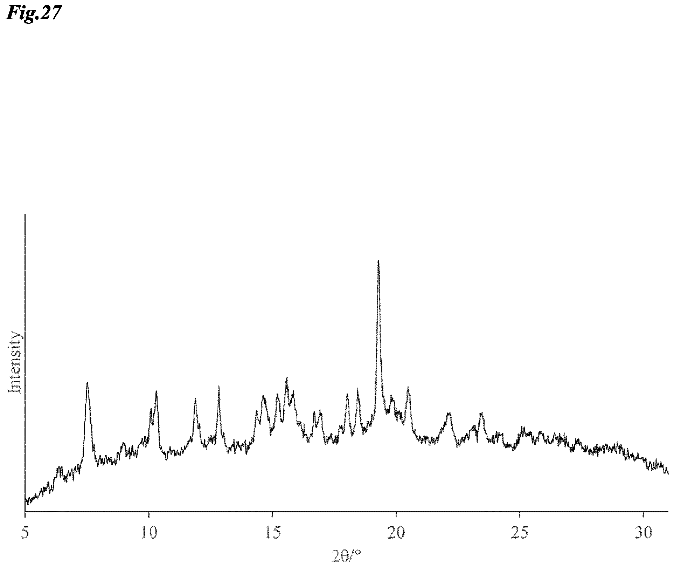

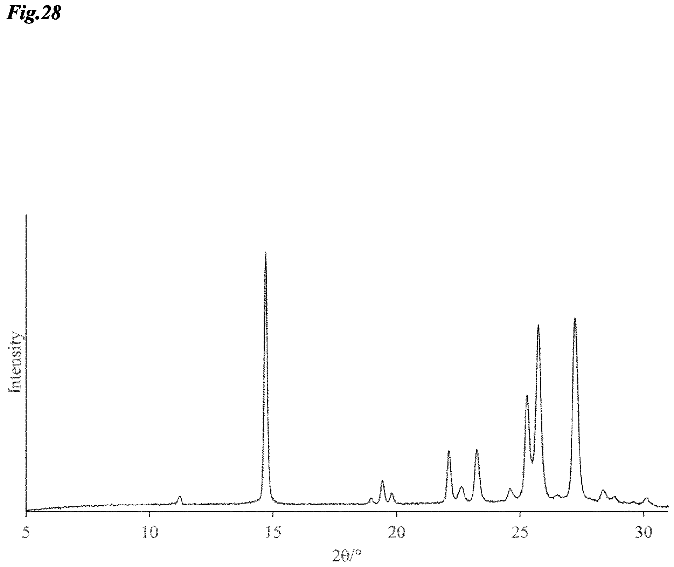

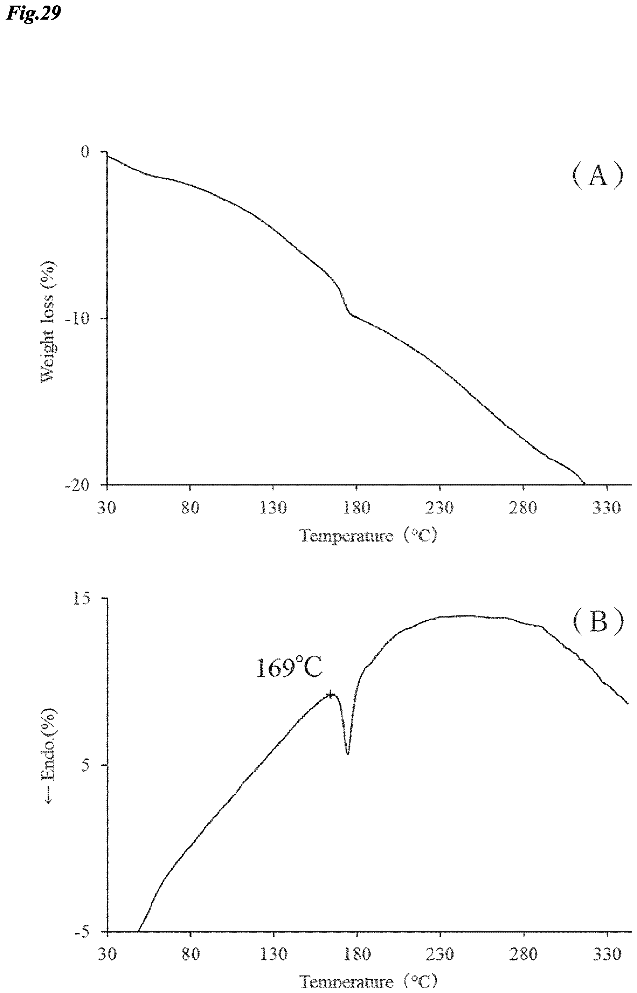

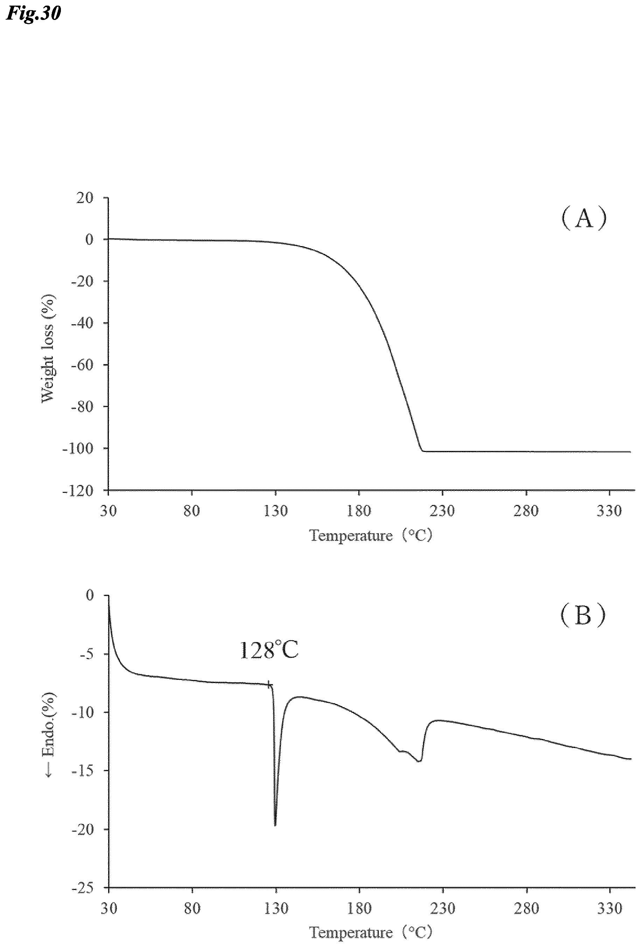

To compound CP05 (30.8 mg) were added a N-methylpyrrolidone/water = 3/1 (v/v) solution (5 v/w) and a saturated nicotinamide in N-methylpyrrolidone/water = 3/1 (v/v) solution (2 v/w). Furthermore, the cocrystal obtained in Example 2-6-3 (about 0.1 mg) was added as seed crystals, and the solution was stirred at room temperature for 2 hours and 40 minutes. After filtration, washing with a N-methylpyrrolidone/water = 3/1 (v/v) solution and vacuum drying for 16 hours yielded 13.3 mg of a crystal. The results of powder X-ray diffraction are shown in Figure 27. The obtained cocrystal was confirmed to have a diffraction pattern that is different from that of compound CP05 described in Figure 24 and nicotinamide described in Figure 28. Also, the results of TG-DTA measurement are shown in Figure 29. The obtained cocrystal was confirmed to exhibit a single melting point at about 169°C, which is different from the possible phase transitions of CP05 shown in Figure 25 at about 144°C and 181°C, and from the melting point of nicotinamide shown in Figure 30 at about 128°C.

### (Comparative Example 2-7-1)

To compound CP06 (73.0 mg) was added dimethyl sulfoxide (5 v/w). This solution (15 µL) was freeze-dried for 3 days. To the obtained powder, an acetone/n-heptane = 1/4 (v/v) solution (15 µL) was added, and the mixture was shaken for 10 days to obtain a crystal.

### (Comparative Example 2-7-2)

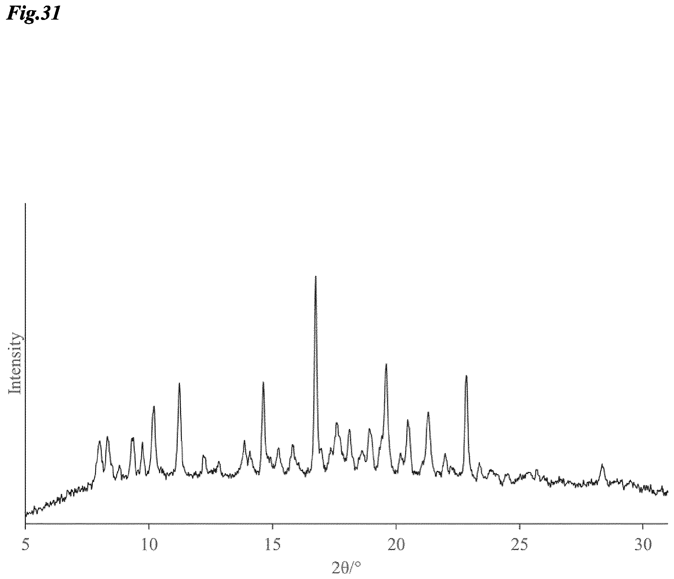

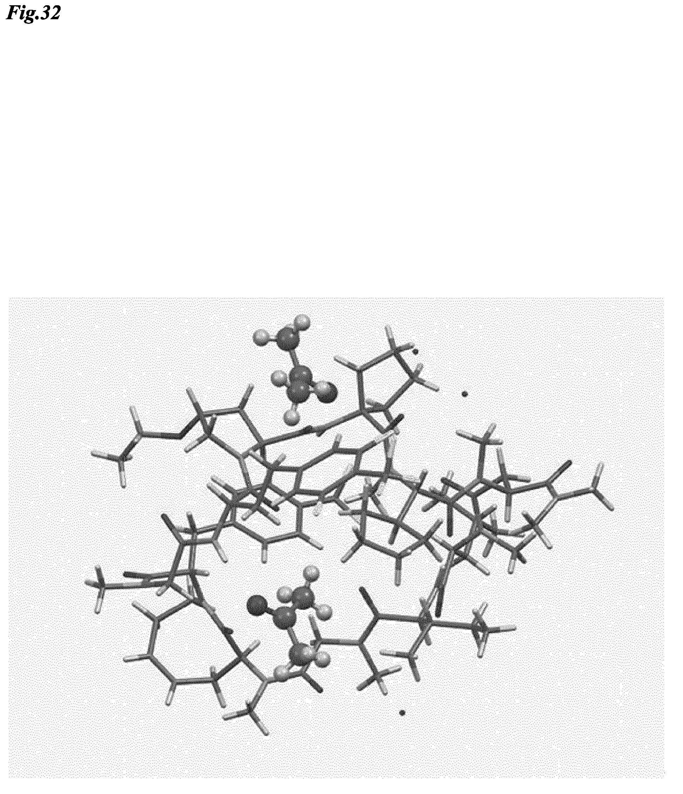

To compound CP06 (11.3 mg) was added acetone (3 v/w). The crystal obtained in Comparative Example 2-7-1 (about 0.1 mg) was further added as seed crystals. Thereafter, n-heptane (2 v/w) was added, and the mixture was stirred at room temperature to obtain a crystal. The results of powder X-ray diffraction are shown in Figure 31. The results of single crystal X-ray structural analysis are shown in Figure 32. It was confirmed by the present analysis that the crystal of CP06 was a solvate crystal containing water and acetone.

### (Example 2-7-3)

To compound CP06 (361.3 mg) was added 1,4-dioxane (5 v/w). To this solution (15 µL) was added a 100 mg/mL aqueous urea solution (15 µL), and the mixture was freeze-dried for 3 days. To the obtained powder was added an acetone/water = 1/1 (v/v) solution (15 µL), and the mixture was shaken for 7 days to obtain a cocrystal.

### (Example 2-7-4)

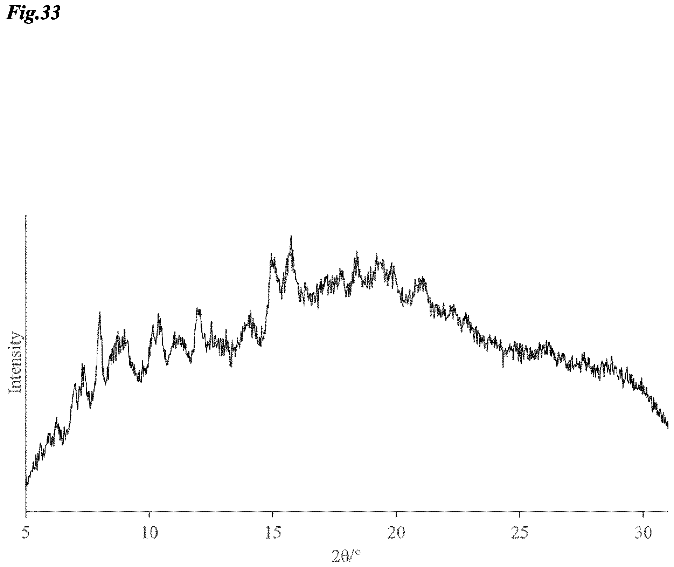

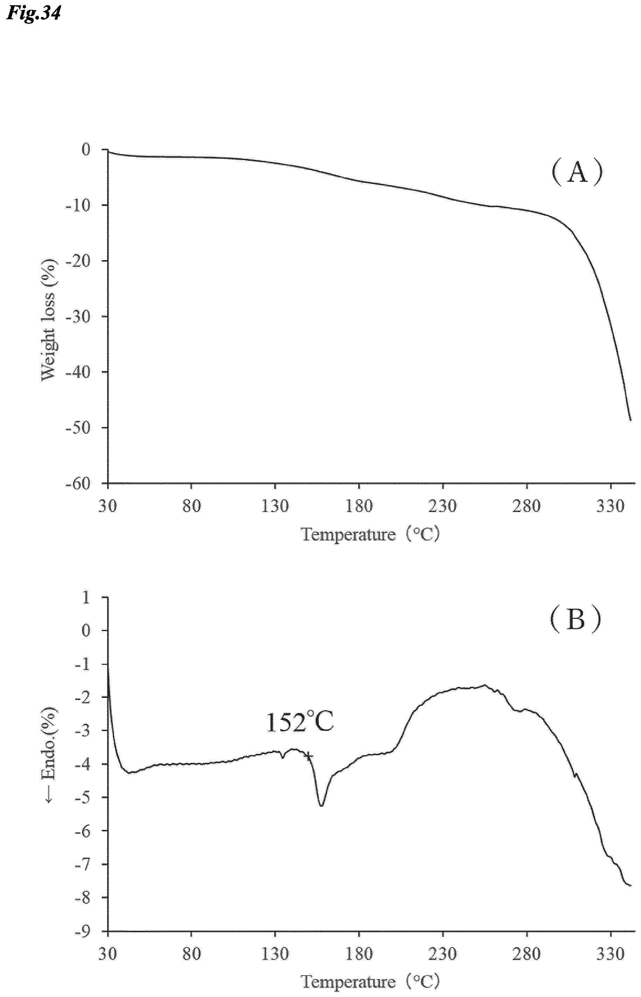

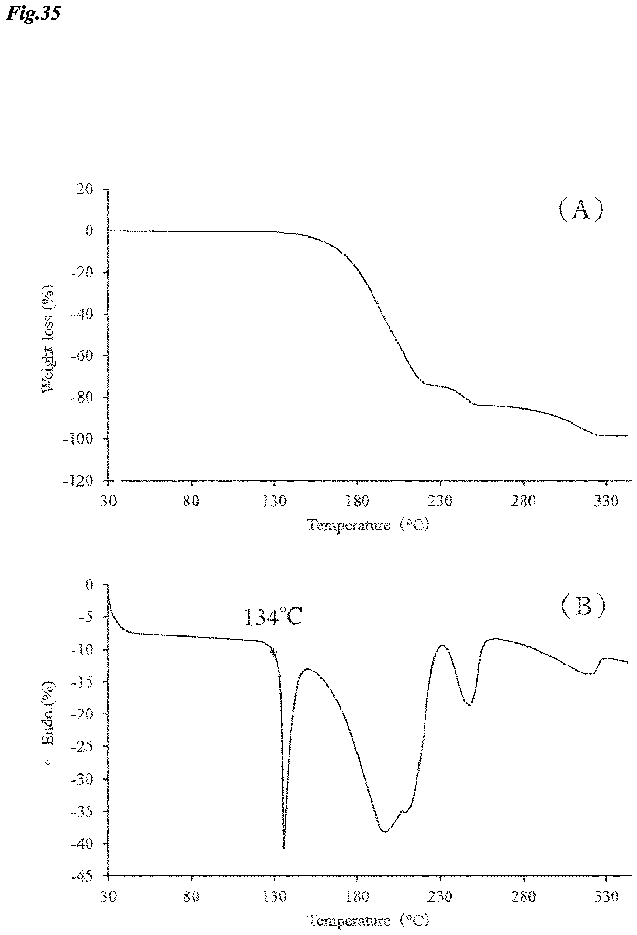

To compound CP06 (21.1 mg) were added an acetone/water = 1/1 (v/v) solution (5 v/w) and a saturated urea in acetone/water = 1/1 (v/v) solution (2 v/w). Furthermore, the cocrystal obtained in Example 2-7-3 (about 0.1 mg) was added as seed crystals, and the solution was stirred at room temperature. After filtration, washing with an acetone/water = 1/1 (v/v) solution and vacuum drying for 16 hours yielded 15.3 mg of a crystal. The results of powder X-ray diffraction are shown in Figure 33. The obtained cocrystal was confirmed to have a diffraction pattern that is different from that of compound CP06 described in Figure 31 and urea described in Figure 3. Also, the results of TG-DTA measurement are shown in Figure 34. The obtained cocrystal was confirmed to exhibit a single melting point at about 152°C, which is different from the melting point of urea shown in Figure 35 at about 134°C. Also, when the content analysis for urea was carried out by the internal standard method using ultra-high performance liquid chromatography in the manner shown in Example 3-5, the content of urea was found to be 11.0%.

### (Comparative Example 2-7-5)

To compound CP06 (9.2 mg) was added an acetone/water = 1/1 (v/v) solution (7 v/w). The cocrystal obtained in Example 2-7-3 (about 0.1 mg) was added as seed crystals. The mixture was stirred for the same period of time as in Example 2-7-4, but no crystal was obtained.

### (Example 2-7-6)

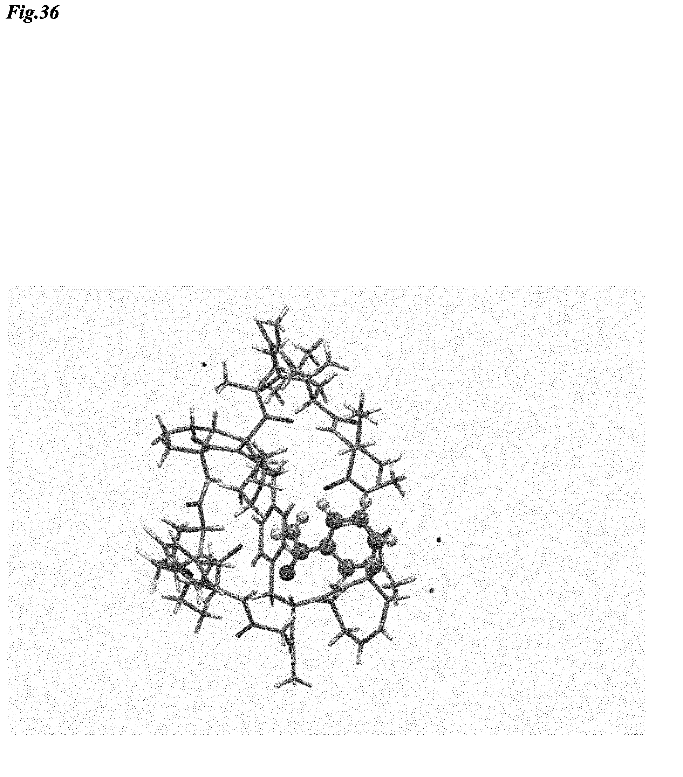

To compound CP06 (361.3 mg), 1,4-dioxane (5 v/w) was added. To this solution (15 µL), a 100 mg/mL aqueous nicotinamide solution (15 µL) was added, and the mixture was freeze-dried for 3 days. To the obtained powder, a 2-methoxyethanol/water = 1/1 (v/v) solution (15 µL) was added, and the mixture was shaken for 7 days to obtain a crystal. The results of single crystal X-ray structural analysis are shown in Figure 36. It was confirmed by the present analysis that the cocrystal of CP06 and nicotinamide was formed.

### (Example 2-7-7)

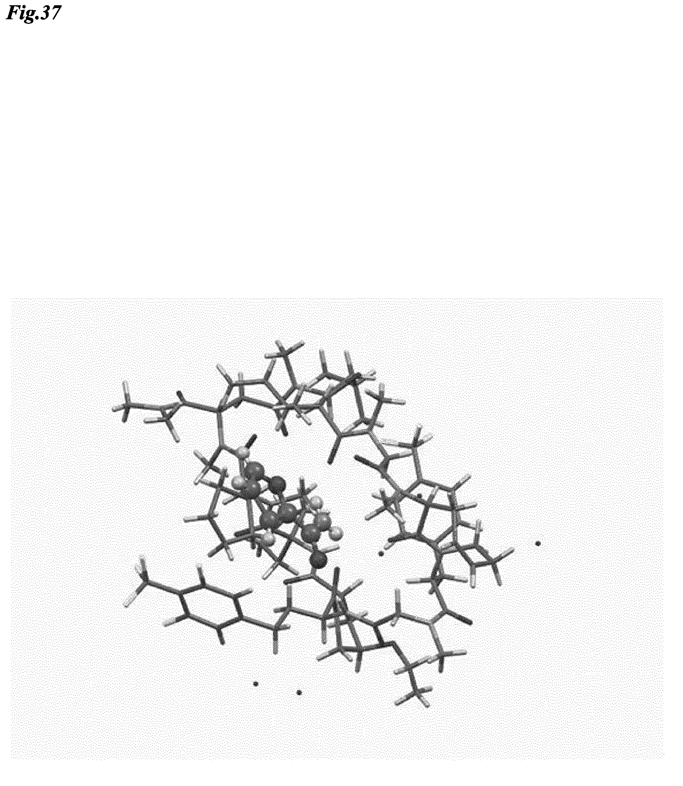

1,4-dioxane (5 v/w) was added to compound CP06 (361.3 mg). To this solution (15 µL) was added a 100 mg/mL solution of 2-furamide in dimethyl sulfoxide (15 µL), and the mixture was freeze-dried for 3 days. To the obtained powder, a N-methylpyrrolidone/water = 1/1 (v/v) solution (15 µL) was added, and the mixture was shaken for 7 days to obtain a crystal. The results of single crystal X-ray structural analysis are shown in Figure 37. It was confirmed by the present analysis that the cocrystal of CP06 and 2-furamide was formed.

### [Example 3] Evaluation of crystal of cyclic peptide

### (Example 3-1)

The powder X-ray diffraction measurement in Example 2 was performed under the following conditions.
Measurement apparatus: D8 Discover, 2D VÅNTEC-500 solid state detector (manufactured by Bruker)
Radiation source: CuKα
Tube voltage and tube current: 40 kV and 40 mA or 50 kV and 1000 µA
Measurement range: 5 to 31°
Exposure time: 40 to 600 seconds

### (Example 3-2)

The single crystal X-ray structural analysis in Examples 2-2, 2-3, and 2-4 was performed under the following conditions.
Measurement apparatus: XtaLAB Synergy Custom with a VariMax Cu diffractometer
Radiation source: CuKα
Tube voltage and tube current: 40 kV and 30 mA
Temperature: -180°C

Also, for the crystal obtained in Example 2-5, single crystal X-ray structural analysis was carried out at the Photon Factory of the High Energy Accelerator Research Organization.
Beamline: BL-5A
Wavelength: 0.85Å
Temperature: -178°C

Whichever apparatus is used, the measurement was carried out with strategies and exposure times that were considered to provide diffraction spots sufficient for structural analysis.

Also, in the structural analysis, initial structure determination was carried out by the direct method (SHELXT 2014/5 or SHELXT 2018/2, Olex2-1.5, OlexSys Ltd.), and structural refinement was carried out by the full-matrix least-squares method (SHELXL 2017/1 or SHELXL 2018/3, OlexSys Ltd.).

### (Example 3-3)

The thermogravimetry-differential thermal analysis (TG-DTA) in Example 2 was performed under the following conditions. The onset temperature of the endothermic peak was defined as the melting point.
Apparatus: STA7200RV (manufactured by Hitachi High-Tech Science Corporation)
Measurement atmosphere: nitrogen
Heating conditions: The temperature was raised from 30°C to 350°C at 10°C/min.

### (Example 3-4)

For the crystal obtained in Example 2-1-1, the content analysis was carried out by ultra-high performance liquid chromatography using the following measurement method, and the content of compound CP04 was calculated. Also, the solubility measurement in Example 3-6 was performed by the same method.
Apparatus: ACQUITY UPLC H-Class (manufactured by Waters Corporation)
Detector: PDA (measurement wavelength: 225 nm)
Column: BIOShell 160Å Peptide C18 15 cm × 2.1 mm, 2.7 µm
Column temperature: 50°C
Flow rate: 0.3 mL/min
Mobile phase A: 0.05% trifluoroacetic acid aqueous solution
Mobile phase B: 0.05% trifluoroacetic acid acetonitrile solution
Gradient conditions (% of mobile phase B): 50% (0 min) → 100% (15 min) → 100% (20 min) Internal standard substance: hexyl benzoate (Example 2-1-1) or 2-(1-naphthyl)ethanol (Example 3-6)

### (Example 3-5)

For the crystal obtained in Example 2-1-1, the content analysis was carried out by ultra-high performance liquid chromatography using the following measurement method, and the amount of urea was calculated.
Apparatus: ACQUITY UPLC H-Class Plus (manufactured by Waters Corporation), Corona Veo RS (manufactured by Thermo Fisher Scientific)
Detector: Corona Veo RS
Column: SeQuant (R) ZIC-HILIC 3.5 µm, 100Å, 150 × 4.6 mm (Merck)
Column temperature: 40°C
Flow rate: 0.4 mL/min
Mobile phase: water/acetonitrile = 15/85 (isocratic conditions)
Inverse gradient: water/acetonitrile = 85/15
Internal standard substance: uracil

### (Example 3-6)

The solubility of compound CP04 in the cocrystal containing compound CP04 and urea obtained in Example 2-1-2 and in the hydrate crystal (Form C) of compound CP04 was compared. 4 mL of water was added to 4.224 mg of sodium lauryl sulfate to prepare a dissolving solvent. 1 mg each of the cocrystal obtained in Example 2-1-2 and the hydrate crystal (Form C) of compound CP04 were weighed into a separate 2-mL glass vial, 1 mL of the above dissolving solvent was added to each, and the mixture was stirred at 300 rpm with a stirrer. After 10 minutes, each suspension was filtered and the solubility was measured by the internal standard method under the conditions of Example 3-4, with the results shown in Table 3 below.

**[Table 4]**

| Solid state | Solubility |
|---|---|
| Cocrystal obtained in Example 2-1-1 | 21 µg/mL |
| Hydrate crystal (Form C) of compound CP04 | < 7 µg/mL |

From the above results, it was shown that the cocrystal containing urea has higher solubility than the solvate crystal.

### (Example 3-7)

For the crude product of compound CP03 used in Example 2-5-6 and the nicotinamide cocrystal, purity measurement was performed by ultra-high performance liquid chromatography.
Apparatus: ACQUITY UPLC H-Class (manufactured by Waters Corporation)
Detector: PDA (measurement wavelength: 225 nm)
Column: BIOShell 160Å Peptide C18 15 cm × 2.1 mm, 2.7 µm
Column temperature: 50°C
Flow rate: 0.3 mL/min
Mobile phase A: 0.05% trifluoroacetic acid aqueous solution
Mobile phase B: 0.05% trifluoroacetic acid acetonitrile solution
Gradient conditions (% of mobile phase B): 30% (0 min) → 100% (21 min) → 100% (26 min)
Injection volume: 2.0 µL
Analysis time: 30 minutes

### (Example 3-8)

The solubility of compound CP05 in the cocrystal containing compound CP05 and nicotinamide obtained in Example 2-6-4 and in the crystal of compound CP05 obtained in Comparative Example 2-6-2 was compared. 4 mL of water was added to 4.2 mg of sodium lauryl sulfate to prepare a dissolving solvent. 1 mg each of the cocrystal obtained in Example 2-6-4 and the crystal of compound CP05 obtained in Comparative Example 2-6-2 were weighed into a separate 2-mL glass vial, 1 mL of the above dissolving solvent was added to each, and the mixture was stirred at 300 rpm with a stirrer. After 10 minutes, each suspension was filtered and the solubility was measured by the internal standard method under the conditions of Example 3-4, with the results shown below.

**[Table 5]**

| Solid state | Solubility |
|---|---|
| Cocrystal obtained in Example 2-6-4 | 17 µg/mL |
| Crystal obtained in Comparative Example 2-6-2 | < 13 µg/mL |

From the above results, it was shown that the cocrystal containing nicotinamide has higher solubility than the crystal of compound CP05 obtained in Comparative Example 2-6-2.

### (Example 3-9)

The solubility of compound CP06 in the cocrystal containing compound CP06 and urea obtained in Example 2-7-4 and in the crystal of compound CP06 obtained in Comparative Example 2-7-2 was compared. 4 mL of water was added to 4.2 mg of sodium lauryl sulfate, thereby preparing a dissolving solvent. 1 mg each of the cocrystal obtained in Example 2-7-4 and the crystal of compound CP06 obtained in Comparative Example 2-7-2 were weighed into a separate 2-mL glass vial, 1 mL of the above dissolving solvent was added to each, and the mixture was stirred at 300 rpm with a stirrer. After 10 minutes, each suspension was filtered and the solubility was measured by the internal standard method under the conditions of Example 3-4, with the results shown as follows.

**[Table 6]**

| Solid state | Solubility |
|---|---|
| Cocrystal obtained in Example 2-7-4 | 24 µg/mL |
| Crystal obtained in Comparative Example 2-7-2 | < 13 µg/mL |

From the above results, it was shown that the cocrystal containing urea has higher solubility than the solvate crystal.

## Claims

1. A method for producing a cocrystal containing a cyclic peptide and a compound with a melting point of 20°C or higher, the method comprising a step of bringing the cyclic peptide into contact with the compound and a solvent.

2. The method according to claim 1, wherein the compound is a compound with a molecular weight (g/mol) of 500 or less, 400 or less, 300 or less, 200 or less, 150 or less, or 125 or less.

3. The method according to claim 1 or 2, wherein the compound is a compound represented by R¹-C(=O)-NR²R³ or R¹-C≡N, where R¹ is a C₁-C₄ alkyl, phenyl, 2-pyridyl, 3-pyridyl, 4-pyridyl, 2-furyl, -NH₂, -NH-OH, -C(=O)-NH₂, -CH₂-C(=O)-NH₂, or -(CH₂)₂-C(=O)-NH₂, and R² and R³ are each independently hydrogen or a C₁-C₄ alkyl.

4. A method for producing a cocrystal containing a cyclic peptide and a compound selected from the group consisting of urea, nicotinamide, 2-furamide, and 4-cyanopyridine, the method comprising a step of bringing the cyclic peptide into contact with the compound and a solvent.

5. The method according to claim 4, wherein the cyclic peptide has the following characteristic (i):
(i) a characteristic of containing a cyclic portion composed of 8 to 16 residues of amino acids in total.

6. The method according to any one of claims 1 to 5, wherein the solvent is solvent (A) with a molecular weight of 30 or more and 170 or less, or a mixed solvent of solvent (A) with a molecular weight of 30 or more and 170 or less and solvent (B) with a molecular weight of 18 or more and 75 or less, the solvent (A) is one or more types selected from the group consisting of an amide-based solvent, a sulfoxide-based solvent, an aromatic hydrocarbon-based solvent, a halogen-based solvent, an alcohol-based solvent, an ether-based solvent, an ester-based solvent, a nitrile-based solvent, a carboxylic acid-based solvent, and a ketone-based solvent, and the solvent (B) is one or more types selected from the group consisting of an alkyl alcohol-based solvent and water.

7. The method according to claim 6, wherein the solvent (A) is one or more types selected from the group consisting of an amide-based solvent, an alcohol-based solvent, an ether-based solvent, a carboxylic acid-based solvent, and a ketone-based solvent.

8. The method according to claim 6 or 7, wherein the solvent (B) is one or more types of solvents selected from the group consisting of an alkyl alcohol-based solvent and water.

9. The method according to any one of claims 1 to 8, wherein the step of bringing the cyclic peptide into contact with the compound and the solvent does not comprise adding a seed crystal.

10. The method according to any one of claims 1 to 8, wherein the step of bringing the cyclic peptide into contact with the compound and the solvent comprises adding a seed crystal.

11. The method according to any one of claims 1 to 10, further comprising a solid-liquid separation step after the step of bringing the cyclic peptide into contact with the compound and the solvent.

12. The method according to any one of claims 6 to 11, wherein the cocrystal contains urea, the solvent (A), and the solvent (B).

13. A method for screening a method for producing a cocrystal containing a cyclic peptide and a compound, the method comprising the following steps (a) and (b):
(a) producing a cocrystal containing the cyclic peptide and the compound by following the method according to any one of claims 1 to 12; and
(b) analyzing the cocrystal by single crystal X-ray analysis and/or powder X-ray crystal diffraction.

14. Use of urea, nicotinamide, 2-furamide, or 4-cyanopyridine in production of a cocrystal containing a cyclic peptide and urea, nicotinamide, 2-furamide, or 4-cyanopyridine.

15. A method for purifying a cyclic peptide, the method comprising a step of producing a cocrystal containing a cyclic peptide and a compound by following the method according to any one of claims 1 to 12, and a step of collecting the cocrystal by solid-liquid separation.
